# EUROPEAN PATENT APPLICATION

(11) **EP 4 427 738 A2**
(43) Date of publication of application: **11.09.2024**
(21) Application number: 24191543.8
(22) Date of filing: 31.07.2020
(51) Int. Cl.: A61K 9/00

(54) **DOSING PROTOCOLS AND REGIMENS FOR AMINOSTEROL TREATMENT**

(30) Priority: 02.08.2019 US 201962882313 P; 02.08.2019 US 201962882329 P; 03.09.2019 US 201962895461 P
(62) Divisional of application: 20849342.9
(71) Applicant: Enterin, Inc., Philadelphia, Pennsylvania 19103 (US)
(72) Inventor: BARBUT, Denise, Philadelphia, 19103 (US); ZASLOFF, Michael, Philadelphia, 19103 (US)
(74) Representative: Altmann Stößel Dick Patentanwälte PartG mbB

(57) **Abstract**

The present application relates generally to a method of treating a cutaneous or mucosal wound in a subject in need comprising topically administering to the wound site a therapeutically effective amount of a topical pharmaceutical composition comprising at least one aminosterol or a salt or derivative thereof. Also provided are methods for treating, preventing, and/or slowing the onset or progression of a disease or condition, or a symptom thereof, amenable to treatment with an aminosterol or a salt or derivative thereof in a subject in need, comprising administering to the subject a therapeutically effective amount of at least one aminosterol or a salt or derivative thereof, wherein the subject is not lying down.

## Description

### FIELD

The present application relates generally to methods of treating a cutaneous or mucosal wound, or a mucosal surface, by topically administering at least one aminosterol. In addition, the present application relates to aminosterol dosing protocols for the treatment of disease.

### BACKGROUND

Aminosterols are amino derivatives of a sterol. Examples of aminosterols include squalamine and aminosterol 1436 (also known as trodusquemine and MSI-1436).

Squalamine is a unique compound with a structure of a bile acid coupled to a polyamine (spermidine):

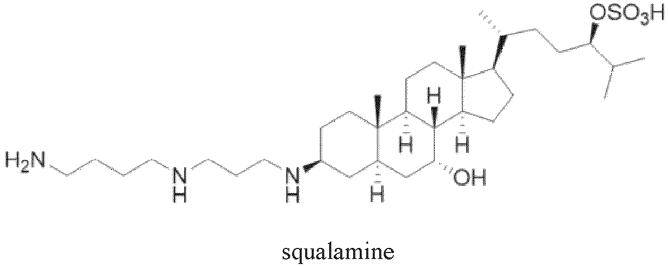

The discovery of squalamine, the structure of which is shown above, was reported by Michael Zasloff in 1993 (U.S. Patent No. 5,192,756). Squalamine was discovered in various tissues of the dogfish shark *(Squalus acanthias*) in a search for antibacterial agents. The most abundant source of squalamine is in the livers of *Squalus acanthias,* although it is found in other sources, such as lampreys (Yun et al., J. of Lipid Research, 48:2579-2586 (2007)).

Several clinical trials have been conducted relating to the use of squalamine, including the following:
(1) ClinicalTrials.gov Identifier NCT01769183 for "Squalamine for the Treatment in Proliferative Diabetic Retinopathy," by Elman Retina Group (6 participants; study completed Aug. 2014) =2);
(2) ClinicalTrials.gov Identifier NCT02727881 for "Efficacy and Safety Study of Squalamine Ophthalmic Solution in Subjects With Neovascular AMD (MAKO)," by Ohr Pharmaceutical Inc. (230 participants; study completed Dec. 2017);
(3) ClinicalTrials.gov Identifier NCT02614937 for "Study of Squalamine Lactate for the Treatment of Macular Edema Related to Retinal Vein Occlusion," by Ohr Pharmaceutical Inc. (20 participants; study completed Dec. 2014);
(4) ClinicalTrials.gov Identifier NCT01678963 for "Efficacy and Safety of Squalamine Lactate Eye Drops in Subjects With Neovascular (Wet) Age-related Macular Degeneration (AMD)," by Ohr Pharmaceutical Inc. (142 participants; study completed Mar. 2015);
(5) ClinicalTrials.gov Identifier NCT00333476 for "A Study of MSI-1256F (Squalamine Lactate) To Treat "Wet" Age-Related Macular Degeneration," by Genaera Corporation (140 participants; study terminated);
(6) ClinicalTrials.gov Identifier NCT00094120 for "MSI-1256F (Squalamine Lactate) in Combination With Verteporfin in Patients With "Wet" Age-Related Macular Degeneration (AMD)," by Genaera Corporation (60 participants; study completed Feb. 2007);
(7) ClinicalTrials.gov Identifier NCT00089830 for "A Safety and Efficacy Study of MSI-1256F (Squalamine Lactate) To Treat "Wet" Age-Related Macular Degeneration," by Genaera Corporation (120 participants; study completed May 2007);
(8) ClinicalTrials.gov Identifier NCT03047629 for "Evaluation of Safety and Tolerability of ENT-01 for the Treatment of Parkinson's Disease Related Constipation (RASMET)," by Enterin Inc. (50 participants; study completed Jun. 14, 2018);
(9) ClinicalTrials.gov Identifier NCT03781791 for "Orally Administered ENT-01 for Parkinson's Disease-Related Constipation (KARMET) (KARMET)," by Enterin, Inc. (72 participants; estimated study completion date of Nov. 2019); and
(10) ClinicalTrials.gov Identifier NCT03938922 for "A Study to Evaluate ENT-01 for the Treatment of Parkinson's Disease Dementia," by Enterin Inc. (estimated enrollment of 40 participants; estimated start date of Jul. 1, 2019; estimated completion date of Feb. 25, 2020).

Aminosterol 1436 is an aminosterol isolated from the dogfish shark, which is structurally related to squalamine (U.S. Patent No. 5,840,936). It is also known as MSI-1436, trodusquemine and produlestan.

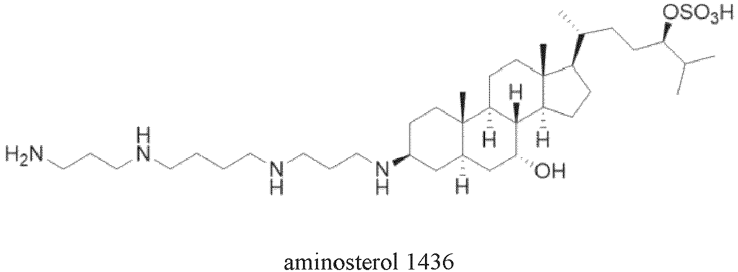

Several clinical trials have been conducted relating to the use of Aminosterol 1436:
(1) ClinicalTrials.gov Identifier NCT00509132 for "A Phase I, Double-Blind, Randomized, Placebo-Controlled Ascending IV Single-Dose Tolerance and Pharmacokinetic Study of Trodusquemine in Healthy Volunteers," by Genaera Corp.;
(2) ClinicalTrials.gov Identifier NCT00606112 for "A Single Dose, Tolerance and Pharmacokinetic Study in Obese or Overweight Type 2 Diabetic Volunteer," by Genaera Corp.;
(3) ClinicalTrials.gov Identifier NCT00806338 for "An Ascending Multi-Dose, Tolerance and Pharmacokinetic Study in Obese or Overweight Type 2 Diabetic Volunteers," by Genaera Corp.; and
(4) ClinicalTrials.gov Identifier: NCT02524951 for "Safety and Tolerability of MSI-1436C in Metastatic Breast Cancer," by DepyMed Inc.

There is a need in the art for novel methods of treatment using aminosterol compounds. The present disclosure satisfies these needs.

### SUMMARY

The present disclosure provides novel methods for aminosterol dosing protocols for treatment of disease.

In another aspect, provided are novel methods for treating cutaneous or mucosal wounds comprising administering at least one pharmaceutically acceptable aminosterol or a salt or derivative thereof.

In one aspect, a method of treating a cutaneous or mucosal wound in a subject in need is provided, the method comprising topically administering to the wound a therapeutically effective amount of a pharmaceutical composition comprising at least one aminosterol or a salt or derivative thereof.

In some embodiments, the wound is an abrasion, a laceration, a puncture wound, an avulsion, or any combination thereof In some embodiments, the wound is present at a surgical site. In some embodiments, the wound is the result of trauma. In some embodiments, the trauma is a blast injury or gun shot wound. In some embodiments, the wound is a burn wound. In some embodiments, the burn would is a first, second, third, or fourth degree burn wound.

In some embodiments, the burn wound is a first-degree burn wound, and wherein the wound is substantially healed in less than about 10, less than about 9.5, less than about 9, less than about 8.5, less than about 8, less than about 7.5, less than about 7, less than about 6.5, less than about 6, less than about 5.5, less than about 5, less than about 4.5, less than about 4, less than about 3.5, or less than about 3 days.

In some embodiments, the burn wound is a second-degree burn wound, and wherein the wound is substantially healed in less than about 3, less than about 2.5, less than about 2, less than about 2.5, or less than about 1 week. In some embodiments, the burn wound is a second-degree burn wound, and wherein the wound is substantially healed in less than about 21, less than about 20, less than about 19, less than about 18, less than about 17, less than about 16, less than about 15, less than about 14, less than about 13, less than about 12, less than about 11, less than about 10, less than about 9, less than about 8, or less than about 7 days.

In some embodiments, the rate or amount of healing of the wound is increased as compared to a similar wound not administered a composition comprising at least one aminosterol or salt or derivative thereof. In some embodiments, the wound healing is measured quantitatively or qualitatively by wound closure or by reference to a clinically recognized scale or tool.

In some embodiments, the method results in at least about 5%, at least about 10%, about 15%, about 20%, about 25%, about 30%, about 40%, about 45%, about 50%, about 55%, about 60%, about 65%, about 70%, about 75%, about 80%, about 85%, about 90%, about 95%, or about 100% increase in the rate or amount of wound healing, as compared to a similar wound not topically treated with a composition comprising at least one aminosterol or salt or derivative thereof.

In some embodiments, the method reduces the severity and/or incidence of one or more complications or side effects associated with wounds, including but not limited to burn wounds, as compared to that observed in the absence of aminosterol administration.

In some embodiments, the complication or side effect is selected from the group consisting of a microbial infection, blood loss, shock, formation of scar tissue, hypothermia, hypovolemia, shock, pulmonary dysfunction, abdominal or extremity compartment syndrome, and cardiac failure in the subject,.

In some embodiments, the incidence and/or severity of the complication or side effect is decreased by about 5%, about 10%, about 15%, about 20%, about 25%, about 30%, about 40%, about 45%, about 50%, about 55%, about 60%, about 65%, about 70%, about 75%, about 80%, about 85%, about 90%, about 95%, or about 100%, as compared to a similar wound not topically treated with a composition comprising at least one aminosterol or salt or derivative thereof, as measured quantitatively or qualitatively by reference to a clinically recognized scale or tool.

In some embodiments, the subject is a member of a patient population characterized by an impediment to normal cutaneous wound healing. In some embodiments, the subject is diabetic.

In some embodiments, the wound is accompanied by a microbial infection. In some embodiments, the microbial infection is a bacterial, viral or yeast infection. In some embodiments, the microbial infection is *Staphylococcus* spp., *Staphylococcus aureus,* methicillin-resistant *Staphylococcus aureus, Pseudomonas* spp., *Pseudomonas aeruginosa, Enterococcus* spp., vancomycin-resistant *Enterococcus, Pseudomonas* spp., *Acinetobacter* spp., non-albicans *Candida* spp., or *Aspergillus* spp.

In some embodiments, the pharmaceutical composition is administered in conjunction with a skin graft. In some embodiments, the pharmaceutical aminosterol composition is administered in conjunction with acellular or cellular dermal matrices. In some embodiments, the pharmaceutical aminosterol composition is administered to a cutaneous wound. In some embodiments, the topical pharmaceutical aminosterol composition is administered to a non-cutaneous wound.

In another aspect, a method of rejuvenating or improving the health of mucosal tissue in a subject in need is provided, the method comprising topically administering to the mucosal tissue a therapeutically effective amount of a pharmaceutical composition comprising at least one aminosterol or a salt or derivative thereof.

In some embodiments, the method results in an increased thickness of the mucosal tissue, as compared to the same type and age of mucosal tissue not treated with an aminosterol composition. In some embodiments, the thickness of the mucosal tissue is increased by about 5%, about 10%, about 15%, about 20%, about 25%, about 30%, about 40%, about 45%, about 50%, about 55%, about 60%, about 65%, about 70%, about 75%, about 80%, about 85%, about 90%, about 95%, or about 100%. In some embodiments, the increase is measured quantitatively or qualitatively by reference to a clinically recognized scale or tool.

In some embodiments, the method results in increasing the expression in the mucosal tissue of one or more proteins having an epithelial barrier function. In some embodiments, the mRNA or gene correlated with protein expression is selected from the group consisting of caspase 14, collagen type XVII alpha 1, corneodesmosin, cornifelin, cystatin E/M, dermokine, desmocollin 1, desmoglein 1 beta, filaggrin, gap junction protein beta 4, gap junction protein beta 6, H19 imprinted maternally expressed transcript, hornerin, kallikrein related-peptidase 7 (chymotryptic stratum, keratin 1, keratin 10, keratinocyte differentiation associated protein, keratinocyte expressed proline-rich, late cornified envelope 1A1, late cornified envelope 1A2, late cornified envelope 1B, late cornified envelope 1C, late cornified envelope 1E, late cornified envelope 1F, late cornified envelope 1G, late cornified envelope 1H, late cornified envelope 1I, late cornified envelope 1J, late cornified envelope 1L, late cornified envelope 1M, late cornified envelope 3C, late cornified envelope 3E, late cornified envelope 3F, lectin galactose binding soluble 7, Loricrin, and sciellin.

In some embodiments, the method results in increasing the expression in the mucosal tissue of one or more proteins having a muscle tissue related function. In some embodiments, the mRNA or gene correlated with protein expression is selected from the group consisting of myoglobin, myosin binding protein C slow-type, myosin heavy polypeptide 1 skeletal muscle, myosin heavy polypeptide 8 skeletal muscle, myosin light chain phosphorylatable fast ske, myosin light polypeptide 3, myozenin 1, myozenin 2, and titin-cap.

In some embodiments, protein expression is increased by at least about 90%. In some embodiments, protein expression is increased by at least about 100%, at least about 200%, at least about 250%, at least about 300%, at least about 350%, at least about 400%, at least about 450%, at least about 500%, at least about 550%, at least about 600%, at least about 650%, at least about 700%, at least about 750%, at least about 800%, at least about 850%, at least about 900%, at least about 950%, or at least about 1000%.

In some embodiments the aminosterol dosage applied to the skin or mucosal surface (a) ranges from about 1 to about 500 mg/day; (b) ranges from about 0.001 mL/cm² to about 15.0 mL/ cm²; (c) ranges from about 0.001 g/cm² to about 15.0 g/ cm²; or (d) ranges from about 0.001 g/cm² to about 15.0 g/ cm².

In some embodiments, the pharmaceutical aminosterol composition is formulated into any pharmaceutically acceptable dosage form. In some embodiments, the pharmaceutical aminosterol composition is emulsified in a gel matrix.

In some embodiments, the pharmaceutical aminosterol composition is formulated into a nasal spray, aerosol, spray, gel, solution, cream, ointment, eye drops, or a transdermal patch. In some embodiments, the pharmaceutical aminosterol composition is administered to a mucosal surface.

In some embodiments, the aminosterol dosage ranges from about 1 to about 500 mg/day. In some embodiments, the pharmaceutical composition comprises an aminosterol is administered in combination with at least one additional active agent to achieve either an additive or synergistic effect.

In some embodiments, the additional active agent is administered via a method selected from the group consisting of concomitantly; as an admixture; separately and simultaneously or concurrently; and/or separately and sequentially.

In some embodiments, the method comprises orally or intranasally administering to the subject a non-topical pharmaceutical composition comprising the same or a different aminosterol, or a salt or derivative thereof.

In some embodiments, (a) the non-topical pharmaceutical composition is administered orally and comprises an aminosterol or a salt or derivative thereof at about 10 mg up to about 500 mg, in single or divided doses; or (b) the non-topical pharmaceutical composition is administered intranasally and comprises an aminosterol at about 0.001 mg to about 6 mg, in single or divided doses.

In some embodiments, each dose of a non-topical pharmaceutical composition comprising an aminosterol or a salt or derivative thereof administered orally is taken on an empty stomach, optionally within two hours of the subject waking. In some embodiments, no food is taken after about 60 to about 90 minutes of taking the aminosterol dose.

**Dosing protocols:** In one aspect, a method of treating, preventing, and/or slowing the onset or progression of a disease or condition, or a symptom thereof, amenable to treatment with an aminosterol or a salt or derivative thereof in a subject in need is provided; the method comprises administering to the subject a therapeutically effective amount of at least one aminosterol or a salt or derivative thereof, wherein the subject is not lying down or while the subject is not lying down.

In another aspect, a method of treating, preventing, and/or slowing the onset or progression of a disease or condition, or a symptom thereof, amenable to treatment with an aminosterol or a salt or derivative thereof in a subject in need is provided; the method comprising requiring the subject to not lie down and then administering to the subject a therapeutically effective amount of at least one aminosterol or a salt or derivative thereof.

In some embodiments, the subject remains not lying down for about 5 minutes after administration of the at least one aminosterol or a salt or derivative thereof. In some embodiments, the subject remains not lying down for about 15, about 30, about 45, or about 60 minutes, or any amount in-between these values, after administration of the at least one aminosterol or a salt or derivative thereof. In some embodiments, the subject remains not lying down for about 5 minutes to about 30 minutes, or any amount in-between these two values, after administration of the at least one aminosterol or a salt or derivative thereof In some embodiments, the subject remains not lying down for about 30 to about 60 minutes, or any amount in-between these two values, after administration of the at least one aminosterol or a salt or derivative thereof.

In some embodiments, the subject experiences fewer side effects, or one or more side effects are reduced in severity and/or frequency, as compared to a subject administered the at least one aminosterol or a salt or derivative thereof, that is lying down or while lying down. In some embodiments, the side effect is selected from the group consisting of gastrointestinal discomfort, diarrhea, nausea, vomiting, and dizziness. In some embodiments, the severity and/or frequency of the side effect is reduced by about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%.

In some embodiments, "lying down" is defined as the subject's body is predominantly flat, or in a horizontal, recumbent, or prostrate position. In some embodiments, the subject is standing up. In some embodiments, the subject is sitting down.

In some embodiments, the aminosterol or a salt or derivative thereof is taken on an empty stomach, optionally within about two hours of the subject waking. In some embodiments, no food is consumed after about 60 to about 90 minutes, or any amount in-between these two values, of administration of the at least one aminosterol or a salt or derivative thereof.

In some embodiments, the therapeutically effective amount of the at least one aminosterol or a salt or derivative thereof comprises: (a) about 0.1 to about 20 mg/kg body weight of the subject; (b) about 0.1 to about 15 mg/kg body weight of the subject; (c) about 0.1 to about 10 mg/kg body weight of the subject; (d) about 0.1 to about 5 mg/kg body weight of the subject; (e) about 0.1 to about 2.5 mg/kg body weight of the subject; (f) about 0.001 to about 500 mg/day.; (g) about 0.001 to about 250 mg/day; (h) about 0.001 to about 125 mg/day; (i) about 0.001 to about 50 mg/day; (j) about 0.001 to about 25 mg/day; or (k) about 0.001 to about 10 mg/day.

In some embodiments, the method further comprises determining a dose of an aminosterol or a salt or derivative thereof for the subject. The aminosterol dose can be determined based on the effectiveness of the aminosterol dose in improving or resolving a symptom of the disease or condition, followed by administering the aminosterol dose to the subject for a defined period of time. The method comprises: (i) identifying the symptom; (ii) identifying a starting aminosterol dose for the subject; and (iii) administering an escalating dose of the aminosterol or a salt or derivative thereof to the subject over a defined period of time until an effective dose for the symptom is identified, wherein the effective dose is the aminosterol dose where improvement or resolution of the symptom is observed, and fixing the aminosterol dose at that level for that particular symptom in that particular subject.

In some embodiments, administering comprises: (a) administration selected from the group consisting of oral, nasal, sublingual, buccal, rectal, vaginal, intravenous, intra-arterial, intradermal, intraperitoneal, intrathecal, intramuscular, epidural, intracerebral, intracerebroventricular, transdermal, pulmonary, inhalation, or any combination thereof; (b) nasal administration; (c) oral administration; or (d) non-oral administration.

In some embodiments, (a) the aminosterol or a salt or derivative thereof is administered orally and the dose of the aminosterol or a salt or derivative thereof for the subject following escalation is fixed at a range of from about 1 mg up to about 500 mg; (b) the aminosterol or a salt or derivative thereof is administered orally and the starting aminosterol dosage ranges from about 1 mg up to about 150 mg/day; (c) the aminosterol or a salt or derivative thereof is administered orally and the dose of the aminosterol or a salt or derivative thereof for the subject following escalation is fixed at a range of from about 25 mg up to about 500 mg/day; and/or (d) the aminosterol or a salt or derivative thereof is administered orally and the dosage of the aminosterol or a salt or derivative thereof is escalated in about 25 mg increments;

In some embodiments, (a) the aminosterol or a salt or derivative thereof is administered intranasally and the starting aminosterol dosage ranges from about 0.001 mg to about 3 mg/day; (b) the aminosterol or a salt or derivative thereof is administered intranasally and the dose of the aminosterol or a salt or derivative thereof for the subject following escalation is fixed at a range of from about 0.001 mg up to about 6 mg/day; (c) the aminosterol or a salt or derivative thereof is administered intranasally and the dose of the aminosterol or a salt or derivative thereof for the subject following escalation is a dose which is subtherapeutic when given orally or by injection; (d) the aminosterol or a salt or derivative thereof is administered intranasally and the dosage of the aminosterol or a salt or derivative thereof is escalated in increments of about 0.1, about 0.2, about 0.25, about 0.3, about 0.35, about 0.4, about 0.45, about 0.5, about 0.55, about 0.6, about 0.65, about 0.7, about 0.75, about 0.8, about 0.85, about 0.9, about 0.95, about 1, about 1.1, about 1.2, about 1.3, about 1.4, about 1.5, about 1.6, about 1.7, about 1.8, about 1.9, or about 2 mg;

In some embodiments, (a) the dosage of the aminosterol or a salt or derivative thereof is escalated every about 3 to about 5 days; (b) the dose of the aminosterol or a salt or derivative thereof is escalated every about 1, about 2, about 3, about 4, about 5, about 6, about 7, about 8, about 9, about 10, about 11, about 12, about 13, or about 14 days; or (c) the dose of the aminosterol or a salt or derivative thereof is escalated about 1×/week, about 2x/week, about every other week, or about 1×/month.

In some embodiments, (a) the fixed dose of the aminosterol or a salt or derivative thereof is administered once per day, every other day, once per week, twice per week, three times per week, four times per week, five times per week, six times per week, every other week, or every few days; (b) the fixed dose of the aminosterol or a salt or derivative thereof is administered for a first defined period of time of administration, followed by a cessation of administration for a second defined period of time, followed by resuming administration upon recurrence of the symptom and/or a different related symptom; (c) the fixed aminosterol dose is incrementally reduced after the fixed dose of aminosterol or a salt or derivative thereof has been administered to the subject for a defined period of time; (d) the fixed aminosterol dose is varied plus or minus a defined amount to enable a modest reduction or increase in the fixed dose; and/or (e) the fixed aminosterol dose is varied plus or minus a defined amount to enable a modest reduction or increase in the fixed dose, wherein the fixed aminosterol dose is increased or decreased by about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20%; and/or (q) the starting dose of the aminosterol or a salt or derivative thereof is higher if the symptom is severe.

In some embodiments, (a) the improvement or resolution of the symptom is measured quantitatively or qualitatively by one or more medically-recognized techniques, scales or tools; and/or (b) the improvement in the symptom is at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100%.

In some embodiments, the symptom is selected from the group consisting of: (a) at least one non-motor aspect of experiences of daily living as defined by Part I of the Unified Parkinson's Disease Rating Scale selected from the group consisting of cognitive impairment, hallucinations and psychosis, depressed mood, anxious mood, apathy, features of dopamine dysregulation syndrome, sleep problems, daytime sleepiness, pain, urinary problems, constipation problems, lightheadedness on standing, and fatigue; (b) at least one motor aspect of experiences of daily living as defined by Part II of the Unified Parkinson's Disease Rating Scale selected from the group consisting of speech, saliva and drooling, chewing and swallowing, eating tasks, dressing, hygiene, handwriting, turning in bed, tremors, getting out of a bed, a car, or a deep chair, walking and balance, and freezing; (c) at least one motor symptom identified in Part III of the Unified Parkinson's Disease Rating Scale selected from the group consisting of speech, facial expression, rigidity, finger tapping, hand movements, pronation-supination movements of hands, toe tapping, leg agility, arising from chair, gait, freezing of gait, postural stability, posture, body bradykinesia, postural tremor of the hands, kinetic tremor of the hands, rest tremor amplitude, and constancy of rest tremor; (d) at least one motor complication identified in Part IV of the Unified Parkinson's Disease Rating Scale selected from the group consisting of time spent with dyskinesias, functional impact of dyskinesias, time spent in the off state, functional impact of fluctuations, complexity of motor fluctuations, and painful off-state dystonia; (e) constipation; (f) depression; (g) cognitive impairment; (h) sleep problem, sleep disorder, or sleep disturbance; (i) circadian rhythm dysfunction; (j) hallucinations; (k) fatigue; (l) REM disturbed sleep; (m) REM behavior disorder; (n) erectile dysfunction; (o) apnea; (p) postural hypotension; (q) correction of blood pressure or orthostatic hypotension; (r) nocturnal hypertension; (s) regulation of temperature; (t) improvement in breathing or apnea; (u) correction of cardiac conduction defect; (v) amelioration of pain; (w) restoration of bladder sensation and urination; (x) urinary incontinence; (y) control of nocturia; (z) schizophrenia; (aa) neurodegeneration, or a neurodegenerative or neurological disorder; (bb) autism; and/or (cc) an inflammatory disease or condition caused by excessive expression or concentration of alpha-synuclein in the subject.

In some embodiments, the symptom is constipation, or optionally wherein the subject suffers from or is at risk of developing constipation, and wherein the method results in treating, preventing, and/or delaying the progression and/or onset of constipation in the subject.

Where the symptom is constipation, in some embodiments, (a) the aminosterol causes the subject to have a bowel movement; (b) the method results in an increase in the frequency of bowel movement in the subject; (c) the method results in an increase in the frequency of bowel movement in the subject and the increase in the frequency of bowel movement is defined as: (i) an increase in the number of bowel movements per week of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%; and/or (ii) a percent decrease in the amount of time between each successive bowel movement selected from the group consisting of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%; (d) the fixed escalated aminosterol dose for constipation is defined as the aminosterol dose that results in a complete spontaneous bowel movement (CSBM) within 24 hours of dosing on at least 2 of 3 days at a given dose; (e) as a result of the method the subject has the frequency of bowel movement recommended by a medical authority for the age group of the subject; and/or (f) the starting aminosterol dose is determined by the severity of the constipation, wherein: (i) if the average complete spontaneous bowel movement (CSBM) or spontaneous bowel movement (SBM) is one or less per week, then the starting aminosterol dose is at least about 150 mg; and/or (ii) if the average CSBM or SBM is greater than one per week, then the starting aminosterol dose is about 75 mg or less.

In some embodiments, the symptom is depression, or optionally wherein the subject suffers from is or at risk of developing depression, and wherein the method results in treating, preventing, and/or delaying the progression and/or onset of depression in the subject.

Where the symptom is depression, in some embodiments, (a) the method results in improvement in the subject's depression, as measured by one or more clinically-recognized depression rating scales; (b) the method results in improvement in the subject's depression, as measured by one or more clinically-recognized depression rating scales and the improvement is in one or more depression characteristics selected from the group consisting of mood, behavior, bodily functions such as eating, sleeping, energy, and sexual activity, and/or episodes of sadness or apathy; and/or (c) the method results in improvement in the subject's depression, as measured by one or more clinically-recognized depression rating scales, and the improvement a subject experiences following treatment is about 5, about 10, about 15, about 20, about 25, about 30, about 35, about 40, about 45, about 50, about 55, about 60, about 65, about 70, about 75, about 80, about 85, about 90, about 95 or about 100%.

In some embodiments, the symptom is cognitive impairment, or optionally wherein the subject suffers from is or at risk of developing cognitive impairment, and wherein the method results in treating, preventing, and/or delaying the progression and/or onset of cognitive impairment in the subject.

Where the symptom is cognitive impairment, in some embodiments, (a) progression or onset of the cognitive impairment is slowed, halted, or reversed over a defined period of time following administration of the aminosterol or a salt or derivative thereof, as measured by a medically-recognized technique; (b) the cognitive impairment is positively impacted by the administration of the aminosterol or a salt or derivative thereof, as measured by a medically-recognized technique; (c) the cognitive impairment is positively impacted by the administration of the aminosterol or a salt or derivative thereof, as measured by a medically-recognized technique and the positive impact on and/or progression of cognitive decline is measured quantitatively or qualitatively by one or more techniques selected from the group consisting of ADASCog, Mini-Mental State Exam(MMSE), Mini-cog test, Woodcock-Johnson Tests of Cognitive Abilities, Leiter International Performance Scale, Miller Analogies Test, Raven's Progressive Matrices, Wonderlic Personnel Test, IQ tests, or a computerized tested selected from Cantab Mobile, Cognigram, Cognivue, Cognision, and Automated Neuropsychological Assessment Metrics Cognitive Performance Test (CPT); and/or (d) the progression or onset of cognitive impairment is slowed, halted, or reversed by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%, as measured by a medically-recognized technique.

In some embodiments, the symptom is a sleep problem, sleep disorder, or sleep disturbance, or optionally wherein the subject suffers from is or at risk of developing a sleep problem, sleep disorder, or sleep disturbance, and wherein the method results in treating, preventing, and/or delaying the progression and/or onset of the sleep problem, sleep disorder, or sleep disturbance in the subject.

Where the symptom is a sleep problem, sleep disorder, or sleep disturbance, in some embodiments, (a) the sleep problem, sleep disorder, or sleep disturbance comprises a delay in sleep onset, sleep fragmentation, REM-behavior disorder, sleep-disordered breathing including snoring and apnea, day-time sleepiness, micro-sleep episodes, narcolepsy, circadian rhythm dysfunction, REM disturbed sleep, or any combination thereof; (b) the sleep problem, sleep disorder, or sleep disturbance comprises REM-behavior disorder, which comprises vivid dreams, nightmares, and acting out the dreams by speaking or screaming, or fidgeting or thrashing of arms or legs during sleep; (c) administration of the aminosterol decreases the occurrence of at least one symptom of the sleep disorder or disturbance; (d) the method results in a positive change in the sleeping pattern of the subject; wherein the positive change is defined as: (i) an increase in the total amount of sleep obtained of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%; and/or (ii) a percent decrease in the number of awakenings during the night selected from the group consisting of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%; (e) as a result of the method the subject obtains the total number of hours of sleep recommended by a medical authority for the age group of the subject; (f) the method results in a positive change in the sleeping pattern of the subject, and optionally wherein the positive change is defined as: (1) an increase in the total amount of sleep obtained of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%; and/or (ii) a percent decrease in the number of awakenings during the night selected from the group consisting of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%; (g) the sleep disorder comprises a loss of diurnal rhythm (Circadian rhythm), and optionally wherein the loss of diurnal rhythm is caused by: (i) dysfunction of the suprachiasmatic nucleus, and optionally wherein the aminosterol reverses the dysfunction of the suprachiasmatic nucleus, restores the diurnal rhythm, and treats the sleep disorder; (ii) dysfunction of the enteric nervous system, and optionally wherein the aminosterol reverses the dysfunction of the enteric nervous system, restores the diurnal rhythm, and treats the sleep disorder; (iii) dysfunction of the olfactory nervous system, and optionally wherein the aminosterol reverses the dysfunction of the olfactory system, restores the diurnal rhythm, and treats the sleep disorder; (iv) dysfunction of circadian rhythm caused by visual loss, and optionally wherein the aminosterol reverses the dysfunction of the circadian rhythm caused by visual loss; (v) dysfunction of circadian rhythm caused by jet lag, and optionally wherein the aminosterol reverses the dysfunction of the circadian rhythm caused by jet lag; and/or (vi) dysfunction of circadian rhythm caused by night-shift work, and optionally wherein the aminosterol reverses the dysfunction of the circadian rhythm caused by night-shift work; and/or (h) wherein the sleep disorder is associated with a neurodegenerative disorder, and wherein: (i) treating the sleep disorder prevents or delays the onset or progression of the neurodegenerative disorder; and/or (ii) the neurodegenerative disorder is selected from the group consisting of Alzheimer's disease, Parkinson's disease, Lewy Body dementia, frontotemporal dementia, supranuclear palsy, multi-system atrophy, Parkinsonism, amyotrophic lateral sclerosis (ALS), Huntington's Disease, schizophrenia, Friedreich's ataxia, Multiple sclerosis (MS), spinal muscular atrophy, progressive nuclear palsy, degenerative processes associated with aging, dementia of aging, Guadeloupian Parkinsonism, spinocerebellar ataxia, and vascular dementia.

In some embodiments, the symptom is schizophrenia, or optionally wherein the subject is at risk of developing, or is suffering from, schizophrenia, and the method results in treating, preventing, and/or delaying the progression and/or onset of schizophrenia in the subject.

Where the symptom is schizophrenia, in some embodiments, (a) the method results in improvement in one or more schizophrenia characteristics or behaviors, as measured using a clinically recognized rating scale; (b) the method results in improvement in one or more schizophrenia characteristics or behaviors, and wherein the schizophrenia characteristics or behaviors are selected from the group consisting of unclear or confusing thinking, reduced social engagement, reduced emotional expression, abnormal social behavior, failure to understand reality, lack of motivation, and hearing voices that others do not hear, as measured using a clinically-recognized scale; (b) the method results in improvement in one or more schizophrenia characteristics or behaviors, and wherein the improvement a subject experiences in one or more schizophrenia characteristics or behaviors following treatment is about 5, about 10, about 15, about 20, about 25, about 30, about 35, about 40, about 45, about 50, about 55, about 60, about 65, about 70, about 75, about 80, about 85, about 90, about 95 or about 100%.

In some embodiments, the symptom is hallucinations, or optionally wherein the subject suffers from, is or at risk of developing, hallucinations, and the method results in treating, preventing, and/or delaying the progression and/or onset of hallucinations in the subject.

Where the symptom is hallucinations, in some embodiments, (a) the hallucinations comprise a visual, auditory, tactile, gustatory or olfactory hallucination; (b) the hallucinations are the result of: (i) a neurodegenerative or neurological disorder, and optionally the neurodegenerative or neurological disorder is selected from the group consisting of Alzheimer's disease, Parkinson's disease, Lewy Body dementia, frontotemporal dementia, supranuclear palsy, multi-system atrophy, Parkinsonism, amyotrophic lateral sclerosis (ALS), Huntington's disease, schizophrenia, Friedreich's ataxia, Multiple sclerosis (MS), spinal muscular atrophy, progressive nuclear palsy, degenerative processes associated with aging, dementia of aging, Guadeloupian Parkinsonism, spinocerebellar ataxia, and vascular dementia; (ii) a neurodegenerative or neurological disorder, wherein the neurodegenerative or neurological disorder is the result of a sleep disorder; a focal brain lesion; a focal brain lesion which is occipital lobe lesions or temporal lobe lesions; a temporal lobe lesion selected from the group consisting of lesions of the uncinate gyrus, cerebral peduncles, and substantia nigra; a diffuse involvement of the cerebral cortex; a diffuse involvement of the cerebral cortex caused by a viral infectious disease; a diffuse involvement of the cerebral cortex caused by a viral infectious disease, wherein the viral infectious disease is selected from the group consisting of acute metabolic encephalopathies, encephalitis, and meningitis; a diffuse involvement of the cerebral cortex caused by a cerebral vasculitis condition; a diffuse involvement of the cerebral cortex caused by a cerebral vasculitis condition, wherein the cerebral vasculitis condition is caused by an autoimmune disorder; a bacterial or viral infection, or a systemic vasculitis; and/or a diffuse involvement of the cerebral cortex caused by a cerebral vasculitis condition, wherein the cerebral vasculitis condition is caused by an autoimmune disorder which is Systemic Lupus Erythematosus (SLE); (ii) a psychiatric disorder, and optionally the psychiatric disorder is selected from the group consisting of bipolar disorder, borderline personality disorder, depression (mixed), dissociative identity disorder, generalized anxiety disorder, major depression, obsessive compulsive disorder, post-traumatic stress disorder, psychosis (NOS), schizoaffective disorder, and schizophrenia; (iii) a neurological disorder; (iv) a brain tumor; (v) a sensory loss, and optionally wherein the sensory loss is visual, auditory, gustatory, tactile, or olfactory; and/or (vi) dysfunction of the enteric nervous system; (c) the method results in a decreased number or severity of hallucinations of the subject, and optionally wherein the decrease in number or severity in hallucinations is defined as a reduction in occurrences or severity of hallucinations selected from the group consisting of by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%.; and/or; (d) the method results in the subject being hallucination-free.

Where the symptom is hallucinations, in some embodiments, the aminosterol: (a) reverses dysfunction caused by the neurodegenerative or neurological disorder and treats and/or prevents the hallucination; (b) reverses dysfunction caused by the psychiatric disorder and treats and/or prevents the hallucination; (c) reverses dysfunction caused by the sensory loss and treats and/or prevents the hallucination; and/or (d) reverses dysfunction of the enteric nervous system and treats and/or prevents the hallucination.

In some embodiments, the symptom is neurodegeneration, or optionally wherein the subject is at risk for developing, or is suffering from, neurodegeneration, and the method results in treating, preventing, and/or delaying the progression and/or onset of neurodegeneration in the subject.

Where the symptom is neurodegeneration, in some embodiments, (a) the neurodegeneration is age-related; (b) the neurodegeneration is correlated with age-related dementia; (c) the neurodegeneration is correlated with a neurodisease; and/or (d) the neurodegeneration is correlated with one or more conditions or diseases selected from the group consisting of Alzheimer's disease, Parkinson's disease, Lewy body dementia, frontotemporal dementia, supranuclear palsy, multi-system atrophy, Parkinsonism, amyotrophic lateral sclerosis (ALS), Huntington's disease, schizophrenia, Friedreich's ataxia, Multiple sclerosis (MS), spinal muscular atrophy, progressive nuclear palsy, degenerative processes associated with aging, dementia of aging, Guadeloupian Parkinsonism, spinocerebellar ataxia, and vascular dementia, (e) progression or onset of the neurodegeneration is slowed, halted, or reversed over a defined period of time following administration of the fixed escalated dose of the aminosterol or a salt or derivative thereof, as measured by a medically-recognized technique, optionally wherein the positive impact and/or progression of neurodegeneration is measured quantitatively or qualitatively by one or more techniques selected from the group consisting of electroencephalogram (EEG), neuroimaging, functional MRI, structural MRI, diffusion tensor imaging (DTI), [18F]fluorodeoxyglucose (FDG) PET, agents that label amyloid, [18F]F-dopa PET, radiotracer imaging, volumetric analysis of regional tissue loss, specific imaging markers of abnormal protein deposition, multimodal imaging, and biomarker analysis; (f) the neurodegeneration is positively impacted by the fixed escalated dose of the aminosterol or a salt or derivative thereof, as measured by a medically-recognized technique, and optionally wherein the positive impact and/or progression of neurodegeneration is measured quantitatively or qualitatively by one or more techniques selected from the group consisting of electroencephalogram (EEG), neuroimaging, functional MRI, structural MRI, diffusion tensor imaging (DTI), [18F]fluorodeoxyglucose (FDG) PET, agents that label amyloid, [18F]F-dopa PET, radiotracer imaging, volumetric analysis of regional tissue loss, specific imaging markers of abnormal protein deposition, multimodal imaging, and biomarker analysis; and/or (g) the progression or onset of neurodegeneration is slowed, halted, or reversed by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%, as measured by a medically-recognized technique.

In some embodiments, the symptom is autism or autism spectrum disorder or a related symptom, or optionally wherein the subject is at risk for developing, or is suffering from, autism or autism spectrum disorder, and the method results in treating, preventing, and/or delaying the progression and/or onset of autism or autism spectrum disorder in the subject.

Where the symptom is autism or autism spectrum disorder or a related symptom, In some embodiments, the method results in improvement: (a) in one or more of the subject's autism or autism spectrum disorder characteristics or behaviors, as measured by a clinically-recognized rating scale; (b) in one or more autism or autism spectrum disorder characteristics or behaviors selected from the group consisting of social skills, repetitive behaviors, speech, nonverbal communication, sensory sensitivity, behavior, social interaction, and communication skills, as measured using a clinically-recognized scale; and/or (c) wherein the improvement a subject experiences following treatment in one or more autism or autism spectrum disorder characteristics or behaviors is about 5, about 10, about 15, about 20, about 25, about 30, about 35, about 40, about 45, about 50, about 55, about 60, about 65, about 70, about 75, about 80, about 85, about 90, about 95 or about 100%.

In some embodiments, the symptom is an inflammatory disease or condition caused by excessive expression or concentration of alpha-synuclein in the subject, or optionally wherein the subject suffers from, is or at risk of developing, an inflammatory disease or condition caused by excessive expression or concentration of alpha-synuclein in the subject, and wherein the method results in treating, preventing, and/or delaying the progression and/or onset of the inflammatory disease or condition caused by excessive expression or concentration of alpha-synuclein in the subject.

Where the symptom is inflammatory disease or condition caused by excessive expression or concentration of alpha-synuclein in the subject, in some embodiments, (a) the method results in a decrease in intensity of inflammation, blood levels of inflammatory markers, inflammatory markers in tissue, number of inflammatory cells in tissue, or any combination thereof, as compared to a control or as compared to the qualitative or quantitative amount from the same patient or subject prior to treatment; (b) the method results in a decrease in concentration of alpha-synuclein in the subject, and optionally wherein the decrease in alpha-synuclein concentration in is measured qualitatively, quantitatively, or semi-quantitatively by one or more methods selected from the group consisting of (i) first determining the concentration of alpha-synuclein in a tissue sample from the subject prior to treatment, followed by: (i) after treatment determining the alpha-synuclein concentration in the same tissue type from the same subject; or (ii) after treatment comparing the alpha-synuclein concentration in the same tissue type to a control; (ii) measuring the intensity of inflammation over time; (iii) measuring the amount of inflammatory markers over time; (iv) measuring the amount of inflammatory markers in blood, plasma, or tissue over time, either qualitatively or quantitatively; (v) measuring the amount of one or more inflammatory marker cytokines in blood, plasma, or tissue over time, either qualitatively or quantitatively; (vi) measuring the amount of one or more plasma markers of inflammation such as TNF, IL-8, or CRP in blood, plasma, or tissue over time, either qualitatively or quantitatively; and (vii) measuring the amount of inflammatory cells in blood, plasma, or tissue over time, either qualitatively or quantitatively; (c) the method results in a decrease in concentration of alpha-synuclein in the subject, wherein the decrease is about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%; (d) the method is applied to a patient population susceptible to excessive expression of alpha-synuclein, resulting in an excessive or high concentration of alpha-synuclein.

In the methods of the invention, each defined period of time (e.g., measuring progression or onset of a symptom to determine whether the symptom is slowed, halted, or reversed over a defined period of time) can be independently selected from the group consisting of about 1 day to about 10 days, about 10 days to about 30 days, about 30 days to about 3 months, about 3 months to about 6 months, about 6 months to about 12 months, and greater than about 12 months.

In another embodiment, in the methods of the invention the aminosterol or a salt or derivative thereof is administered in combination with at least one additional active agent to achieve either an additive or synergistic effect. For example, the additional active agent can be administered via a method selected from the group consisting of (a) concomitantly; (b) as an admixture; (c) separately and simultaneously or concurrently; or (d) separately and sequentially. Further, (a) the additional active agent can be a different aminosterol from that administered in the primary method; and/or (b) the method can comprise administering a first aminosterol which is aminosterol 1436 or a salt or derivative thereof intranasally and administering a second aminosterol which is squalamine or a salt or derivative thereof administered orally.

**Aminosterols:** In all of the methods described herein, the aminosterol or a salt or derivative thereof can be a pharmaceutically acceptable grade of at least one aminosterol or a pharmaceutically acceptable salt or derivative thereof.

For example, the aminosterol or a salt or derivative thereof can: (a) be isolated from the liver of *Squalus acanthias*; (b) be squalamine; (c) be a squalamine isomer; (d) be a squalamine salt; (e) be a phosphate salt of squalamine; (f) be aminosterol 1436; (g) be an aminosterol 1436 isomer; (h) be an aminosterol 1436 salt; (i) be a phosphate salt of aminosterol 1436; (j) comprise a sterol nucleus and a polyamine attached at any position on the sterol, such that the molecule exhibits a net charge of at least + 1; (k) comprise a bile acid nucleus and a polyamine, attached at any position on the bile acid, such that the molecule exhibits a net charge of at least + 1; (l) be a derivative modified to include one or more of the following: (i) substitutions of the sulfate by a sulfonate, phosphate, carboxylate, or other anionic moiety chosen to circumvent metabolic removal of the sulfate moiety and oxidation of the cholesterol side chain; (ii) replacement of a hydroxyl group by a non-metabolizable polar substituent, such as a fluorine atom, to prevent its metabolic oxidation or conjugation; and/or (iii) substitution of one or more ring hydrogen atoms to prevent oxidative or reductive metabolism of the steroid ring system; (m) a derivative of squalamine modified through medical chemistry to improve bio-distribution, ease of administration, metabolic stability, or any combination thereof; and/or (n) be a synthetic aminosterol.

In one embodiment of the methods of the disclosure, the aminosterol is a phosphate salt. In another embodiment, the aminosterol is selected from the group consisting of: 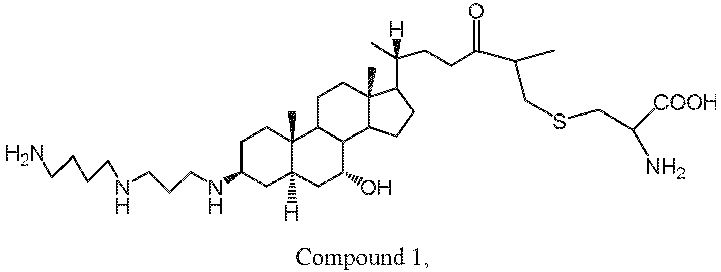 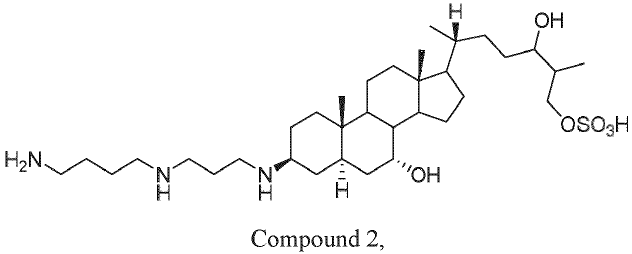 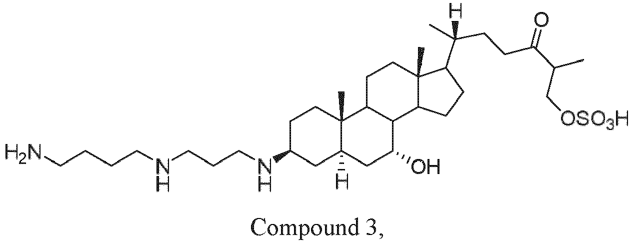 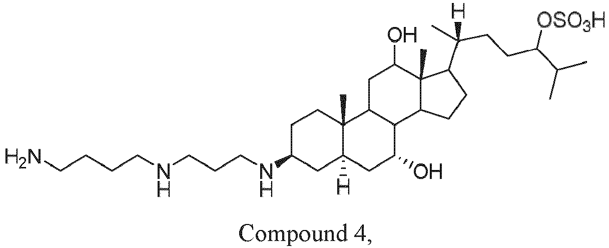 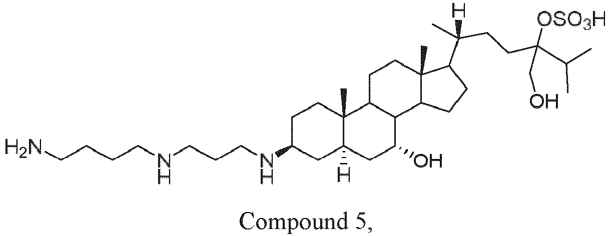 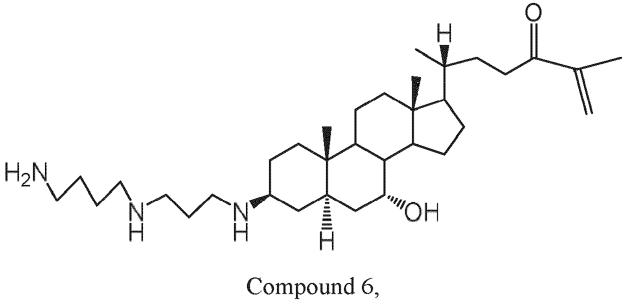 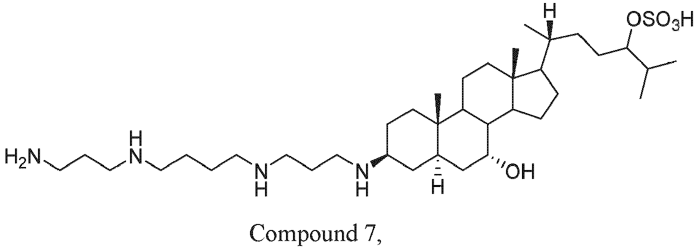 and/or

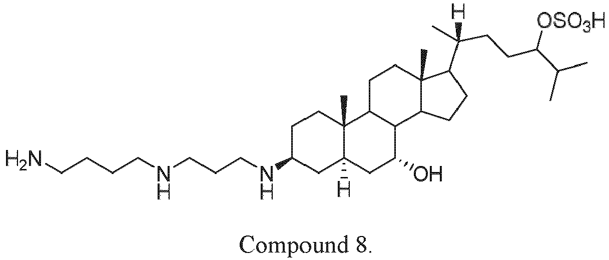

In another embodiment of the invention, the aminosterol is at least one compound from the following: 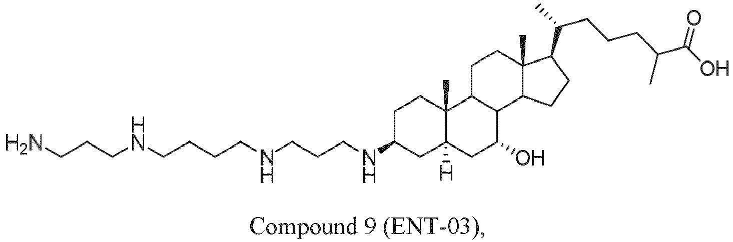 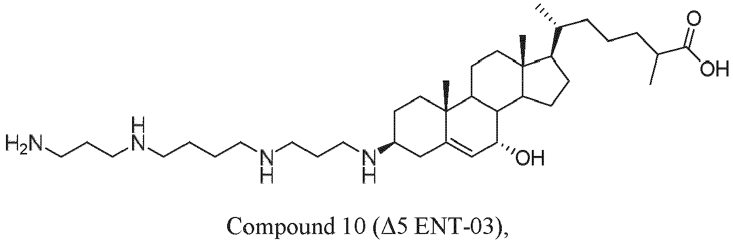 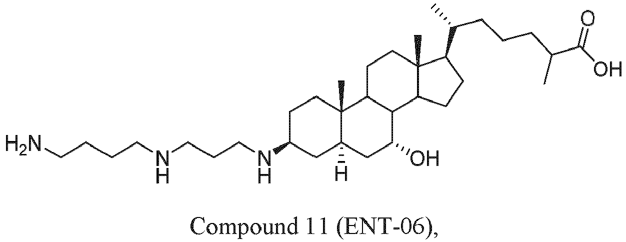 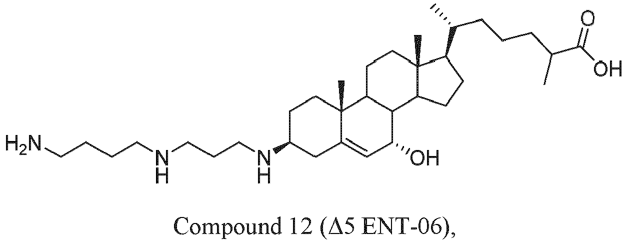 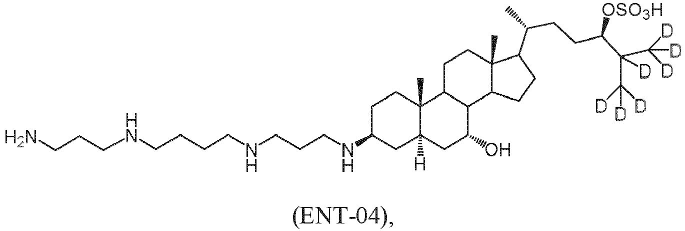 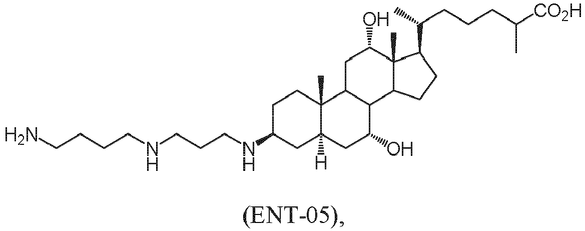 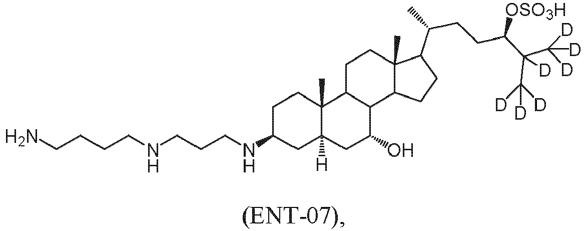 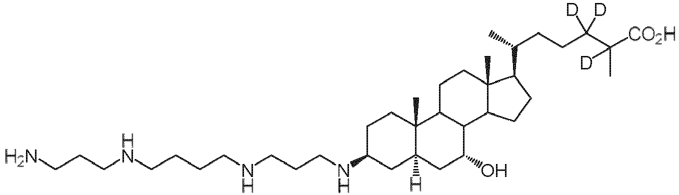 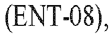 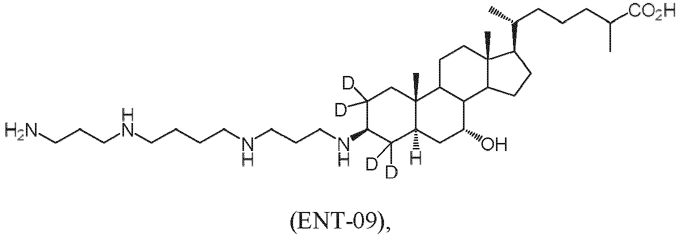 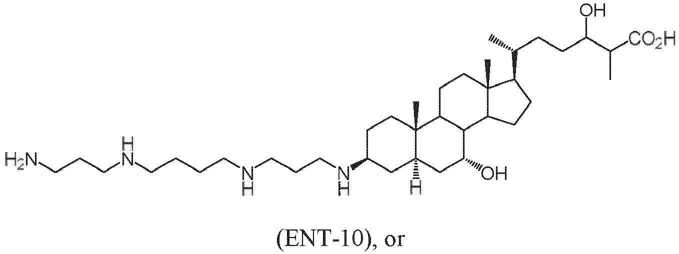 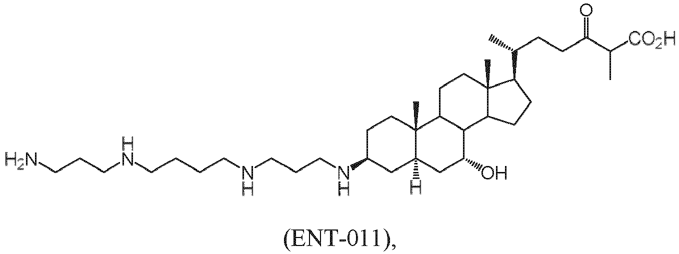 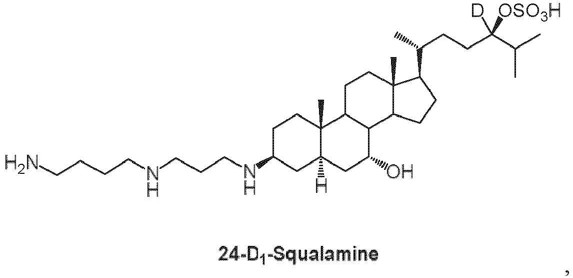 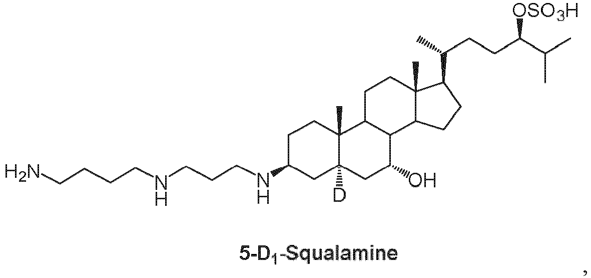 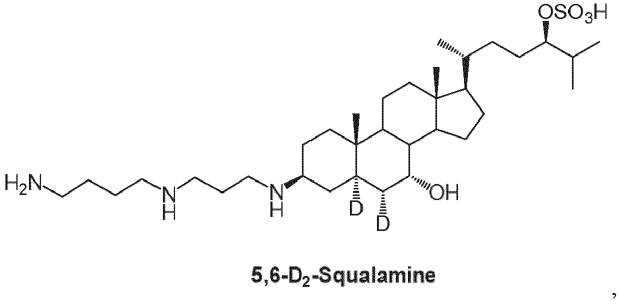 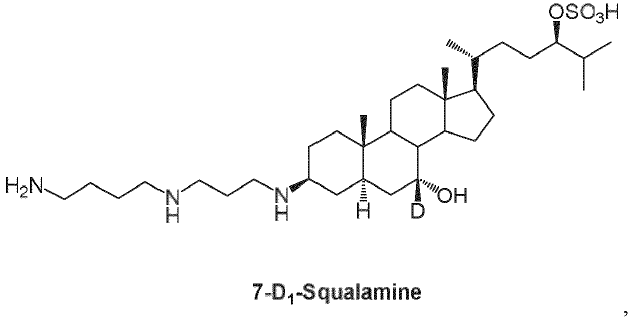 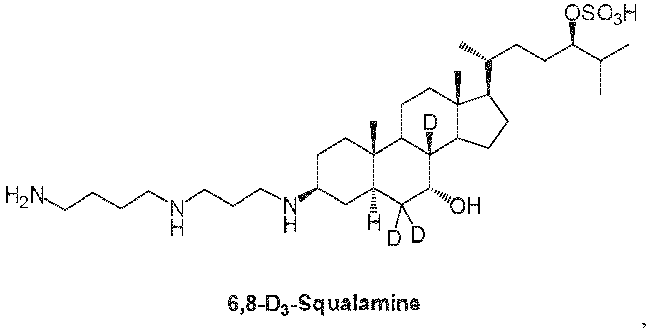 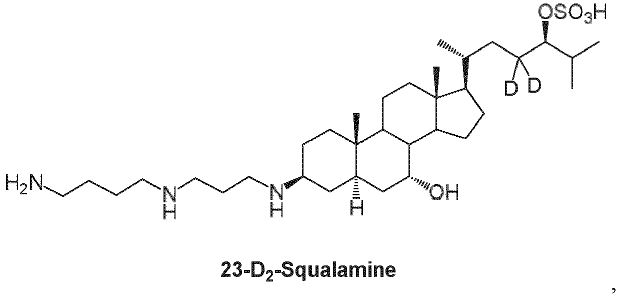 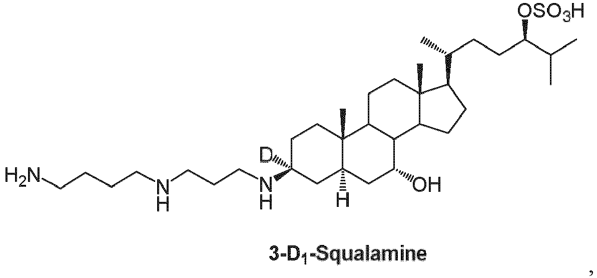 or

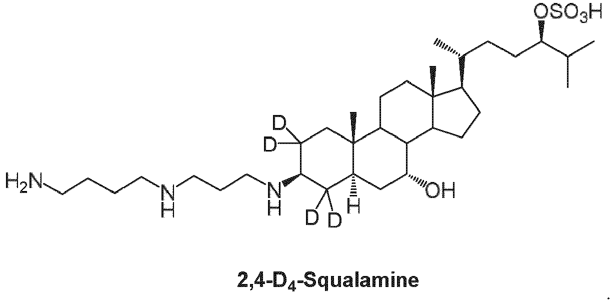

In yet another aspect of the disclosure, the aminosterol can be selected from the group consisting aminosterol 1436 or a pharmaceutically acceptable salt thereof, squalamine or a pharmaceutically acceptable salt thereof, or a combination thereof.

In one embodiment, the aminosterol can be administered as a composition comprising one or more of the following: (a) an aqueous carrier; (b) a buffer; (c) a sugar; and/or (d) a polyol compound. Finally, in the methods of the disclosure the subject can be a human.

Also encompassed by the disclosure is a drug container comprising a label and at least one aminosterol or a salt or derivative thereof, for the treatment of a disease or condition, or a symptom thereof in a subject, wherein the label requires that the subject not be lying down when the aminosterol is administered to the subject. In one aspect, the label requires that the subject is standing up or sitting. In another aspect, the label requires that the administration comprises oral administration. Further, the label can require that the subject is standing up or sitting for about 5 minutes, or any other suitable time period described herein, after administration of the at least one aminosterol or a salt or derivative thereof. In yet another aspect, the label can require that the subject is standing up or sitting for about 15 minutes after administration of the at least one aminosterol or a salt or derivative thereof. Moreover, the label can require that the subject is standing up or sitting for about 30, about 45, or about 60 minutes, or any amount in-between these values, after administration of the at least one aminosterol or a salt or derivative thereof.

In one aspect of the disclosure, relating to a drug container comprising a label and at least one aminosterol or a salt or derivative thereof, for the treatment of a disease or condition, or a symptom thereof in a subject: (a) the symptom is selected from the group consisting of constipation, hallucinations, cognitive impairment, and inflammation; (b) the symptom is correlated with a synucleopathy, a neurodegenerative disease, a neurological disease or disorder, a psychological and/or behavior disorder, or a cerebral or general ischemic disorder or condition; (c) the disease or condition is a synucleopathy, neurodegenerative disease, or neurological disease or disorder; (d) the disease or condition is a psychological and/or behavior disorder; or (e) the disease or condition is a cerebral or general ischemic disorder or condition. In another aspect of the disclosure, (a) the synucleopathy, neurodegenerative disease, or neurological disease or disorder is selected from the group consisting of Parkinson's disease, Alzheimer's disease, schizophrenia, multiple system atrophy, Lewy body dementia, dementia with Lewy bodies, Huntington's Disease, Multiple Sclerosis, Amyotorphic Lateral Sclerosis, Friedreich's ataxia, vascular dementia, spinal muscular atrophy, supranuclear palsy, progressive nuclear palsy, frontotemporal dementia, progressive nuclear palsy, Guadeloupian Parkinsonism, spinocerebellar ataxia, parkinsonism, traumatic brain injury, degenerative processes associated with aging, and dementia of aging; or (b) the psychological or behavior disorder is selected from the group consisting of depression, autism, autism spectrum disorder, down syndrome, Gaucher's disease, Krabbe's disease, lysosomal conditions affecting glycosphingolipid metabolism, ADHD, agitation, anxiety, delirium, irritability, illusion and delusions, amnesia, apathy, bipolar disorder, disinhibition, aberrant motor and obsessive-compulsive behaviors, addiction, cerebral palsy, epilepsy, major depressive disorder, and sleep disorders such as REM sleep behavior disorder (RBD), sleep fragmentation, REM behavior disorder, circadian rhythm dysfunction, sleep apnea, and cognitive impairment; or (c) the cerebral or general ischemic disorder or condition is selected from the group consisting of microangiopathy, intrapartum, cerebral ischemia, cerebral ischemia during/after cardiac arrest or resuscitation, cerebral ischemia due to intraoperative problems, cerebral ischemia during carotid surgery, chronic cerebral ischemia due to stenosis of blood-supplying arteries to the brain, sinus thrombosis or thrombosis of cerebral veins, cerebral vessel malformations, diabetic retinopathy, high cholesterol, myocardial infarction, cardiac insufficiency, cardiac failure, congestive heart failure, myocarditis, pericarditis, perimyocarditis, coronary heart disease, angina pectoris, congenital heart disease, shock, ischemia of extremities, stenosis of renal arteries, diabetic retinopathy, thrombosis associated with malaria, artificial heart valves, anemias, hypersplenic syndrome, emphysema, lung fibrosis, erectile dysfunction, cardiac conduction defects, high blood pressure, low blood pressure, and pulmonary edema.

Both the foregoing summary and the following description of the drawings and detailed description are exemplary and explanatory. They are intended to provide further details, but are not to be construed as limiting. Other objects, advantages, and novel features will be readily apparent to those skilled in the art from the following detailed description.

### BRIEF DESCRIPTION OF THE DRAWINGS

Figure 1: Images of a mucosal layer of the stomach showing a reduced mucosal layer in the 78 week old stomach (Fig. 1B) vs. the younger 20 week old stomach (Fig. 1A).
Figures 2A - 2D: Images of three-week old abrasion in adult male human treated topically with aminosterol at days 1-4 post-treatment.

### DETAILED DESCRIPTION

### I. Overview

In a first aspect, the present application relates to the surprising discovery that topical administration or application of a pharmaceutical composition comprising at least one aminosterol, or a salt or derivative thereof, to a cutaneous or mucosal wound promotes wound healing, as well as related benefits. Thus, the present disclosure provides a method of treating a cutaneous or mucosal wound in a subject in need comprising topically administering to the wound site a therapeutically effective amount of a topical pharmaceutical composition comprising at least one aminosterol or a salt or derivative thereof. In another aspect, provided is a method of rejuvenating mucosal tissue comprising topically administering or applying a pharmaceutical composition comprising at least one aminosterol, or a salt or derivative thereof, to a mucosal surface. The rejuvenation can comprise, for example, thickening of the mucosal tissue, as well as other benefits described herein.

In another aspect, the present application relates to the surprising discovery that administration of aminosterols to a subject that is not lying down more effectively relieves constipation with reduced side effects as compared to aminosterol administration when the subjects is lying down. Also provided are methods of treatment where aminosterol is administered to a subject not lying down.

### A. Topical/mucosal Administration

**Wounds:** An open wound is an injury involving an external or internal break in body tissue, usually involving the skin but as used herein can also involve mucosal tissue. There are four types of open wounds, which are classified depending on their cause: abrasions, lacerations, punctures, and avulsions. The main complication of an open wound is the risk for infection.

An abrasion occurs when skin rubs or scrapes against a rough or hard surface. Road rash is an example of an abrasion. There is usually not a lot of bleeding, but the wound needs to be scrubbed and cleaned to avoid infection. A laceration is a deep cut or tearing of skin. Accidents with knives, tools, and machinery are frequent causes of lacerations. In the case of deep lacerations, bleeding can be rapid and extensive. A puncture is a small hole caused by a long, pointy object, such as a nail or needle. Sometimes, a bullet can cause a puncture wound. Punctures may not bleed much, but these wounds can be deep enough to damage internal organs. A puncture wound also presents a risk of infection. Finally, an avulsion is a partial or complete tearing away of skin and the tissue beneath. Avulsions usually occur during violent accidents, such as body-crushing accidents, explosions, and gunshots. They bleed heavily and rapidly.

Conditions that can develop from an open wound include, for example, lockjaw, which is a condition caused by an infection from the bacteria that cause tetanus. It can cause muscle contractions in your jaw and neck. Another infection that can be caused by an open wound is necrotizing fasciitis, which is a severe soft tissue infection caused by a variety of bacteria including Clostridium and Streptococcus that can lead to tissue loss and sepsis. Finally, cellulitis, which is an infection of skin that is not in immediate contact with the wound, can be the result of an open wound.

Current treatments for open wounds include, for example, tissue adhesives such as skin glue, sutures, or stitches. The methods of the present disclosure can be used in conjunction with any conventional method for treating cutaneous or mucosal injuries or wounds, including burn wounds.

In one aspect of the disclosure, the rate or amount of healing of a wound is increased by about 5%, about 10%, about 15%, about 20%, about 25%, about 30%, about 40%, about 45%, about 50%, about 55%, about 60%, about 65%, about 70%, about 75%, about 80%, about 85%, about 90%, about 95%, or about 100%, as measured quantitatively or qualitatively by reference to a clinically recognized scale or tool, and/or by the amount of wound closure, and as compared to a similar burn wound not treated with an aminosterol composition.

The method can reduce the incidence, frequency or severity of one or more complications associated with wounds, such as microbial infections, blood loss, shock, hypothermia, hypovolemia, tetanus, formation of scar tissue, pulmonary dysfunction, abdominal or extremity compartment syndrome, and/or cardiac failure in the subject. For example, the incidence, frequency, or severity of one or more complications associated with a wound can be decreased by about 5%, about 10%, about 15%, about 20%, about 25%, about 30%, about 40%, about 45%, about 50%, about 55%, about 60%, about 65%, about 70%, about 75%, about 80%, about 85%, about 90%, about 95%, or about 100%, as measured quantitatively or qualitatively by reference to a clinically recognized scale or tool, and as compared to a similar wound not treated with an aminosterol composition.

**Burn wounds:** Burns are one of the most common household injuries, especially among children. Burns are characterized by severe skin damage that causes the affected skin cells to die. There are four types of burns: first-, second-, third-, and fourth-degree. Each degree is based on the severity of damage to the skin, with first-degree being the most minor and third-degree being the most severe. Damage includes: (1) first-degree burns: red, nonblistered skin; (2) second-degree burns: blisters and some thickening of the skin; (3) third-degree burns: widespread thickness with a white, leathery appearance; and (4) fourth-degree burns: all of the symptoms of a third-degree burn and also extending beyond the skin into tendons and bones.

In one aspect, also encompassed is a method of topically treating a burn wound comprising administering a pharmaceutical composition comprising at least one aminosterol or a salt or derivative thereof.

The method can reduce the incidence, frequency or severity of one or more complications associated with burn wounds, such as microbial infections, blood loss, shock, hypothermia, hypovolemia, tetanus, formation of scar tissue, pulmonary dysfunction, abdominal or extremity compartment syndrome, and/or cardiac failure in the subject. For example, the incidence or severity of an infection, blood loss, shock, hypothermia and/or hypovolemia can be decreased by about 5%, about 10%, about 15%, about 20%, about 25%, about 30%, about 40%, about 45%, about 50%, about 55%, about 60%, about 65%, about 70%, about 75%, about 80%, about 85%, about 90%, about 95%, or about 100%, as measured quantitatively or qualitatively by reference to a clinically recognized scale or tool, and as compared to a similar burn wound not treated with an aminosterol composition.

In another aspect of the disclosure, the rate or amount of healing of a burn wound, which can be a first, second, third, or fourth degree burn wound, is increased by about 5%, about 10%, about 15%, about 20%, about 25%, about 30%, about 40%, about 45%, about 50%, about 55%, about 60%, about 65%, about 70%, about 75%, about 80%, about 85%, about 90%, about 95%, or about 100%, as measured quantitatively or qualitatively by reference to a clinically recognized scale or tool, and as compared to a similar burn wound not treated with an aminosterol composition.

First-degree burns cause minimal skin damage. They are also called "superficial burns" because they affect the outermost layer of skin. Signs of a first-degree burn include: redness, minor inflammation, or swelling, pain, dry, and peeling skin occurs as the burn heals. Since this burn affects the top layer of skin, the signs and symptoms disappear once the skin cells shed. First-degree burns usually heal within 7 to 10 days without scarring.

In one aspect of the disclosure, encompassed is a method of topically treating a first-degree burn wound, where the rate of healing is increased. Thus, for example, for a first degree burn treated with an aminosterol composition can heal in less than about 7-10 days, e.g., in about 3, about 3.5, about 4, about 4.5, about 5, about 5.5, about 6, about 6.5, about 7, about 7.5, about 8, about 8.5, or about 9 days. The rate of healing can be measured quantitatively or qualitatively by reference to a clinically recognized scale or tool, and as compared to a similar burn wound not treated with an aminosterol composition.

Conventional treatments for first-degree burns include: soaking the wound in cool water for five minutes or longer, taking acetaminophen or ibuprofen for pain relief, applying lidocaine (an anesthetic) with aloe vera gel or cream to soothe the skin, and/or using an antibiotic ointment and loose gauze to protect the affected area.

Burns have a variety of causes, including: scalding from hot, boiling liquids, chemical burns, electrical burns, fires, including flames from matches, candles, and lighters, and excessive sun exposure. The type of burn is not based on the cause of it. Scalding, for example, can cause all three burns, depending on how hot the liquid is and how long it stays in contact with the skin. Chemical and electrical burns warrant immediate medical attention because they can affect the inside of the body, even if skin damage is minor.

Second-degree burns are more serious because the damage extends beyond the top layer of skin. This type burn causes the skin to blister and become extremely red and sore. Some blisters pop open, giving the burn a wet or weeping appearance. Over time, thick, soft, scab-like tissue called fibrinous exudate may develop over the wound. Some second-degree burns take longer than three weeks to heal, but most heal within two to three weeks without scarring, but often with pigment changes to the skin. The worse the blisters are, the longer the burn will take to heal. In some severe cases, skin grafting is required to fix the damage. Skin grafting takes healthy skin from another area of the body and moves it to the site of the burned skin.

In one aspect of the disclosure, encompassed is a method of topically treating a second-degree burn wound, where the rate of healing is increased. Thus, for example, for a second degree burn treated with an aminosterol composition can heal in less than about 3, about 2.5, about 2, or about 1 week; or less than about 21, about 20, about 19, about 18, about 17, about 16, about 15, about 14, about 13, about 12, about 11, about 10, about 9, about 8, about or 7 days. The rate of healing can be measured quantitatively or qualitatively by reference to a clinically recognized scale or tool, and as compared to a similar burn wound not treated with an aminosterol composition.

Treatments for a second-degree burn generally include: running the skin under cool water for 15 minutes or longer, taking over-the-counter pain medication (acetaminophen or ibuprofen), applying antibiotic cream to blisters, and/or skin grafts.

Excluding fourth-degree burns, third-degree burns are the most severe. They cause the most damage, extending through every layer of skin. There is a misconception that third-degree burns are the most painful. However, with this type of burn the damage is so extensive that there may not be any pain because of nerve damage. Depending on the cause, the symptoms third-degree burns can exhibit include waxy and white color, char, dark brown color, raised and leathery texture, and blisters that do not develop. Without surgery, these wounds heal with severe scarring and contracture. There is no set timeline for complete spontaneous healing for third-degree burns.

As compared with first- and second-degree burns, third-degree burns carry the most risk for complications, such as infections, blood loss, and shock. At the same time, all burns carry the risk of infections because bacteria can enter broken skin.

Tetanus is another possible complication with burns of all levels. Like sepsis, tetanus is a bacterial infection. It affects the nervous system, eventually leading to problems with muscle contractions. As a rule of thumb, every member of your household should receive updated tetanus shots every 10 years to prevent this type of infection.

Severe burns also carry the risk of hypothermia and hypovolemia. Dangerously low body temperatures characterize hypothermia. While this may seem like an unexpected complication of a burn, the condition is actually prompted by excessive loss of body heat from an injury. Hypovolemia, or low blood volume, occurs when your body loses too much blood from a burn.

Extensive damage from severe second-degree and third-degree burns can lead to problems in deep skin tissues, bones, and organs. Patients may require surgery, physical therapy, rehabilitation, and/or lifelong assisted care.

**Mucosal tissue rejuvenation:** A mucous membrane or mucosa is a membrane that lines various cavities in the body and covers the surface of internal organs. It consists of one or more layers of epithelial cells overlying a layer of loose connective tissue. Mucosal tissue is mostly of endodermal origin and is continuous with the skin (cutaneous tissue) at various body openings such as the eyes, ears, inside the nose, inside the mouth, lip, vagina, the urethral opening and the anus. Some mucous membranes secrete mucus, a thick protective fluid. The function of the mucous membrane is to stop pathogens and dirt from entering the body and to prevent bodily tissues from becoming dehydrated.

In one aspect of the disclosure, encompassed is a method of topically treating mucosal tissue with a pharmaceutical composition comprising at least one aminosterol or a salt or derivative thereof. The method can, for example, rejuvenate the mucosal tissue by, for example resulting in an increased thickness of the mucosal tissue. In one embodiment, following aminosterol treatment the thickness of the mucosal tissue is increased by about 5%, about 10%, about 15%, about 20%, about 25%, about 30%, about 40%, about 45%, about 50%, about 55%, about 60%, about 65%, about 70%, about 75%, about 80%, about 85%, about 90%, about 95%, or about 100%, as measured quantitatively or qualitatively by reference to a clinically recognized scale or tool, and as compared to the same type and approximate age of mucosal tissue not treated with an aminosterol composition.

**Gene expression/transcription:** It was surprisingly discovered that topical administration of an aminosterol to mucosal tissue results in an increase in gene/protein transcription (see Example 1) on the order of several magnitudes. This increased expression/transcription is believed to promote regeneration of a depleted mucosal layer. The genes/proteins evaluated are identified in Table 1 below, along with a brief summary of their known function.

Thus, in one aspect of the disclosure, described is a method of topically treating mucosal tissue with a composition comprising at least one pharmaceutically acceptable aminosterol or a salt or derivative thereof, wherein the method results in increasing the expression in the mucosal tissue of one or more proteins. The proteins can have, for example, an epithelial barrier function or relate to muscle tissue related function.

Examples of proteins or genes correlated with epithelial barrier function include, for example, caspase 14, collagen type XVII alpha 1, corneodesmosin, comifelin, cystatin E/M, dermokine, desmocollin 1, desmoglein 1 beta, filaggrin, gap junction protein beta 4, gap junction protein beta 6, H19 imprinted maternally expressed transcript, hornerin, kallikrein related-peptidase 7 (chymotryptic stratum, keratin 1, keratin 10, keratinocyte differentiation associated protein, keratinocyte expressed proline-rich, late cornified envelope 1A1, late cornified envelope 1A2, late cornified envelope 1B, late cornified envelope 1C, late cornified envelope 1E, late cornified envelope 1F, late cornified envelope 1G, late cornified envelope 1H, late cornified envelope 1I, late cornified envelope 1J, late cornified envelope 1L, late cornified envelope 1M, late cornified envelope 3C, late cornified envelope 3E, late cornified envelope 3F, lectin galactose binding soluble 7, Loricrin, and sciellin.

Examples of proteins or genes correlated with muscle tissue related function include, for example, of myoglobin, myosin binding protein C slow-type, myosin heavy polypeptide 1 skeletal muscle, myosin heavy polypeptide 8 skeletal muscle, myosin light chain phosphorylatable fast ske, myosin light polypeptide 3, myozenin 1, myozenin 2, and titin-cap.

In another aspect, protein expression is increased by at least about 90%, at least about 100%, at least about 200%, at least about 250%, at least about 300%, at least about 350%, at least about 400%, at least about 450%, at least about 500%, at least about 550%, at least about 600%, at least about 650%, at least about 700%, at least about 750%, at least about 800%, at least about 850%, at least about 900%, at least about 950%, or at least about 1000%. Increase in protein expression can be measured using any conventional means.

| **Table 1: Genes/proteins evaluated for expression amounts in Example 1** | |
|---|---|
| **Epithelial Barrier Functions** | |
| **Gene/mRNA** | **Function** |
| caspase_14 | This gene encodes a member of the cysteine-aspartic acid protease (caspase) family. Sequential activation of caspases plays a central role in the execution-phase of cell apoptosis. |
| Collagen type XVII alpha 1 | This gene encodes a transmembrane protein which plays a critical role in maintaining the linkage between the intracellular and the extracellular structural elements involved in epidermal adhesion. |
| Corneodesmosin | This gene encodes a protein found in corneodesmosomes, which localize to human epidermis and other cornified squamous epithelia. During maturation of the cornified layers, the protein undergoes a series of cleavages, which are thought to be required for desquamation. |
| Cornifelin | This gene encodes human comifelin protein, which is expressed in the uterus, cervix, and skin |
| cystatin_E/M | This gene encodes cystatin M/E protein, which is a member of a superfamily of evolutionarily-related cysteine protease inhibitors that provide regulatory and protective functions against uncontrolled proteolysis by cysteine proteases. |
| Dermokine | This gene expresses a protein that may act as a soluble regulator of keratinocyte differentiation. |
| Desmocollin 1 | This gene encodes a protein which is a calcium-dependent glycoprotein that is a member of the desmocollin subfamily of the cadherin superfamily. These desmosomal family members, along with the desmogleins, are found primarily in epithelial cells where they constitute the adhesive proteins of the desmosome cell-cell junction and are required for cell adhesion and desmosome formation. |
| Desmoglein 1 beta | This gene encodes Desmoglein-1, which is expressed everywhere in the skin epidermis, but mainly in the superficial upper layers of the skin epidermis. |
| Filaggrin | This gene encodes filaggrin protein, which is essential for the regulation of epidermal homeostasis |
| Gap junction protein beta 4 | This gene encodes a transmembrane connexin protein that is a component of gap junctions Mutations in this gene have been associated with erythrokeratodermia variabilis, progressive symmetric erythrokeratoderma and hearing impairment. |
| Gap junction protein beta 6 | This gene encodes a protein that forms channels called gap junctions that permit the transport of nutrients, charged atoms (ions), and signaling molecules between adjoining cells |
| H19 imprinted maternally expressed transcript | This gene encodes the H19 protein, which has a role in the negative regulation (or limiting) of body weight and cell proliferation. This gene also has a role in the formation of some cancers and in the regulation of gene expression. |
| Homerin | This gene encodes the hornerin protein, which has a function similar to but distinct from that of filaggrin in cornification. |
| Kallikrein related-peptidase 7 (chymotryptic stratum | This gene encodes a member of the kallikrein subfamily of serine proteases that are involved in diverse physiological functions such as skin desquamation, tooth enamel formation, seminal liquefaction, synaptic neural plasticity and brain function. |
| Keratin 1 | This gene encodes the protein Keratin 1, which is produced in keratinocytes in the outer layer of the skin (the epidermis), including the skin on the palms of the hands and soles of the feet. The keratin 1 protein partners with another keratin protein, either keratin 9 or keratin 10, to form molecules called keratin intermediate filaments. |
| Keratin 10 | This gene encodes the protein Keratin 10, which is produced in keratinocytes in the outer layer of the skin (the epidermis). |
| Keratinocyte differentiation associated protein | This gene encodes a protein which may function in the regulation of keratinocyte differentiation and maintenance of stratified epithelia. |
| Keratinocyte expressed proline rich | This gene encodes a proline-rich skin protein possibly involved in keratinocyte differentiation. |
| Late cornified envelope 1A1 | The late cornified envelope (LCE) gene cluster within the epidermal differentiation complex on human chromosome one (mouse chromosome three) contains multiple conserved genes encoding stratum-corneum proteins. |
| Late cornified envelope 1A2 | |
| Late cornified envelope 1B | |
| Late cornified envelope 1C | |
| Late cornified envelope 1E | |
| Late cornified envelope 1F | |
| Late cornified envelope 1G | |
| Late cornified envelope 1H | |
| Late cornified envelope 1I | |
| Late cornified envelope 1J | |
| Late cornified envelope 1L | |
| Late cornified envelope 1M | |
| Late cornified envelope 3C | |
| Late cornified envelope 3E | |
| Late cornified envelope 3F | |
| Lectin galactose binding soluble 7 | Lectin galactoside-binding soluble 3 binding protein is a heavily glycosylated secreted molecule that has been shown previously to be up-regulated in many cancers and has been implicated in tumor metastatic processes, as well as in other cell adhesion and immune functions. |
| Loricrin | Loricrin is a major protein component of the cornified cell envelope found in terminally differentiated epidermal cells |
| Sciellin | This protein localizes to the periphery of cells and may function in the assembly or regulation of proteins in the cornified envelope. |

| **Muscle Tissue** | |
|---|---|
| Myoglobin | Myoglobin, an iron-containing protein in muscle, receives oxygen from the red blood cells and transports it to the mitochondria of muscle cells, where the oxygen is used in cellular respiration to produce energy. |
| Myosin binding protein C slow-type | MYBPC proteins play structural and regulatory roles in muscle function. |
| Myosin heavy polypeptide 1 skeletal muscle | This gene encodes a member of the class II or conventional myosin heavy chains, and functions in skeletal muscle contraction. |
| Myosin heavy polypeptide 8 skeletal muscle | Myosins are a large family of motor proteins that share the common features of ATP hydrolysis (ATPase enzyme activity), actin binding and potential for kinetic energy transduction. |
| Myosin light chain phosphorylatable fast ske | Myosins are a large family of motor proteins that share the common features of ATP hydrolysis (ATPase enzyme activity), actin binding and potential for kinetic energy transduction. |
| Myosin light polypeptide 3 | Myosins are a large family of motor proteins that share the common features of ATP hydrolysis (ATPase enzyme activity), actin binding and potential for kinetic energy transduction. |
| Myozenin 1 | The protein encoded by this gene is primarily expressed in the skeletal muscle, and belongs to the myozenin family. Members of this family function as calcineurin-interacting proteins that help tether calcineurin to the sarcomere of cardiac and skeletal muscle They play an important role in modulation of calcineurin signaling |
| Myozenin 2 | Myozenin-2 is a protein that in humans is encoded by the MYOZ2 gene. The Calsarcin-1 isoform is a muscle protein expressed in cardiac muscle and slow-twitch skeletal muscle, which functions to tether calcineurin to alpha-actinin at Z-discs, and inhibit the pathological cardiac hypertrophic response. |

| **Table 1: Genes/proteins evaluated for expression amounts in Example 1** | |
|---|---|
| titin-cap | Sarcomere assembly is regulated by the muscle protein titin. Titin is a giant elastic protein with kinase activity that extends half the length of a sarcomere. It serves as a scaffold to which myofibrils and other muscle related proteins are attached. This gene encodes a protein found in striated and cardiac muscle that binds to the titin Z1-Z2 domains and is a substrate of titin kinase, interactions thought to be critical to sarcomere assembly Mutations in this gene are associated with limb-girdle muscular dystrophy type 2G. |

**α-synuclein:** Squalamine and aminosterol 1436 inhibit the initiation of aggregation of the α-synuclein protein abundantly present in neurons (Perni et al., 2017; Perni et al. 2018). Aggregation of α-synuclein within the enteric nervous system (ENS), autonomic nerves and brain is characteristic of Parkinson's disease (PD). Accumulation of α-synuclein in the bowel has been observed in the preclinical phase of Parkinson's disease (Hilton et al., 2014). Constipation is also associated with the preclinical phase of Parkinson's disease (Abbott et al., 2001). Constipation is suspected to correlate with the formation of toxic αS aggregates within the enteric nervous system (ENS) (Braak et al. 2003). It is believed that aminosterols are effective against constipation due to their activity against α-synuclein aggregates. α-synuclein is implicated in a variety of other conditions and brain-gut disorders as discussed further below. Thus, it is believed that aminosterols may be effective for the treatment of these other conditions and disorders.

Constipation serves as an early indicator of many neurodiseases such as PD to the extent that it is suspected to correlate with the formation of toxic αS aggregates within the enteric nervous system (ENS) (Braak et al. 2003). As a result of the normal trafficking of αS aggregates from the ENS to the central nervous system (CNS) via afferent nerves such as the vagus (Holmqvist et al. 2014; Svensson et al. 2015), neurotoxic aggregates accumulate progressively within the brainstem and more rostral structures. Inhibiting αS aggregation in the ENS may, thus, reduce the continuing neuro disease process in both the ENS and CNS (Phillips et al. 2008). This relationship between the ENS and CNS is sometimes described herein as "brain-gut" in relation to a class of disorders or the axis of aminosterol activity.

A strategy that targets neurotoxic aggregates of αS in the gastrointestinal tract represents a novel approach to the treatment of PD and other neurodiseases and conditions described herein that may restore the function of enteric nerve cells and prevent retrograde trafficking to the brain. Such actions may potentially slow progression of the disease in addition to restoring gastrointestinal function.

Accordingly, but not to be bound by theory, the methods described herein are expected to apply to the treatment of any of the described symptoms as well as treatment and/or prevention of brain-gut disorders other than PD sharing such symptoms. Examples of such brain-gut disorders include but are not limited to (i) age-related neurodegeneration, (ii) age-related neurodegeneration correlated with age-related dementia, (iii) neurodiseases such as Alzheimer's disease (AD), Huntington's Disease, Multiple Sclerosis, Amyotrophic Lateral Sclerosis (ALS), multiple system atrophy (MSA), schizophrenia, Friedreich's ataxia, vascular dementia, Lewy Body dementia or disease, spinal muscular atrophy, supranuclear palsy, frontotemporal dementia, progressive nuclear palsy, Guadeloupian Parkinsonism, spinocerebellar ataxia, and autism or autism spectrum disorder.

Not to be bound by theory, it is believed that aminosterols target neurotoxic aggregates of αS in the gastrointestinal tract, and restore function of the enteric nerve cells. The now-functional enteric nerve cells prevent retrograde trafficking of proteins, such as alpha-synuclein, to the brain. In addition to restoring gastrointestinal function, this effect is believed to slow and possibly reverse disease progression.

Not to be bound by theory, it is believed that aminosterols improve bowel function by acting locally on the gastrointestinal tract (as supported by the oral bioavailability <0.3%). An orally administered aminosterol such as squalamine, the active ion ofENT-01, stimulates gastrointestinal motility in mice with constipation due to overexpression of human αS (West et al, manuscript in preparation). Perfusion of an aminosterol such as squalamine through the lumen of an isolated segment of bowel from the PD mouse model results in excitation of IPANs (intrinsic primary afferent neuron), the major sensory neurons of the ENS that communicate with the myenteric plexus, increasing the frequency of propulsive peristaltic contractions and augmenting neural signals projecting to the afferent arm of the vagus.

Systemic absorption of the aminosterol following oral administration is negligible as observed in prior studies involving mice, rats and dogs. Prior studies have demonstrated that intravenous administration of squalamine is not associated with increased gastrointestinal motility, despite reaching systemic blood levels one thousand-fold greater than that achieved by orally administered squalamine.

These data suggest that the effect of aminosterols is mediated by local action in the G.I. tract. It is believed that aminosterol administration to a patient that is not lying down results in faster and more effective distribution of aminosterol through the G.I. tract. The topical action would also explain why adverse events are largely confined to the gastrointestinal tract.

Without being bound by any theory, it is believed that aminosterols act directly on receptor sites in the gut rather than being absorbed and acting centrally on the nervous system.

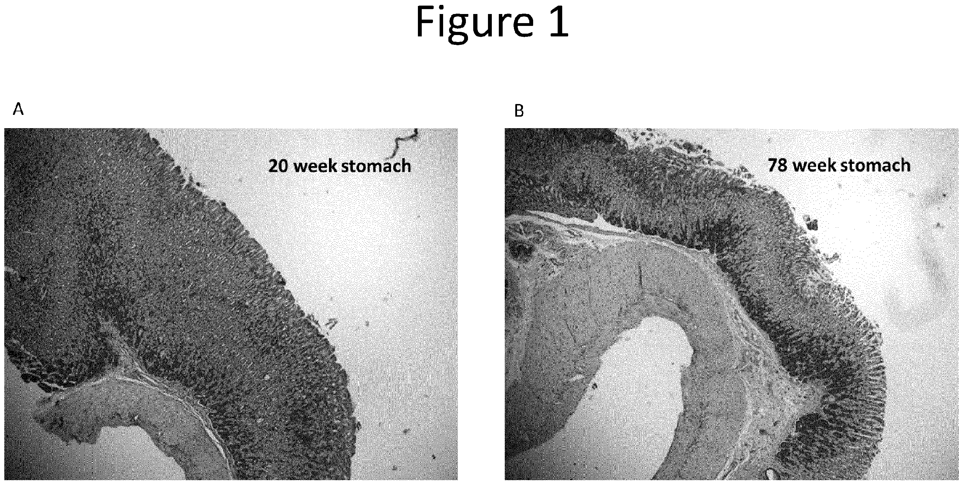

**Experimental Results:** The gastric mucosa is the mucous membrane layer of the stomach, which contains the glands and the gastric pits. In humans, it is about 1 mm thick, and its surface is smooth, soft, and velvety, and includes an epithelial layer. Over time, the thickness of the mucosal layer is depleted as the organism enters old age. This is visually shown in Figs 1A and 1B. Specifically, comparison of the images showing mucosal tissue in the stomach of a young mouse (20 week, Fig. 1A) versus an old mouse (78 week, Fig. 1B) shows a reduced thickness of the mucosal layer in the older specimen. This reduction in mucosa is associated with a reduced RNA transcriptome in the stomach in aged mice vs. young mice.

The observation that aging involves a depletion of gene expression in the gut is evidenced by reduced transcription in young vs. old mice (see Table 2 in the examples, below). In particular, Table 2 shows that transcription of various genes, expressing proteins related to, for example, muscle tissue function and epithelial barrier function, dramatically and significantly decreases over time. Tissues evaluated include stomach, duodenum, jejunum, ileum, caecum, colon, and rectum. The stomach tissues were then sent for histology, and the transcriptomes were analyzed by RNAseq. In particular, Table 2 shows a significant *decrease* in expression of certain genes as the mouse ages. Universally, *every single gene tested* showed a significant decrease in expression in aged tissue as compared to young tissue. This decrease in expression is observed for genes having a known epithelial barrier function as well as for genes associated with muscle tissue.

Surprisingly, topical administration or application of an aminosterol to mucosal tissue resulted in increased expression of *every single epithelial barrier gene and muscle tissue gene measured.* Further, the increases in protein expression were significant and dramatic, and ranged from a low of a 94% increase in protein expression to a high of a 847% increase in protein expression upon topical exposure to an aminosterol. This result suggests that topical application of aminosterols have a rejuvenating effect on mucosa. When taken orally, aminosterols act topically on tissue of the gut as aminosterols have minimal blood absorption (e.g., less than 0.3%).

Topical aminosterol administration to a 3-week old human skin wound (Example 3) demonstrated the therapeutic and regenerative effects of the methods of the disclosure on human cutaneous wounds, as well as an increased rate of healing. Surprisingly, it was observed that topical aminosterol application caused the skin wound to quickly blister over. This is significant because the top layer of the blister facilitates healing and protection of the wound thereunder. Such surprising and unexpected healing results relating to topical administration of an aminosterol have not been shown or described prior to the present disclosure.

In the study described in Example 4, an aminosterol composition was administered to patients and complete spontaneous bowel movement (CSBM) frequency assessed. The study results surprisingly showed that in patients who were administered aminosterol and were standing up, the treatment was about 50% more effective against constipation than when the composition was administered to a patient lying down. Furthermore, aminosterol side effects including nausea, diarrhea, vomiting, and dizziness were reduced in patients administered aminosterol while not lying down as compared to those lying down.

### B. Dosing Protocols

In another aspect of the disclosure, a method is provided for treating, preventing, and/or slowing the onset or progression of a disease or condition, or a symptom thereof, amenable to treatment with an aminosterol or a salt or derivative thereof in a subject in need, comprising administering to the subject a therapeutically effective amount of at least one aminosterol or a salt or derivative thereof, wherein the subject is not lying down.

In another aspect of the disclosure, a method of treating, preventing, and/or slowing the onset or progression of a disease or condition, or a symptom thereof, amenable to treatment with an aminosterol or a salt or derivative thereof in a subject in need is provided. The method comprising administering to the subject a therapeutically effective amount of at least one aminosterol or a salt or derivative thereof, wherein the subject experiences fewer side effects, or one or more side effects are reduced in severity, when the aminosterol or a salt or derivative thereof is taken by the subject when the subject is not lying down.

In one embodiment, the side effect is selected from the group consisting of gastrointestinal discomfort, diarrhea, nausea, vomiting, and dizziness. In one embodiment, the severity of the side effect is reduced by about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%.

The severity of the side effect may be measured by a clinically recognized scale or tool known in the art. Non-limiting examples include The Amer Dizziness Diagnostic Scale (ADDS), The Vertigo Symptom Scale, Dizziness Handicap Inventory, Vestibular Disorders of Daily Living Scale, Activities-specific Balance Confidence, Vertigo Handicap Questionnaire, Vertigo, Dizziness, Imbalance Questionnaire, UCLA Dizziness Questionnaire, Dizzy Factor Inventory, European Evaluation of Vertigo, Meniere's Disease Patients-Oriented Severity Index, The Gastrointestinal Symptom Rating Scale (GSRS), CODA score (see Lee et al, 2014), Vesikari scale, Clark scale, Visual Analogue Scale (VAS), Melzack Questionnaire, Muth Questionnaire, Postoperative Nausea and Vomiting (PONV), MASCC Antiemesis Tool, and Pictoral Baxter Retching Faces Scale.

In one embodiment, "lying down" is defined as the subject's body is predominantly flat, or in a horizontal, recumbent, or prostrate position. In one embodiment, the subject is standing up. In one embodiment, the subject is sitting down. In one embodiment, the aminosterol or a salt or derivative thereof is taken on an empty stomach, optionally within two hours of the subject waking.

In some embodiments, the subject remains not lying down after aminosterol administration for a time period of about 1 minute to about 3 hours. In some embodiments, the time period is about 1 minute to about 10 min, about 10 min to about 20 min, about 20 min to about 30 min, about 30 min to about 40 min, about 40 min to about 50 min, about 50 min to about 60 min, about 60 min to about 70 min, about 70 min to about 80 min, about 80 min to about 90 min, about 90 min to about 100 min, about 100 min to about 110 min, about 110 min to about 120 min, about 120 min to about 130 min, about 130 min to about 140 min, about 140 min to about 150 min, about 150 min to about 160 min, about 160 min to about 170 min, or about 170 min to about 180 min.

In one embodiment, the therapeutically effective amount of the at least one aminosterol, or a salt or derivative thereof comprises about 0.1 to about 20 mg/kg body weight of the subject. In one embodiment, the therapeutically effective amount of the at least one aminosterol, or a salt or derivative thereof comprises about 0.1 to about 15 mg/kg, about 0.1 to about 10 mg/kg, about 0.1 to about 5 mg/kg or about 0.1 to about 2.5 mg/kg body weight of the subject.

In one embodiment, the therapeutically effective amount of the at least one aminosterol, or a salt or derivative thereof comprises about 0.001 to about 500 mg/day. In one embodiment, the therapeutically effective amount of the at least one aminosterol, or a salt or derivative thereof comprises about 0.001 to about 250 mg/day, about 0.001 to about 125 mg/day, about 0.001 to about 50 mg/day, about 0.001 to about 25 mg/day, or about 0.001 to about 10 mg/day.

In one embodiment, the method further comprises (a) determining a dose of an aminosterol or a salt or derivative thereof for the subject, wherein the aminosterol dose is determined based on the effectiveness of the aminosterol dose in improving or resolving the symptom, (b) followed by administering the aminosterol dose to the subject for a defined period of time, wherein the method comprises: (i) identifying the symptom; (ii) identifying a starting aminosterol dose for the subject; and (iii) administering an escalating dose of the aminosterol or a salt or derivative thereof to the subject over a defined period of time until an effective dose for the symptom is identified, wherein the effective dose is the aminosterol dose where improvement or resolution of the symptom is observed, and fixing the aminosterol dose at that level for that particular symptom in that particular subject. Symptoms may include, for example, any of those described in Section II.A below.

In some embodiments, each defined period of time is independently selected from the group consisting of about 1 day to about 10 days, about 10 days to about 30 days, about 30 days to about 3 months, about 3 months to about 6 months, about 6 months to about 12 months, and about greater than 12 months.

A "fixed aminosterol dose," also referred to herein as a "fixed escalated aminosterol dose," which will be therapeutically effective is determined for each patient by establishing a starting dose of an aminosterol composition and a threshold for improvement of a particular symptom of the disease or condition. Following determining a starting aminosterol dosage for a particular patient, the aminosterol dose is then progressively escalated by a consistent amount over consistent time intervals until the desired improvement is achieved; this aminosterol dosage is the "fixed escalated aminosterol dosage" for that particular patient for that particular symptom.

In exemplary embodiments, an orally administered aminosterol dose is escalated every about 3 to about 5 days by about 25 mg until the desired improvement is reached. Symptoms evaluated, along with tools for measuring symptom improvement, may be specifically described below.

The therapeutically effective "fixed aminosterol dose" is then maintained throughout treatment and/or prevention. Thus, even if the patient goes "off drug" and ceases taking the aminosterol composition, the same "fixed dose" is taken with no ramp up period following re-initiation of aminosterol treatment.

Not to be bound by theory, it is believed that the aminosterol dose is dependent on the severity of nerve damage relating to the symptom establishing the "fixed aminosterol dose" threshold - *e.g.* for constipation, the dose may be related to the extent of nervous system damage in the patient's gut.

Administration may be via any route. Non-limiting examples include oral, nasal, sublingual, buccal, rectal, vaginal, intravenous, intra-arterial, intradermal, intraperitoneal, intrathecal, intramuscular, epidural, intracerebral, intracerebroventricular, transdermal, pulmonary, inhalation, or any combination thereof. In some embodiments, administration comprises oral and/or nasal administration.

In one embodiment, (a) the aminosterol or a salt or derivative thereof is administered orally and the dose of the aminosterol or a salt or derivative thereof for the subject, optionally following escalation, is fixed at a range of from about 1 mg up to about 500 mg; (b) the aminosterol or a salt or derivative thereof is administered orally and the aminosterol dosage (optionally the starting dosage) ranges from about 1 mg up to about 150 mg/day; (c) the aminosterol or a salt or derivative thereof is administered orally and the dose of the aminosterol or a salt or derivative thereof for the subject, optionally following escalation, is fixed at a range of from about 25 mg up to about 500 mg/day; and/or (d) the aminosterol or a salt or derivative thereof is administered orally and the dosage of the aminosterol or a salt or derivative thereof is escalated in about 25 mg increments.

In another embodiment, (a) the aminosterol or a salt or derivative thereof is administered intranasally and the aminosterol dosage (optionally the starting dosage) ranges from about 0.001 mg to about 3 mg/day; (b) the aminosterol or a salt or derivative thereof is administered intranasally and the dose of the aminosterol or a salt or derivative thereof for the subject (optionally following escalation) is fixed at a range of from about 0.001 mg up to about 6 mg/day; (c) the aminosterol or a salt or derivative thereof is administered intranasally and the dose of the aminosterol or a salt or derivative thereof for the subject (optionally following escalation) is a dose which is subtherapeutic when given orally or by injection; and/or (d) the aminosterol or a salt or derivative thereof is administered intranasally and the dosage of the aminosterol or a salt or derivative thereof is escalated in increments of about 0.1, about 0.2, about 0.25, about 0.3, about 0.35, about 0.4, about 0.45, about 0.5, about 0.55, about 0.6, about 0.65, about 0.7, about 0.75, about 0.8, about 0.85, about 0.9, about 0.95, about 1, about 1.1, about 1.2, about 1.3, about 1.4, about 1.5, about 1.6, about 1.7, about 1.8, about 1.9, or about 2 mg.

Oral dosage of an aminosterol can range from about 1 to about 500 mg/day, or any amount in-between these two values. Other exemplary dosages of orally administered aminosterols include, but are not limited to, about 5, about 10, about 15, about 20, about 25, about 30, about 35, about 40, about 45, about 50, about 55, about 60, about 65, about 70, about 75, about 80, about 85, about 90, about 95, about 100, about 105, about 110, about 115, about 120, about 125, about 130, about 135, about 140, about 145, about 150, about 155, about 160, about 165, about 170, about 175, about 180, about 185, about 190, about 195, about 200, about 205, about 210, about 215, about 220, about 225, about 230, about 235, about 240, about 245, about 250, about 255, about 260, about 265, about 270, about 275, about 280, about 285, about 290, about 295, about 300, about 305, about 310, about 315, about 320, about 325, about 330, about 335, about 340, about 345, about 350, about 355, about 360, about 365, about 370, about 375, about 380, about 385, about 390, about 395, about 400, about 405, about 410, about 415, about 420, about 425, about 430, about 435, about 440, about 445, about 450, about 455, about 460, about 465, about 470, about 475, about 480, about 485, about 490, about 495, or about 500 mg/day.

Intranasal dosages of an aminosterol are much lower than oral dosages of an aminosterol. Examples of such intranasal aminosterol low dosages include, but are not limited to, about 0.001 to about 6 mg, or any amount in-between these two values. For example, the low dosage of an intranasal administered aminosterol can be about 0.001, about 0.005, about 0.01, about 0.02, about 0.03, about 0.04, about 0.05, about 0.06, about 0.07, about 0.08, about 0.09, about 0.1, about 0.2, about 0.3, about 0.4, about 0.5, about 0.6, about 0.7, about 0.8, about 0.9, about 1, about 1.1, about 1.2, about 1.3, about 1.4, about 1.5, about 1.6, about 1.7, about 1.8, about 1.9, about 2, about 2.1, about 2.2, about 2.3, about 2.4, about 2.5, about 2.6, about 2.7, about 2.8, about 2.9, about 3, about 3.1, about 3.2, about 3.3, about 3.4, about 3.5, about 3.6, about 3.7, about 3.8, about 3.9, about 4, about 4.1, about 4.2, about 4.3, about 4.4, about 4.5, about 4.6, about 4.7, about 4.8, about 4.9, about 5, about 5.1, about 5.2, about 5.3, about 5.4, about 5.5, about 5.6, about 5.7, about 5.8, about 5.9, or about 6 mg/day.

For intranasal (IN) administration, it is contemplated that the aminosterol dosage may be selected such that it would not provide any pharmacological effect if administered by any other route - e.g., a "subtherapeutic" dosage, and, in addition, does not result in negative effects. For example, Aminosterol 1436 is known to have the pharmacological effects of a reduction in food intake and weight loss. Therefore, in the IN methods of the disclosure, if the aminosterol is Aminosterol 1436 or a salt or derivative thereof, then if the IN Aminosterol 1436 dosage is administered via another route, such as oral, IP, or IV, then the Aminosterol 1436 dosage will not result in a noticeable reduction in food intake or noticeable weight loss. Similarly, squalamine is known to produce the pharmacological effects of nausea, vomiting and /or reduced blood pressure. Thus, in the IN methods of the disclosure, if the aminosterol is squalamine or a salt or derivative thereof, then if the IN squalamine dosage is administered via another route, such as oral, IP, or IV, then the squalamine dosage will not result in noticeable nausea, vomiting, and/or a reduction in blood pressure. Suitable exemplary aminosterol dosages are described above.

When determining a "fixed aminosterol dosage" for a particular patient, a patient is started at a lower dose and then the dose is escalated until a positive result is observed for the symptom being evaluated. Aminosterol doses can also be de-escalated (reduced) if any given aminosterol dose induces a persistent undesirable side effect, such as diarrhea, vomiting, or nausea.

The starting aminosterol dose is dependent on the severity of the symptom - *e.g.* for a patient experiencing severe constipation, defined as less than one spontaneous bowel movement (SBM) a week, the starting oral aminosterol dose can be about 150 mg or greater. In contrast, for a patient having moderate constipation, e.g., defined as having more than one SBM a week, the starting aminosterol dose can be about 75 mg. Thus, as an example, a patient experiencing moderate constipation can be started at an aminosterol dosage of about 75 mg/day, whereas a patient experiencing severe constipation can be started at an aminosterol dosage of about 150 mg/day.

In other embodiments, a patient experiencing moderate symptoms of the condition or disease (for the symptom being used to calculate a fixed escalated aminosterol dose) can be started at an oral aminosterol dosage of from about 10 mg/day to about 75 mg/day, or any amount in-between these values. For example, the starting oral aminosterol dosage for a moderate symptom can be about 10, about 15, about 20, about 25, about 30, about 35, about 40, about 45, about 60, about 65, about 70, or about 75 mg per day.

In yet further embodiments, when the patient is experiencing severe symptoms of the condition or disease (for the symptom being used to calculate the fixed escalated aminosterol dose), the patient can be started at an oral aminosterol dosage ranging from about 75 to about 175 mg/day, or any amount in-between these two values. For example, the starting oral aminosterol dosage for a severe symptom can be about 75, about 80, about 85, about 90, about 95, about 100, about 105, about 110, about 115, about 120, about 125, about 130, about 135, about 140, about 145, about 150 about 155, about 160, about 165, about 170, or about 175 mg per day.

In some embodiments, the starting oral aminosterol dose may be about 125 mg or about 175 mg/day; again dependent on the severity of the symptom, such as constipation.

Starting IN aminosterol dosages prior to dose escalation can be, for example, about 0.001 mg to about 3 mg/day, or any amount in-between these two values. For example, the starting aminosterol dosage for IN administration, prior to dose escalation, can be, for example, about 0.001, about 0.005, about 0.01, about 0.02, about 0.03, about 0.05, about 0.06, about 0.07, about 0.08, about 0.09, about 0.1, about 0.15, about 0.2, about 0.25, about 0.3, about 0.35, about 0.4, about 0.45, about 0.5, about 0.55, about 0.6, about 0.65, about 0.7, about 0.75, about 0.8, about 0.85, about 0.9, about 1.0, about 1.1, about 1.25, about 1.3, about 1.4, about 1.5, about 1.6, about 1.7, about 1.75, about 1.8, about 1.9, about 2.0, about 2.1, about 2.25, about 2.3, about 2.4, about 2.5, about 2.6, about 2.7, about 2.75, about 2.8, about 2.9, or about 3 mg/day.

In another embodiment, the dosage of the aminosterol or a salt or derivative thereof is escalated every about 3 to about 5 days; the dose of the aminosterol or a salt or derivative thereof is escalated every about 1, about 2, about 3, about 4, about 5, about 6, about 7, about 8, about 9, about 10, about 11, about 12, about 13, or about 14 days; the dose of the aminosterol or a salt or derivative thereof is escalated about 1×/week, about 2x/week, about every other week, or about 1×/month; the dose of the aminosterol or a salt or derivative thereof is administered once per day, every other day, once per week, twice per week, three times per week, four times per week, five times per week, six times per week, every other week, or every few days; the fixed dose of the aminosterol or a salt or derivative thereof is administered for a first defined period of time of administration, followed by a cessation of administration for a second defined period of time, followed by resuming administration upon recurrence of a symptom being evaluated and/or a related symptom; the aminosterol dose is incrementally reduced after the fixed dose of aminosterol or a salt or derivative thereof has been administered to the subject for a defined period of time; the aminosterol dose is varied plus or minus a defined amount to enable a modest reduction or increase in the fixed dose; (p) the aminosterol dose is varied plus or minus a defined amount to enable a modest reduction or increase in the dose, wherein the aminosterol dose is increased or decreased by about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20%; and/or the starting dose of the aminosterol or a salt or derivative thereof is higher if the symptom being evaluated is severe.

In some embodiments, the symptom is selected from the group consisting of: (a) at least one non-motor aspect of experiences of daily living as defined by Part I of the Unified Parkinson's Disease Rating Scale selected from the group consisting of cognitive impairment, hallucinations and psychosis, depressed mood, anxious mood, apathy, features of dopamine dysregulation syndrome, sleep problems, daytime sleepiness, pain, urinary problems, constipation problems, lightheadedness on standing, and fatigue; (b) at least one motor aspect of experiences of daily living as defined by Part II of the Unified Parkinson's Disease Rating Scale selected from the group consisting of speech, saliva and drooling, chewing and swallowing, eating tasks, dressing, hygiene, handwriting, turning in bed, tremors, getting out of a bed, a car, or a deep chair, walking and balance, and freezing; (c) at least one motor symptom identified in Part III of the Unified Parkinson's Disease Rating Scale selected from the group consisting of speech, facial expression, rigidity, finger tapping, hand movements, pronation-supination movements of hands, toe tapping, leg agility, arising from chair, gait, freezing of gait, postural stability, posture, body bradykinesia, postural tremor of the hands, kinetic tremor of the hands, rest tremor amplitude, and constancy of rest tremor; (d) at least one motor complication identified in Part IV of the Unified Parkinson's Disease Rating Scale selected from the group consisting of time spent with dyskinesias, functional impact of dyskinesias, time spent in the off state, functional impact of fluctuations, complexity of motor fluctuations, and painful off-state dystonia; (e) constipation; (f) depression; (g) cognitive impairment; (h) sleep problem, sleep disorder, or sleep disturbance; (i) circadian rhythm dysfunction; (j) hallucinations; (k) fatigue; (l) REM disturbed sleep; (m) REM behavior disorder; (n) erectile dysfunction; (o) apnea; (p) postural hypotension; (q) correction of blood pressure or orthostatic hypotension; (r) nocturnal hypertension; (s) regulation of temperature; (t) improvement in breathing or apnea; (u) correction of cardiac conduction defect; (v) amelioration of pain; (w) restoration of bladder sensation and urination; (x) urinary incontinence; (y) control of nocturia; (z) schizophrenia; (aa) neurodegeneration, or a neurodegenerative or neurological disorder; (bb) autism or autism spectrum disorder; and/or (cc) an inflammatory disease or condition caused by excessive expression or concentration of alpha-synuclein in the subject.

In some embodiments, (a) the improvement or resolution of the symptom is measured quantitatively or qualitatively by one or more medically-recognized techniques, scales or tools; and/or (b) the improvement in the symptom is at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100%.

### II. Methods of Treatment

### A. Wound Treatment and Mucosal Regeneration

In one aspect, the present disclosure provides a method of treating a cutaneous or mucosal wound in a subject in need comprising topically administering to the wound site a therapeutically effective amount of a topical pharmaceutical composition comprising at least one aminosterol or a salt or derivative thereof.

In another aspect, the disclosure provides a method of preventing a cutaneous or mucosal wound in a subject in need, comprising topically administering to cutaneous or mucosal tissue of the subject a therapeutically effective amount of a topical pharmaceutical composition comprising at least one aminosterol or a salt or derivative thereof.

In some embodiments, the cutaneous wound or mucosal wound is accompanied by one or more symptoms selected from exudate, swelling, erythema, pain, increased local temperature relative to healthy cutaneous or mucosal tissue; and the method ameliorates the one or more symptoms. In some embodiments, the method reduces the occurrence of shock, pulmonary dysfunction, abdominal or extremity compartment syndrome, or cardiac failure in the subject.

The subject may have one or more cutaneous wounds selected from an abrasion, avulsion, laceration, burn, blister, gunshot wound, shrapnel trauma, or puncture. In some embodiments, the cutaneous or mucosal wound is selected from a clean wound (made under sterile conditions, no microorganisms present, i.e., a surgical wound), a contaminated wound (contains pathogens), an infected wound (pathogens are multiplying), or a colonized wound (chronically containing pathogens). In some embodiments, the mucosal or cutaneous wound comprises necrotic tissue. In some embodiments, a pathogen comprises a bacterium, virus, or other microorganism. In some embodiments, the mucosal wound comprises a yeast infection.

In some embodiments, the cutaneous or mucosal wound comprises a microbial infection. In some embodiments, the microbial infection is from one or more of *Staphylococcus* spp., *Staphylococcus aureus,* methicillin-resistant *Staphylococcus aureus, Pseudomonas* spp., *Pseudomonas aeruginosa, Enterococcus* spp., vancomycin-resistant *Enterococcus, Pseudomonas* spp., *Acinetobacter* spp., non-albicans *Candida* spp., and *Aspergillus* spp.

In some embodiments, the cutaneous or mucosal wound comprises a viral infection selected from morbilli, rubella, varicella, parvovirus, erythema subitum, due to herpesvirus 6 and 7, Ebstein Barr virus, pityriasis rosea, enteroviral vesicular stomatitis, Gianotti-Crosti syndrome, asymmetric periflexural exanthem of childhood or APEC, togavirus, bunyavirus, arenavirus, smallpox, cowpox, monkeypox, arboviruses, nairovirus, arenavirus, filovirus, epidermodysplasia verruciformis, AIDS, HIV, immunodeficiency virus infection syndrome, any herpes virus, and flavivirus.

In some embodiments, the cutaneous or mucosal wound is associated with the condition selected from herpes simplex, herpes zoster, herpangina, molluscum contagiosum, warts, verrucas, condyloma, squamous cell papillomas, shingles, toxic epidermal necrolysis, cellulits, or an autoimmune disorder. Exemplary autoimmune disorders which may result in wounds include Bullous pemphigoid, dermatitis herpetiformis, pemphigus vulgaris, mucous membrane pemphigoid, pemphigoid gestationis, epidermolysis bullosa acquisita, linear immunoglobulin A disease, and pemphigus foliaceous.

In some embodiments, the cutaneous or mucosal wound comprises a fungal infection. Exemplary fungal infections include *Candida parapsilosis, Candida tropicalis* , *Trichosporon asahii, Candida albicans,* and *Aspergillus.*

In some embodiments, the method results in at least about a 5% increase in wound healing as measured quantitatively or qualitatively by wound closure or by reference to a clinical scale or tool, as compared to wound healing observed in the absence of aminosterol administration. In some embodiments, at least about 10%, about 15%, about 20%, about 25%, about 30%, about 40%, about 45%, about 50%, about 55%, about 60%, about 65%, about 70%, about 75%, about 80%, about 85%, about 90%, about 95%, or about 100% increase in wound healing results from the method, as measured by a clinical scale or tool.

The clinical scale or tool to assess the increase in wound healing may be selected from, for example, Pressure Sore Status Tool (PSST), Pressure Ulcer Scale for Healing (PUSH), Sessing Scale, Wound Healing Scale (WHS), and Sussman Wound Healing Tool (SWHT).

In some embodiments, the formation of scar tissue is reduced as compared to scar tissue formation observed in the absence of aminosterol administration. The reduction in scar tissue formation may be measured quantitatively or qualitatively by reference to a clinical scale or tool. In some embodiments, the clinical scale or tool for measuring the reduction in scar tissue is selected from Vancouver Scar Scale (VSS), Manchester Scar Scale (MSS), Patient and Observer Scar Assessment Scale (POSAS), Visual Analog Scale (VAS), and Stony Brook Scar Evaluation Scale (SBSES).

In some embodiments, the reduction in scar tissue formation comprises an increase in pliability, a decrease in firmness, a decrease in color that contrasts healthy tissue, a decrease in thickness, and a decrease in perfusion. The increase in pliability, decrease in firmness, decrease in color that contrasts healthy tissue, decrease in thickness, and decrease in perfusion may be at least about 10%, about 15%, about 20%, about 25%, about 30%, about 40%, about 45%, about 50%, about 55%, about 60%, about 65%, about 70%, about 75%, about 80%, about 85%, about 90%, about 95%, or about 100% as measured using a clinical scale or tool.

Pliability may be measured using pneumatonometer and cutometer. Firmness may be measured with a durometer. The decrease in color that contrasts against healthy tissue can be measured by Chromameter (Minolta, Tokyo, Japan), the DermaSpectrometer (cyberDERM, Inc, Media, PA, USA), the Mexameter (Courage-Khazaka, Cologne, Germany), and the tristimulus colorimeter. Thickness may be measured using the tissue ultrasound palpation system (TUPS). Perfusion may be measured using Laser Doppler perfusion imaging.

In some embodiments, the method further comprises administering either orally or intranasally a non-topical pharmaceutical composition (in addition to the topical pharmaceutical composition comprising an aminosterol) comprising the same or a different aminosterol to that in the topical pharmaceutical composition, or a salt or derivative thereof.

In some embodiments, the topical pharmaceutical composition is administered in conjunction with a skin graft. A skin graft may involve the transplantation of cutaneous and/or mucosal tissue. The skin graft may be a thin layer skin graft, a split thickness skin graft, a composite graft (containing skin and underlying tissue such as cartilage), or a full thickness skin graft. A thin layer skin graft includes removal of a thin layer of skin from a healthy part of the body, for example peeling a layer of skin off of the donor portion of the body. A full thickness skin grafts involves pinching and cutting away skin from the donor section. In some embodiments, the skin graft is autologous, isogeneic, allogeneic, xenogeneic or prosthetic (synthetic).

In some embodiments, the topical pharmaceutical composition is administered in conjunction with acellular or cellular dermal matrices. Representative acellular dermal matrices are known in the art and commercially available and include AlloDerm (LifeCell Corporation, USA), Flex HD (Ethicon, USA), DermaMatrix (Synthes, USA), AlloMax (Bard Davol, USA) and SurgiMend (TEI Biosciences, USA).

In another aspect, a method of generating mucosal tissue is provided, comprising contacting existing mucosal tissue with an aminosterol or a salt or derivative thereof. In some embodiments, the contacting is *in vitro.* In some embodiments, the contacting is *in vivo* in a subject in need. In some embodiments, contacting existing mucosal tissue in a subject comprises topically administering to the existing mucosal tissue a therapeutically effective amount of a topical pharmaceutical composition comprising at least one aminosterol or a salt or derivative thereof.

In another aspect, a method of generating cutaneous tissue is provided, comprising contacting cutaneous tissue with an aminosterol or a salt or derivative thereof. In some embodiments, the contacting is *in vitro.* In some embodiments, the contacting is *in vivo* in a subject in need. In some embodiments, contacting cutaneous tissue in a subject comprises topically administering to the cutaneous tissue a therapeutically effective amount of a topical pharmaceutical composition comprising at least one aminosterol or a salt or derivative thereof.

As shown in Example 1, aminosterols increase the transcriptome in the subject. In some embodiments, the methods result in an increase in gene transcription in the subject. In some embodiments, the methods result in an increase in protein synthesis or translation in the subject.

In some embodiments, the gene or protein is selected from one or more of caspase 14, collagen type XVII alpha 1, corneodesmosin, cornifelin, cystatin E/M, dermokine, desmocollin 1, desmoglein 1 beta, filaggrin, gap junction protein beta 4, gap junction protein beta 6, H19 imprinted maternally expressed transcript, homerin, kallikrein related-peptidase 7 (chymotryptic stratum, keratin 1, keratin 10, keratinocyte differentiation associated protein, keratinocyte expressed proline-rich, late cornified envelope 1A1, late cornified envelope 1A2, late cornified envelope 1B, late cornified envelope 1C, late cornified envelope 1E, late cornified envelope 1F, late cornified envelope 1G, late cornified envelope 1H, late cornified envelope 1I, late cornified envelope 1J, late cornified envelope 1L, late cornified envelope 1M, late cornified envelope 3C, late cornified envelope 3E, late cornified envelope 3F, lectin galactose binding soluble 7, loricrin, sciellin, myoglobin, myosin binding protein C slow-type, myosin heavy polypeptide 1 skeletal muscle, myosin heavy polypeptide 8 skeletal muscle, myosin light chain phosphorylatable fast ske, myosin light polypeptide 3, myozenin 1, myozenin 2, and titin-cap.

### B. Diseases, Conditions, and Symptoms

### 1. Constipation

Constipation not only constitutes a major economic burden, but it also significantly affects the quality of life of the individual, contributing to social isolation and depression. Furthermore, the severity of the symptoms correlates negatively with patient reported quality of life. An effective pro-kinetic medication for individuals with constipation would be a useful addition to the currently available treatments for this condition.

It was surprisingly discovered that administration of an aminosterol to a patient that is not lying down, resulted in increased efficacy in producing CSBM and reduced side effects. These results are detailed in Example 4. In some embodiments, "lying down" is defined as the subject's body being predominantly flat, or in a horizontal, recumbent, or in prostrate position

Constipation is defined as a lower than normal frequency of bowel movements in a fixed duration of time (*e.g.* less than 3 bowel movements per week). While often dismissed as strictly a gastrointestinal symptom, constipation is believed to be an early indicator of neurodegenerative disease to the extent that ENS degeneration can be indicative of later CNS degeneration. Indeed, not to be bound by theory, but constipation is believed to be one of the earliest indicators of PD pathology. Accordingly, method embodiments disclosed herein relate to the treatment of constipation or the treatment and/or prevention of an underlying disorder associated with constipation.

Constipation is common in PD and often becomes symptomatic years before the onset of the motor dysfunction and the subsequent diagnosis of PD. There is substantial evidence that the neurodegenerative process associated with PD, namely the accumulation of toxic aggregates of alpha-synuclein, occurs within the enteric nervous system years before they appear within the brain. It is believed that the enteric nervous system (ENS), with its vast surface area, is subject to continuous insults from infectious agents and toxic substances. Although the function of alpha-synuclein is not known, inflammation within the nervous system leads to an increase in its intracellular levels. In individuals with PD the increase in alpha-synuclein leads to the formation of neurotoxic aggregates, perhaps because of a failure by the neuron (due to genetic factors) to effectively dispose of them. The aggregates of alpha-synuclein then traffic along the vagal nerve to the dorsal motor nucleus within the brainstem, and from there to more rostral structures.

The individual with PD suffers from a form of constipation that is believed to be caused principally by delayed transit through the colon. In addition, defecation is often impaired by dysfunction of the PD subject's anorectal reflex. For many individuals, bowel issues represent a significant detriment to quality of life. Failure to effectively manage this problem can also lead to bowel obstruction, especially as the terminal phase of PD approaches. A limited number of therapies have been subjected to clinical trials and they include agents that increase the fluid content of the stool, either by blocking fluid resorption or increasing the osmolar load within the intestine.

Constipation is a major clinical component of PD and is reported to occur in greater than 60% of affected individuals. The pathophysiological basis of constipation in PD is generally believed to be due to delayed transit through the colon. Several studies have demonstrated that transit of stool through the colon of an individual with PD is about 50% that measured in age matched controls. As a consequence, both stool frequency and stool consistency are abnormal in PD. For many patients, as well as those caring for these individuals, constipation remains a significant morbidity associated with the condition.

Few placebo-controlled clinical trials have been conducted in the PD population to assess the efficacy of therapeutics that could be of value. Addition of fiber to the diet, although increasing stool volume, is reported to have no effect on colon transit time. An osmotic laxative, polyethylene glycol (Magrogol) has been studied in a small placebo controlled clinical trial of individuals with mild constipation, and shown to provide benefit with respect to stool frequency and consistency. A short term placebo controlled trial of Lubiprostone, a chloride channel activator which increases intestinal fluid secretion, was only effective in about 50% of those treated, and resulted in passage of loose stools/diarrhea in place of constipation. Furthermore, Lubiprostone delays gastric emptying, a function already compromised in PD.

The pathophysiology of the gastrointestinal (GI) dysfunction in PD involves deposition of alpha-synuclein within both the ENS as well as within the brainstem. For reasons that remain unknown alpha-synuclein, which is a protein normally produced in neurons, forms neurotoxic intracellular aggregates in PD. Numerous studies suggest that the alpha-synuclein aggregate formation begins in the ENS of the PD individual many years before the onset of the motor symptoms. As a consequence of the normal retrograde neuronal trafficking that occurs within the vagus nerve, toxic aggregates are transported from the neurons of the ENS to the dorsal motor nucleus of the vagus, and then, gradually to sites within the brain that are involved in physical movement and balance. Because the constipation is fundamentally of an acquired neurodegenerative nature, it differs from other forms of this condition.

Several tools used to measure and evaluate the effect of aminosterol treatment on constipation, including for example: (1) Rome-IV Criteria for Constipation (7 criteria, with constipation diagnosis requiring two or more of the following: (i) straining during at least 25% of defecations, (ii) lumpy or hard stools in at least 25% of defecations, (iii) sensation of incomplete evacuation for at least 25% of defecations, (iv) sensation of anorectal obstruction/blockage for at least 25% of defecations; (v) manual maneuvers to facilitate at least 25% of defecations; (vi) fewer than 3 defecations per week; and (vii) loose stools are rarely present without the use of laxatives; (2) Constipation - Ease of Evacuation Scale (from 1-7, with 7 = incontinent, 4 = normal, and 1 = manual disimpaction); (3) Bristol Stool Chart, which is a patient-friendly means of categorizing stool characteristics (assessment of stool consistency is a validated surrogate of intestinal motility) and stool diary; (4) Unified Parkinson's Disease Scale (UPSRS), section 1.11 (Constipation Problems); (4) Patient Assessment of Constipation Symptoms (PAC-SYM); and (4) Patient Assessment of Constipation Quality of Life (PAC-QOL).

Examples of characteristics of constipation that can be positively affected by the method of the disclosure include, but are not limited to, frequency of constipation, duration of constipation symptoms, bowel movement frequency, stool consistency, abdominal pain, abdominal bloating, incomplete evacuation, unsuccessful attempts at evacuation, pain with evacuation, and straining with evacuation. Potentially all of these characteristics can be positively impacted by the methods of the disclosure. Further, assessments of these characteristics are known in the art, *e.g.* spontaneous bowel movements (SBMs)/week, stool consistency (Bristol Stool Form Scale) (Lewis and Heaton 1997; Heaton et al. 1992), ease of passage (Ease of Evacuation Scale) (Andresen et al. 2007), rescue medication use and symptoms and quality of life related to bowel function (PAC-SYM (Frank et al. 1999) and PAC-QOL (Marquis et al. 2005)).

The methods of using a therapeutically effective amount of an aminosterol for treating, preventing, and/or slowing the onset or progression constipation preferably result in an increase in the number of spontaneous bowel movements per week and/or an improvement in other stool conditions. The increase can be, for example, an increase of between 1 to 3 spontaneous bowel movements in a week, or, optionally, full restoration of regular bowel function.

A study was conducted measuring the effects of aminosterol administration on constipation. In the study, 80% of subjects responded to aminosterol treatment with improved bowel function, with the cumulative response rate increasing in a dose-dependent fashion from 25% at 25 mg to a maximum of 80% at 200 mg. In a second stage of the study, the response rate increased in a dose-dependent fashion from 26% at 75 mg to 85.3% at 250 mg. The dose required for a bowel response was patient-specific and varied from 75 mg to 250 mg. The median efficacious dose was 100 mg.

The average CSBM/week increased from 1.2 at baseline to 3.8 at fixed dose (216% improvement) and SBM increased from 2.6 at baseline to 4.5 at fixed dose (73% improvement). Use of rescue medication decreased from 1.8/week at baseline to 0.3 at fixed dose (83% decrease). Consistency based on the Bristol stool scale also improved, increasing from mean 2.7 to 4.1 (52% improvement) and ease of passage increased from 3.2 to 3.7 (16% improvement). Subjective indices of wellbeing (PAC-QOL) and constipation symptoms (PAC-SYM) also improved during treatment.

The dose that proved efficacious in inducing a bowel response was strongly related to constipation severity at baseline; patients with baseline constipation of < 1 CSBM/week required higher doses for a response (mean 192 mg) than patients with ≥ 1 CSBM/week (mean 120 mg).

This study confirms the hypothesis that gastrointestinal dysmotility in PD results from the progressive accumulation of αS in the ENS, and that aminosterol treatment can restore neuronal function by displacing αS and stimulating enteric neurons. These results demonstrate that the ENS in PD is not irreversibly damaged and can be restored to normal function.

In one embodiment, treatment of a subject having constipation with an aminosterol in a method described herein results in an improvement of one or more characteristics of constipation. The improvement can be, for example, about 5, about 10, about 15, about 20, about 25, about 30, about 35, about 40, about 45, about 50, about 55, about 60, about 65, about 70, about 75, about 80, about 85, about 90, about 95, about 100, about 110, about 120, about 130, about 140, about 150, about 160, about 170, about 180, about 190, about 200, about 210, about 220, about 230, about 240, about 250, about 260, about 270, about 280, about 290, about 300, about 325, about 350, about 375 or about 400%. Examples of constipation characteristics that can be improved by the methods of the disclosure include, but are not limited to, frequency of constipation, duration of constipation symptoms, bowel movement frequency, stool consistency, abdominal pain, abdominal bloating, incomplete evacuation, unsuccessful attempts at evacuation, pain with evacuation, and straining with evacuation. Measurement of a constipation characteristic can be done using any clinically recognized scale or tool.

In some embodiments, the method results in at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100%: (a) increase in frequency of bowel movement; (b) decrease in time between successive bowel movements; or (c) increase in mass of eliminated fecal matter per bowel movement.

For example, for patients with severe constipation, a starting oral aminosterol dosage can be from 75 mg up to about 300 mg, or any amount in-between these two values. In other embodiments, the starting oral aminosterol dosage for severely constipated patients can be, for example, about 75, about 80, about 85, about 90, about 95, about 100, about 105, about 110, about 115, about 120, about 125, about 130, about 135, about 140, about 145, about 150, about 155, about 160, about 165, about 170, about 175, about 180, about 185, about 190, about 195, about 200, about 205, about 210, about 215, about 220, about 225, about 230, about 235, about 240, about 245, about 250, about 255, about 260, about 265, about 270, about 275, about 280, about 285, about 290, about 295, or about 300 mg. A "fixed escalated" oral aminosterol dose for a severely constipated patient is likely to range from about 75 mg up to about 500 mg. A positive effect is defined as a dose that resulted in a CSBM within 24 hours of dosing on at least 2 of 3 days at a given dose.

For patients with less severe constipation, oral aminosterol dosing is started at about 10 to about 75 mg, or any amount in-between these two values as described herein. For example, starting oral aminosterol dosage for patients with moderate to mild constipation can be about 1, about 5, about 10, about 15, about 20, about 25, about 30, about 35, about 40, about 45, about 50, about 55, about 60, about 65, about 70, up to less than or equal to about 75 mg. A fixed escalated oral aminosterol dose for a mild or moderately constipated patient is likely to range from about 5 mg up to about 350 mg, or any amount in-between these two values as described herein.

### 2. Hallucinations

A hallucination is a sensory impression or perception of an object or event, in any of the 5 senses (sight, touch, sound, smell, or taste) that has no basis in external stimulation. Hallucinations can have debilitating impact on the subject's health and life by causing harm to self or others, by making it difficult for the subject to function normally in everyday situations, and by causing sleep disruption. Examples of hallucinations include "seeing" someone not there (visual hallucination), "hearing" a voice not heard by others (auditory hallucination), "feeling" something crawling up your leg (tactile hallucination), "smelling" (olfactory), and "tasting" (gustatory). Other examples of hallucination types include hypnagogic hallucination (a vivid, dreamlike hallucination occurring at sleep onset), hypnopompic hallucination (a vivid, dreamlike hallucination occurring on awakening), kinesthetic hallucination (a hallucination involving the sense of bodily movement), and somatic hallucination a hallucination involving the perception of a physical experience occurring within the body.

In one embodiment, administration of an aminosterol composition is to a patient that is not lying down. In some embodiments, "lying down" is defined as the subject's body being predominantly flat, or in a horizontal, recumbent, or in prostrate position.

Hallucinations can be a result of psychiatric conditions or correlated with diseases, such as a neurodisease. Hallucinations, especially auditory hallucinations, are characteristic of certain psychiatric conditions such as schizophrenia, occurring in up to 70-80% of subjects. They also occur in 30-50% of individuals with borderline personality disorder. Auditory hallucinations can take control of actions or behavior and elicit violent defensive behavior or alternatively lead to self-harming behavior. They can also occur in post-partum psychosis. Auditory hallucinations can less commonly occur in severely depressed patients or even in mania. Substance abuse can also be associated with visual hallucinations. Alcohol intoxication or withdrawal, post-traumatic stress disorder (PTSD) and bereavement can also be associated with visual hallucinations.

Hallucinations can be a result of neurological disorders. In one embodiment the neurological disorder is a brain tumor. In some embodiments, the "focal brain lesions." Formed and unformed visual hallucinations can occur in the presence of temporal and occipital lobe lesions. Occipital lobe lesions typically produce simple geometric patterns or "strings of circles like a bunch of grapes" or stars which can follow the gaze (palinopsia), whereas temporal lobe lesions are associated with complex, formed hallucinations. Temporal lobe lesions and especially lesions of the uncinate gyrus are typically associated with olfactory and gustatory hallucinations. Lesions of the cerebral peduncles and substantia nigra are associated with "peduncular hallucinosis" or colorful vivid images. In some embodiments, the hallucinations are a result of diffuse involvement of the cerebral cortex. In some embodiments, of diffuse involvement. Acute metabolic encephalopathies and encephalitis caused by viral infections or diseases associated with a cerebral vasculitis such as Systemic Lupus Erythematosus (SLE) can cause visual hallucinations.

In some cases, hallucination is the result of a psychiatric or neurological disorder. The aminosterol composition can, for example, reverse the dysfunction of the psychiatric or neurological disorder and treat the hallucination. The psychiatric disorder can be, for example, selected from the group consisting of Bipolar disorder, Borderline personality disorder, Depression (mixed), Dissociative identity disorder, Generalized anxiety disorder, Major depression, Obsessive compulsive disorder, Post-traumatic stress disorder, Psychosis (NOS), Schizoaffective disorder, and Schizophrenia.

In other cases, hallucinations can be the result of a neurological disorder. The neurological disorder can be, for example, the result of (a) a brain tumor, (b) a sleep disorder such as narcolepsy, or (c) a focal brain lesion, such as occipital lobe lesions or temporal lobe lesions. In an exemplary embodiment, the temporal lobe lesion can be lesions of the uncinate gyrus, cerebral peduncles, or substantia nigra. The neurological disorder can be, for example, the result of (d) a diffuse involvement of the cerebral cortex, such as that caused by a viral infectious disease.

The diffuse involvement of the cerebral cortex can be a result of a cerebral vasculitis condition, and the viral infectious disease can be, for example, acute metabolic encephalopathies, encephalitis, or meningitis. The cerebral vasculitis condition can be caused by an autoimmune disorder, a bacterial or viral infection, or a systemic vasculitis. The autoimmune disorder can be, for example, Systemic Lupus Erythematosus (SLE).

Alternatively, hallucinations can be the result of a neurodegenerative disorder. For example, the neurodegenerative disorder can be, for example, such as Parkinson's disease (PD), supranuclear palsy, multi-system atrophy, Parkinsonism, Alzheimer's disease, frontotemporal dementia, amyotrophic lateral sclerosis (ALS), Huntington's Disease, schizophrenia, Friedreich's ataxia, Multiple sclerosis (MS), Lewy Body dementia or disease, spinal muscular atrophy, frontotemporal dementia, progressive nuclear palsy, Guadeloupian Parkinsonism, spinocerebellar ataxia, or vascular dementia. In a preferred embodiment, the aminosterol reverses the dysfunction of the neurodegenerative disorder and treats the hallucination.

Further still, hallucinations may be caused by a sensory loss. The sensory loss can be, for example, visual, auditory, gustatory, tactile, or olfactory. In a preferred embodiment, the aminosterol reverses the dysfunction of the sensory loss and treats the hallucination. In a preferred embodiment, the aminosterol reverses the dysfunction of the enteric nervous system and treats the hallucination.

The methods of using a therapeutically effective amount of an aminosterol to treat and/or prevent hallucinations preferably result in a decrease in hallucinations. The decrease can be, for example, a reduction in occurrences of hallucinations by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%. The methods of the disclosure may also result in the subject being hallucination-free. The hallucination can comprise, for example, a visual, auditory, tactile, gustatory or olfactory hallucination. The improvement can be measured using one or more medically recognized techniques.

In one embodiment, the one or more medically recognized techniques is selected from the group consisting of Chicago Hallucination Assessment Tool (CHAT), The Psychotic Symptom Rating Scales (PSYRATS), Auditory Hallucinations Rating Scale (AHRS), Hamilton Program for Schizophrenia Voices Questionnaire (HPSVQ), Characteristics of Auditory Hallucinations Questionnaire (CAHQ), Mental Health Research Institute Unusual Perception Schedule (MUPS), positive and negative syndrome scale (PANSS), scale for the assessment of positive symptoms (SAPS), Launay-Slade hallucinations scale (LSHS), the Cardiff anomalous perceptions scale (CAPS), and structured interview for assessing perceptual anomalies (SIAPA).

Other tools used to measure and evaluate the effect of aminosterol treatment on hallucinations, include for example: (1) The University of Miami Parkinson's disease Hallucinations Questionnaire (UM-PDHQ); (2) Unified Parkinson's Disease Scale (UPSRS), section 1.2 (Hallucinations and Psychosis); and (3) direct questioning.

### 3. Sleep Disturbance/Sleep Problems (e.g., REM Disturbed Sleep or Circadian Rhythm Dysfunction)

Normal sleep is critically important for the proper functioning of many organ systems, the most important of which is the brain. Disturbances in normal sleep patterns are closely associated with the normal aging process, with the development of cognitive impairment, with impaired memory deposition and consolidation and with the occurrence of neurodevelopmental, neuroaffective and neurodegenerative disorders. The alternating pattern of sleep and wakefulness occurring every 24 hours is known as the circadian rhythm. The rhythm is set by the "zeitgeber" (time setter), an entity known as the suprachiasmatic nucleus (SCN) and located in the hypothalamus. The SCN is normally "entrained" or synchronized by the external light-dark cycle. This relationship between external light and dark and the sleep wake cycle synchronized to it by the SCN can be over ridden during periods of hunger by neural signals emanating in the gut and relayed to the hypothalamus. The circadian sleep-wake cycle can also shift in response to changes in external light-dark cycles, such as the desynchronization that occurs during travel from one time zone to another (jet-lag). Under such circumstances, a progressive adjustment occurs until the SCN is resynchronized with the external light-dark cycle. A similar "phase-shift" and adjustment occurs in night-shift workers.

In one embodiment, administration of an aminosterol composition is to a patient that is not lying down. In some embodiments, "lying down" is defined as the subject's body being predominantly flat, or in a horizontal, recumbent, or in prostrate position.

Under normal circumstances, the properly functioning SCN, synchronized to the external light-dark cycle and to neural signals emanating from the enteric nervous system, will regulate the sleep-wake cycle by sending neural and chemical signals to the surrounding structures and to portions of the brain stem involved in sleep and wakefulness. An individual with a properly functioning hypothalamus and brain stem will go to bed and fall asleep within minutes, remain asleep throughout the night, wake up in the morning and remain awake and alert throughout the day. During the night, the asleep individual will experience several cycles of sleep, beginning with light sleep, progressing through rapid eye movement sleep (REM-sleep) to deep sleep and back. Each complete sleep period lasts about 90 minutes. Periods of REM-sleep are closely associated with dreaming. During REM-sleep, neural signals emanating from certain parts of the brain stem ensure that skeletal muscles become "atonic" or are paralyzed, such that the individual can't "act out" their dreams.

Certain diseases and conditions may impair the normal functioning of the "zeitgeber" or circadian clock. These conditions may be reversible, such as desynchronization resulting from jet-lag, night-shift work or hunger, conditions easily remedied by adaptation or food intake. In contrast, damage to the nerves carrying light-dark related information from the retina to the SCN (conditions which may lead to blindness), or damage to the enteric nerves and neural structures which relay messages from the intestine to the SCN (conditions which may lead to neurodegenerative disorders) can cause permanent dysfunction of the circadian rhythm and abnormal sleep behavior.

Dysfunction of the circadian rhythm manifests first and foremost by abnormal sleep patterns. Such abnormalities typically are mild at onset and worsen progressively over time. A common symptom of sleep disorder is a delay in the onset of sleep. This delay can be as long as several hours, and the individual may not be able to fall asleep until the early hours of the morning. Another common symptom is sleep fragmentation, meaning that the individual awakens several times during the course of the night. Once awakened, the individual may not be able to get back to sleep, and each awake fragment may last an hour or more, further reducing "total sleep time," which is calculated by subtracting total time of the awake fragments from total time spent in bed. Total sleep time also diminishes with age, from about 14 to about 16 hours a day in newborns, to about 12 hours by one year of age, to about 7 to about 8 hours in young adults, progressively declining to about 5 to about 6 hours in elderly individuals. Total sleep time can be used to calculate an individual's "sleep age" and to compare it to their chronologic age. Significant discrepancies between sleep age and chronologic age are a reflection of the severity of the sleep disorder. "Sleep efficiency," defined as the percentage of the time spent in bed asleep is another index that can be used to determine the severity of the sleep disorder. Sleep efficiency is said to be abnormal when the percentage is below about 70%.

Sleep disorders and/or sleep disturbances include but are not limited to REM-behavior disorders, disturbances in the Circadian rhythm, delayed sleep onset, sleep fragmentation, and hallucinations. Other sleep disorders or disturbances that can be treated and/or prevented according to the disclosed methods include but are not limited to hypersomnia (*i.e.,* daytime sleepiness), parasomnias (such as nightmares, night terrors, sleepwalking, and confusional arousals), periodic limb movement disorders (such as Restless Leg Syndrome), jet lag, narcolepsy, advanced sleep phase disorder, non-24 hour sleep-wake syndrome.

Individuals with severe sleep disorders also typically suffer from day-time sleepiness. This can manifest as day-time "napping" for an hour or two, to "dosing off" for a few minutes during a film or to "micro-sleep" episodes lasting seconds to minutes, and of which the individual may or may not be aware. Narcolepsy is a rare and extreme form of day-time sleepiness, with the sudden onset of sleep causing the individual to fall down. Another form of sleep disturbance involves periods of loud snoring alternating with periods of "sleep apnea" (arrested breathing), a condition known as "sleep-disordered breathing." "REM-behavior disorder" (RBD) or "REM-disturbed sleep", is yet another sleep disturbance which occurs as a result of dysfunctional neural communication between the enteric nervous system, structures responsible for sleep in the brain stem and the SCN. In individuals with RBD, neural signaling which causes the paralysis (atonia) of muscles under voluntary control is impaired or altogether absent. As a consequence, "acting-out" of dreams occurs. This can range at one end of the spectrum from an increase in muscle tone detectable by electromyography (EMG) and accompanied by small movements of the hands and feet during REM sleep, to violent thrashing of arms and legs, kicking or punching a bed partner, speaking out loud or screaming, at the other end of the spectrum. Episodes of RBD can occur several times a night or very infrequently, once every few months. They can also be clustered, several occurring within a week, followed by periods of normal sleep. Unless the condition can be treated with a medication that restores normal functioning of the circadian rhythm and improves sleep patterns, individuals with RBD progress to neurodegenerative disorders.

Sleep disturbances include but are not limited to RBD, circadian rhythm dysfunction, delayed sleep onset, Restless leg syndrome, daytime sleepiness, and sleep fragmentation.

A "normal" or "restful" sleep period is defined as a sleep period uninterrupted by wakefulness. Alternatively, a said period can be defined by the recommended or appropriate amount of sleep for the subject's age category, e.g., (i) infants 0-3 months = about 11 to about 19 hours; (ii) infants about 4 to about 11 months = about 12 to about 18 hours; (iii) toddlers about 1 to about 2 years = about 9 to about 16 hours; (iv) preschoolers about 3 to about 5 years = about 10 to about 14 hours; (v) school-aged children about 6 to about 13 years = about 7 to about 12 hours; (v) teenagers about 14 to about 17 years = about 7 to about 11 hours; (vi) young adults about 18 to about 25 years = about 6 to about 11 hours; (vii) adults about 26 to about 64 years = about 6 to about 10 hours; and (viii) older adults ≥ 65 years = about 5 to about 9 hours. Thus, for treating sleep disturbance in a subject, the treatment can result in a restful sleep period of at least about 4, about 5, about 6, about 7, about 8, about 9, about 10, about 11, or about 12 hours.

There are several different scientifically acceptable ways to measure a sleep period uninterrupted by wakefulness. First, electrodes attached to the head of a subject can measure electrical activity in the brain by electroencephalography (EEG). This measure is used because the EEG signals associated with being awake are different from those found during sleep. Second, muscle activity can be measured using electromyography (EMG), because muscle tone also differs between wakefulness and sleep. Third, eye movements during sleep can be measured using electro-oculography (EOG). This is a very specific measurement that helps to identify Rapid Eye Movement or REM sleep. Any of these methods, or a combination thereof, can be used to determine if a subject obtains a restful sleep period following administration of at least one aminosterol or a salt or derivative thereof to the subject.

Further, circadian rhythm regulation can be monitored in a variety of ways, including but not limited to monitoring wrist skin temperature as described by Sarabia et al. 2008. Similarly symptoms of RBD can be monitored using a daily diary and RBD questionnaire (Stiasny-Kolster et al. 2007).

In some embodiments, administration of a therapeutically effective amount of an aminosterol to a patient with disturbed sleep results in improvement in frequency of normal or restful sleep as determined by a clinically recognized assessment scale for one or more types of sleep dysregulation, by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%. The improvement can be measured using any clinically recognized tool or assessment.

Several tools may be used to measure and evaluate the effect of aminosterol treatment on sleep, including for example: (1) Sleep Diary (participants completed a sleep diary on a daily basis throughout the study. The diaries included time into bed and estimated time to sleep as well as wake time and duration during the night.); (2) I-Button Temperature Assessment. The I-Button is a small, rugged self-sufficient system that measures temperature and records the results in a protected memory section. The Thermochron I-Button DS1921H (Maxim Integrated, Dallas, TX) was used for skin temperature measurement. I-Buttons were programmed to sample every 10 mins., and attached to a double-sided cotton sport wrist band using Velcro, with the sensor face of the I-Button placed over the inside of the wrist, on the radial artery of the dominant hand. Subjects removed and replaced the data logger when necessary (i.e., to have a bath or shower). The value of skin temperature assessment in sleep research is that the endogenous skin warming resulting from increased skin blood flow is functionally linked to sleep propensity. From the collected data, the mesor, amplitude, acrophase (time of peak temperature), Rayleight test (an index of interdaily stability), mean waveforms are calculated); (3) Unified Parkinson's Disease Rating Scale (UPDRS), sections 1.7 (sleep problems), 1.8 (daytime sleepiness) and 1.13 (fatigue); (4) Parkinson's Disease Fatigue Scale (PFS-16); (5) REM Sleep Behavior Disorder Screening Questionnaire; and (6) Parkinson's Disease Sleep Scale.

Unpublished data shows that aminosterol administration results in improvement in sleep disorders. The frequency of arm or leg thrashing reported in the sleep diary diminished progressively from 2.2 episodes/week at baseline to 0 at maximal dose (100% improvement). Total sleep time increased progressively from 7.1 hours at baseline to 8.4 hours at 250 mg (an 18% increase) following treatment and was consistently higher than baseline beyond 125 mg. Unlike stool-related indices, the improvement in many CNS symptoms persisted during wash-out.

In an an unpublished study, circadian rhythm of skin temperature was evaluated in 12 patients (i.e., those who had recordings that extended from baseline through washout). Circadian system functionality was evaluated by continuously monitoring wrist skin temperature using a temperature sensor (Thermochron iButton DS1921H; Maxim, Dallas, TX) (Sarabia et al. 2008). Briefly, this analysis includes the following parameters: (i) the inter-daily stability (the constancy of 24-hour rhythmic pattern over days, IS); (ii) intra-daily variability (rhythm fragmentation, IV); (iii) average of 10-minute intervals for the 10 hours with the minimum temperature (L10); (iv) average of 10-minute intervals for the 5 hours with the maximum temperature (M5) and the relative amplitude (RA), which was determined by the difference between M5 and L10, divided by the sum of both. Finally, the Circadian Function Index (CFI) was calculated by integrating IS, IV, and RA. Consequently, CFI is a global measure that oscillates between 0 for the absence of circadian rhythmicity and 1 for a robust circadian rhythm.

A comparison was performed of circadian rhythm parameters during the baseline, fixed dose and washout periods. Aminosterol administration improved all markers of healthy circadian function, including increasing rhythm stability, relative amplitude, and circadian function index, while reducing rhythm fragmentation. The improvement persisted for several of these circadian parameters during the wash-out period. Improvements were also seen in REM-behavior disorder (RBD) and sleep. RBD and total sleep time also improved progressively in a dose-dependent manner.

### 4. Cognitive Impairment

Cognitive impairment, including mild cognitive impairment (MCI), is characterized by increased memory or thinking problems exhibited by a subject as compared to a normal subject of the same age. Approximately 15 to 20 percent of people age 65 or older have MCI, and MCI is especially linked to neurodegenerative conditions such as Alzheimer's disease (AD) or synucleinopathies like Parkinson's disease (PD). In 2002, an estimated 5.4 million people (22%) in the United States over age 70 had cognitive impairment without dementia. Plassman et al. 2009.

In one embodiment, administration of an aminosterol composition is to a patient that is not lying down. In some embodiments, "lying down" is defined as the subject's body being predominantly flat, or in a horizontal, recumbent, or in prostrate position.

Cognitive impairment may entail memory problems including a slight but noticeable and measurable decline in cognitive abilities, including memory and thinking skills. When MCI primarily affects memory, it is known as "amnestic MCI." A person with amnestic MCI may forget information that would previously have been easily recalled, such as appointments, conversations, or recent events, for example. When MCI primarily affects thinking skills other than memory, it is known as "nonamnestic MCI." A person with nonamnestic MCI may have a reduced ability to make sound decisions, judge the time or sequence of steps needed to complete a complex task, or with visual perception, for example.

Related disorders and conditions include, but are not limited to, dementia, Alzheimer's, delirium, Parkinson's, diabetes, high blood pressure, high cholesterol, depression, psychological and behavioral conditions, amnesia, Lewy body diseases, or Huntington's disease, among others.

In some embodiments, administration of a therapeutically effective amount of an aminosterol to a patient in need results in improvement of cognitive impairment as determined by a clinically recognized assessment scale, by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%. The improvement can be measured using any clinically recognized tool or assessment.

In some embodiments, the clinically recognized tool or assessment is selected from the group consisting of ADASCog, Mini-Mental State Exam(MMSE), Mini-cog test, Woodcock-Johnson Tests of Cognitive Abilities, Leiter International Performance Scale, Miller Analogies Test, Raven's Progressive Matrices, Wonderlic Personnel Test, IQ tests, or a computerized tested selected from Cantab Mobile, Cognigram, Cognivue, Cognision, and Automated Neuropsychological Assessment Metrics Cognitive Performance Test(CPT).

Cognitive impairment and the improvement following aminosterol treatment have been assessed using several tools (unpublished data): (1) Mini Mental State Examination (MMSE); (2) Trail Making Test (TMT) Parts A and B; and (3) Unified Parkinson's Disease Rating Scale (UPDRS), sections 1.1 (cognitive impairment).

Assessments were made at baseline and at the end of the fixed dose and washout periods, and an analysis was done with respect to the cognition symptoms. The results showed that the total UPDRS score was 64.4 at baseline, 60.6 at the end of the fixed dose period and 55.7 at the end of the wash-out period (a 13.5% improvement). Part 1 of the UPDRS (which includes section 1.1, cognitive impairment) had a mean baseline score of 11.6, a fixed aminosterol dose mean score of 10.6, and a wash-out mean score of 9.5, demonstrating an almost 20% improvement (UPDRS cognitive impairment is rated from 1 = slight improvement to 4 = severe impairment, so lower scores correlate with better cognitive function). In addition, MMSE improved from 28.4 at baseline to 28.7 during treatment and to 29.3 during wash-out (the MMSE has a total possible score of 30, with higher scores correlating with better cognitive function). Unlike stool-related indices, the improvement in many CNS symptoms persisted during wash-out.

### 5. Depression

Clinical depression is characterized by a sad, blue mood that goes above and beyond normal sadness or grief. Major depression is an episode of sadness or apathy along with other symptoms that lasts at least two consecutive weeks and is severe enough to interrupt daily activities. Depressive events feature not only negative thoughts, moods, and behaviors but also specific changes in bodily functions (like, eating, sleeping, energy and sexual activity, as well as potentially developing aches or pains). One in 10 people will have a depression in their lifetime. Doctors clinically diagnose depression; there is no laboratory test or X-ray for depression.

Increasingly sophisticated forms of brain imaging, such as positron emission tomography (PET), single-photon emission computed tomography (SPECT), and functional magnetic resonance imaging (fMRI), permit a much closer look at the working brain than was possible in the past. An fMRI scan, for example, can track changes that take place when a region of the brain responds during various tasks. A PET or SPECT scan can map the brain by measuring the distribution and density of neurotransmitter receptors in certain areas. Use of this technology has led to a better understanding of which brain regions regulate mood and how other functions, such as memory, may be affected by depression. Areas that play a significant role in depression are the amygdala, the thalamus, and the hippocampus.

Research shows that the hippocampus is smaller in some depressed people. For example, in one fMRI study published in *The Journal of Neuroscience,* investigators studied 24 women who had a history of depression. On average, the hippocampus was 9% to 13% smaller in depressed women as compared with those who were not depressed. The more bouts of depression a woman had, the smaller the hippocampus. Stress, which plays a role in depression, may be a key factor, since experts believe stress can suppress the production of new neurons (nerve cells) in the hippocampus.

Thus, in one embodiment, encompassed are methods of treating, preventing, and/or slowing the onset or progression of depression comprising administering a therapeutically effective amount of an aminosterol. While not wishing to be bound by theory, it is theorized that the aminosterol triggers neurogenesis, which functions to combat depression.

In one embodiment, administration of an aminosterol composition is to a patient that is not lying down. In some embodiments, "lying down" is defined as the subject's body being predominantly flat, or in a horizontal, recumbent, or in prostrate position.

In some embodiments, the methods of the disclosure produce an improvement in a subject's clinical depression. An improvement in a subject's depression can be measured using any clinically-recognized measurement. For example, improvement can be measured using a depression rating scale. In one embodiment of the disclosure, following treatment a subject experiences an about 5, about 10, about 15, about 20, about 25, about 30, about 35, about 40, about 45, about 50, about 55, about 60, about 65, about 70, about 75, about 80, about 85, about 90, about 95 or an about 100% improvement. The improvement can be measured using any clinically recognized tool or assessment.

In some embodiments, the improvement can be measured from one or more medically-recognized techniques selected from the group consisting of the Patient Health Questionnaire-9 (PHQ-9); the Beck Depression Inventory (BDI); Zung Self-Rating Depression Scale; Center for Epidemiologic Studies-Depression Scale (CES-D); and the Hamilton Rating Scale for Depression (HRSD).

It has been shown (unpublished data) that depression and/or mood improvement improve aminosterol treatment. Depression was assessed using several tools: (1) Beck Depression Inventory (BDI-II); (2) Unified Parkinson's Disease Rating Scale (UPDRS), sections 1.3 (depressed mood), 1.4 (anxious mood), 1.5 (apathy), and 1.13 (fatigue); and (3) Parkinson's Disease Fatigue Scale (PFS-16).

Assessments were made at baseline and at the end of the fixed dose and washout periods. An analysis was done with respect to depression and mood scores. Total UPDRS score was 64.4 at baseline, 60.6 at the end of the fixed dose period and 55.7 at the end of the wash-out period, demonstrating a 13.5% improvement, and Part 1 of the UPDRS (which includes mood and depression scores) went from a mean score of 11.6 at baseline, to a mean of 10.6 during the fixed aminosterol dose period, with a mean score of 9.5 during the washout period, demonstrating an improvement of 18%. In addition, BDI-II scores decreased from 10.9 at baseline to 9.9 during treatment and 8.7 at wash-out, showing an improvement in depression scoring of 20%. The improvement in many CNS symptoms persisted during wash-out.

### 6. Alpha-synuclein aggregation

Alpha-synuclein is a potent pro-inflammatory hormone. Inflammation can be blocked by either of two strategies. First, inflammation can be blocked by reducing the tissue concentration of alpha-synuclein by decreasing or stopping production of alpha-synuclein. Alternatively, inflammation can be blocked by interrupting the signaling between alpha-synuclein and inflammatory cells that express CD11b. The subject of the methods of the disclosure can be any mammal, including a human.

The inflammatory disease or condition caused by excessive expression of neuronal alpha-synuclein can be a neurodegenerative disorder (NDD), such as an alpha-synucleinopathy. Exemplary alpha-synucleinopathies include, but are not limited to, PD, Lewy body dementia, multiple system atrophy, amytrophic lateral sclerosis, Huntington's chorea, multiple sclerosis or schizophrenia. In other embodiments, the inflammatory disease or condition caused by excessive expression of neuronal alpha-synuclein can be an autoimmune disease, a chronic inflammatory disease, or an autoinflammatory disease. In other embodiments, the inflammatory disease or condition caused by excessive expression of neuronal alpha-synuclein can be selected from the group consisting of asthma, chronic peptic ulcer, tuberculosis, chronic periodontitis, chronic sinusitis, chronic active hepatitis, psoriatic arthritis, gouty arthritis, acne vulgaris, osteoarthritis, rheumatoid arthritis, lupus, systemic lupus erythematosus, multiple sclerosis, ankylosing spondylitis, Crohn's disease, psoriasis, primary sclerosing cholangitis, ulcerative colitis, allergies, inflammatory bowel diseases, Celiac disease, Chronic prostatitis, diverticulitis, dermatomyositis, polymyositis, systemic sclerosis, glomerulonephritis, hidradenitis suppurativa, hypersensitivities, interstitial cystitis, otitis, pelvic inflammatory disease, reperfusion injury, rheumatic fever, sarcoidosis, transplant rejection, and vasculitis.

In some embodiments of the disclosure, patient populations particularly susceptible to excessive production or secretion of alpha-synuclein can benefit from the methods of the disclosure and are targeted for therapy, including for example preventative therapy. For example, a patient population having a mutated form of alpha-synuclein resulting in increased amounts of alpha-synuclein in tissues can be treated using the methods of the disclosure. Another example of a patient population susceptible for high levels of alpha-synuclein are patients having chronic inflammatory conditions or diseases.

The methods of the disclosure can result in a decrease in intensity of inflammation, blood levels of inflammatory markers, inflammatory markers in tissue, or number of inflammatory cells in tissue, or a combination thereof, as compared to a control or as compared to the qualitative or quantitative amount from the same patient or subject prior to treatment. For example, the decrease can be about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%. The improvement can be measured using any clinically recognized tool or assessment. Non-limiting examples of inflammatory markers include TNF, IL-8, or CRP.

In some embodiments, the decrease is measured quantitatively or qualitatively by a method selected from the group consisting of high-performance liquid chromatography, liquid chromatography mass spectrometry, enzyme linked immunosorbent assay, protein immunoprecipitation, immunoelectrophoresis, Western blot, and protein immunostaining.

In some embodiments of the disclosure, patient populations particularly susceptible to excessive production or secretion of alpha-synuclein can benefit from the methods of the disclosure and are targeted for therapy, including for example preventative therapy. For example, a patient population having a mutated form of alpha-synuclein resulting in increased amounts of alpha-synuclein in tissues can be treated using the methods of the disclosure. Another example of a patient population susceptible for high levels of alpha-synuclein are patients having chronic inflammatory conditions or diseases. A still further example is a patient population having elevated levels of alpha-synuclein aggregation in their enteric nerve cells, manifesting as a constipation.

In one embodiment, administration of an aminosterol composition is to a patient that is not lying down. In some embodiments, "lying down" is defined as the subject's body being predominantly flat, or in a horizontal, recumbent, or in prostrate position.

In addition, it follows from the present disclosure that an individual with an inflammatory condition appropriate for treatment or prophylaxis with the methods targeting alpha-synuclein described herein can be identified by determination of the tissue concentrations of alpha-synuclein at sites of inflammation, with high levels of alpha-synuclein, as compared to a control or healthy subject, correlating with patients appropriate for treatment with a method of the disclosure.

### 6. Neurological Conditions or Diseases

The methods and aminosterol compositions of the disclosure can be used for treating, preventing, and/or slowing the onset or progression of neurodiseases such as Alzheimer's disease (AD), Huntington's Disease, Multiple Sclerosis, Amyotorphic Lateral Sclerosis (ALS), multiple system atrophy (MSA), schizophrenia, Friedreich's ataxia, vascular dementia, Lewy Body dementia or disease, spinal muscular atrophy, supranuclear palsy, frontotemporal dementia, progressive nuclear palsy, Guadeloupian Parkinsonism, spinocerebellar ataxia, and autism or autism spectrum disorder.

In one embodiment, administration of an aminosterol composition is to a patient that is not lying down. In some embodiments, "lying down" is defined as the subject's body being predominantly flat, or in a horizontal, recumbent, or in prostrate position.

### a. Parkinson's Disease

Parkinson's disease (PD) is a progressive neurodegenerative disorder caused by accumulation of the protein α-synuclein (αS) within the enteric nervous system (ENS), autonomic nerves and brain (Braak et al. 2003). While motor symptoms are still required for a diagnosis of Parkinson's disease (Hughes et al. 1992), non-motor symptoms represent a greater therapeutic challenge (Zahodne et al. 2012). These symptoms include constipation (Ondo et al. 2012; Lin et al. 2014), disturbances in sleep architecture (Ondo et al. 2001; Gjerstad et al. 2006), cognitive dysfunction (Auyeung et al. 2012), hallucinations (Friedman et al. 2016), REM behavior disorder (RBD) and depression (Aarsland et al. 2007), all of which result from impaired function of neural pathways not restored by replacement of dopamine. In fact, long-term institutionalization, caregiver burden and decrease in life expectancy correlate more significantly with the severity of these symptoms than with motor symptoms (Goetz et al. 1995).

Parkinson's disease (PD) is a progressive neurodegenerative disease associated with the accumulation of the protein α-synuclein within the peripheral and central nervous system (CNS). Whilst diagnosis of PD is primarily based on the presence of a combination of motor symptoms, non-motor symptoms, including neuropathic constipation, present an common important therapeutic challenge. In 2003, Braak proposed that PD begins within the GI tract caused when neurotoxic aggregates of α-synuclein form within the ENS, evidenced clinically by the appearance of constipation in a majority of people with PD many years before the onset of motor symptoms. A recent study in rats has demonstrated movement of aggregates of α-synuclein from the ENS to the CNS via the vagus and other afferent nerves. Neurotoxic aggregates accumulated progressively within the brainstem and then dispersed rostrally to structures within the diencephalon, eventually reaching the cerebral hemispheres.

The so-called gold standard for PD diagnosis entails expert diagnosis based on patient symptoms. PD and prodromal PD diagnosis is probabilistic, made on the basis of the presence of particular motor and non-motor symptoms, physiological pathologies, genetic characteristics, and environmental factors. Diagnosis may include a combination of markers (any disease indicator, whether a symptom, sign, or biomarker) ranging from mild motor symptoms [i.e., UPDRS-1987 version score ≥ 3 excluding action tremor; or MDS-UPDRS score > 6 excluding postural-action tremor; slowness, loss of muscle movements, tremor, rigidity, imbalance, abnormal posture], non-motor symptoms (i.e., REM SBD, olfactory dysfunction, constipation, excessive daytime somnolence, symptomatic hypotension, erectile/urinary dysfunction, depression, cognition), and ancillary diagnostic tests (i.e., abnormal tracer uptake of the presynaptic dopaminergic system: SPECT or positron emission tomography).

Parkinson's disease is defined as a synucleinopathy, and synuclein deposition remains the main final arbiter of diagnosis. Additionally, patients with dementia and Lewy bodies are considered as having PD if they meet clinical disease criteria. Imaging (e.g., MRI, single photon emission computed tomography [SPECT], and positron emission tomography [PET]) allows in vivo brain imaging of structural, functional, and molecular changes in PD patients.

PD may also be assessed using the Unified Parkinson's Disease Rating Scale (UPDRS) which consists of 42 items in four subscales: (1) Part I, Non-Motor Aspects of Experiences of Daily Living (nM-EDL): cognitive impairment (section 1.1), hallucinations and psychosis (section 1.2), depressed mood (section 1.3), anxious mood (section 1.4), apathy (section 1.5), features of dopamine dysregulation syndrome (section 1.6), sleep problems (section 1.7), daytime sleepiness (section 1.8), pain and other sensations (section 1.9), urinary problems (section 1.10), constipation problems (section 1.11), light headedness on standing (section 1.12), and fatigue (section 1.13); (2) Part II, Motor Aspects of Experiences of Daily Living (M-EDL): speech (section 2.1), saliva & drooling (section 2.2), chewing and swallowing (section 2.3), eating tasks (section 2.4), dressing (section 2.5), hygiene (section 2.6), handwriting (section 2.7), doing hobbies and other activities (section 2.8), turning in bed (section 2.9), tremor (section 2.10), getting out of bed, a car, or a deep chair (section 2.11), walking and balance (section 2.12), and freezing (section 2.13); Part III, Motor Examination: speech (section 3.1), facial expression (section 3.2), rigidity (section 3.3), finger tapping (section 3.4), hand movements (section 3.5), pronation-supination movements of hands (section 3.6), toe tapping (section 3.7), leg agility (section 3.8), arising from chair (section 3.9), gait (3.10), freezing of gait (section 3.11), postural stability (section 3.12), posture (section 3.13), global spontaneity of movement (body bradykinesia) (section 3.14), postural tremor of the hands (section 3.15), kinetic tremor of the hands (section 3.16), rest tremor amplitude (section 3.17), and constancy of rest tremor (section 3.18); Part IV, Motor Complications: time spent with dyskinesias (section 4.1), functional impact of dyskinesias (section 4.2), time spent in the off state (section 4.3), functional impact of fluctuations (section 4.4), complexity of motor fluctuations (section 4.5), and painful off-state dystonia (section 4.6).

Further, symptom-based endpoints can be assessed using known scales. For example, (1) depression can be assessed using the Beck Depression Inventory (BDI-II) (Steer et al. 2000), cognition can be assessed using the Mini Mental State Examination (MMSE), sleep and REM-behavior disorder (RBD) can be assessed using a daily diary and an RBD questionnaire (RBDQ) (Stiasny-Kolster et al. 2007), and hallucinations can be assessed using the PD hallucinations questionnaire (PDHQ) (Papapetropoulos et al. 2008) and direct questioning. Circadian system status can also be assessed by continuously monitoring wrist skin temperature (Thermochron iButton DS1921H; Maxim, Dallas) following published procedures (Sarabia *et al.* 2008).

In another embodiment, administration of a therapeutically effective amount of an aminosterol to a PD patient results in improvement of one or more symptoms of Parkinson's disease or on one or more clinically accepted scoring metrics, by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%. The improvement can be measured using any clinically recognized tool or assessment.

In one embodiment, administration of an aminosterol composition is to a patient that is not lying down. In some embodiments, "lying down" is defined as the subject's body being predominantly flat, or in a horizontal, recumbent, or in prostrate position.

Not to be bound by theory, it is believed that prior or co-administration of an aminosterol composition according to the disclosure may reduce the dopamine dosage required to elicit a therapeutic effect for Parkinson's symptoms and/or increase the period during which the patient is sensitive to dopamine. It is also theorized that prior or co-administration of an aminosterol composition according to the disclosure may delay the time period when a patient is advised to begin dopamine therapy. This is significant, as currently patients are encouraged to delay initiation of dopamine treatment as long as possible, as after a period of time subjects become resistant to dopamine.

As detailed in Example 4, a study was conducted to evaluate the impact of aminosterols on PD. Remarkable improvement in a wide variety of symptoms correlated with PD were observed, including a significant and positive effect on bowel function and neurologic symptoms of PD. Following aminosterol treatment, the Unified Parkinson's Disease Rating Scale (UPDRS) score, a global assessment of motor and non-motor symptoms, showed significant improvement. Improvement was also seen in the motor component. Improvements were also seen in cognitive function (MMSE scores), hallucinations, REM-behavior disorder (RBD) and sleep. The study was the first proof of concept demonstration that directly targeting αS pharmacologically can achieve beneficial GI, autonomic and CNS responses in neurodiseases such as PD.

### b. Alzheimer's Disease

The symptoms of Alzheimer's disease are primarily marked by cognitive deficits including memory impairment, language dysfunction, and visuospatial skills; functional impairment that may span occupational and social issues (e.g., activities of daily living); and behavioral symptoms including depression, anxiety, aggression and psychosis may also appear as the disease progresses in severity.

At this time, unambiguous diagnosis of AD requires clinical findings of cognitive deficits consistent with AD and post-mortem identification of brain pathologies consistent with AD. The term AD dementia is used to describe dementia that is due to the pathophysiologies of AD. The term "probable Alzheimer's disease" is used in life when a subject demonstrates clinical characteristics of AD and when other possible biological causes of dementia (e.g. PD or stroke) are excluded.

There are currently a variety of art-accepted methods for diagnosing probable AD. Typically, these methods are used in combination. These methods include determining an individual's ability to carry out daily activities and identifying changes in behavior and personality. Dementia of the AD type is also typically characterized by an amnestic presentation (memory deficit) or language, visuospatial or executive function deficits. Cognitive ability/impairment may be determined by art-accepted methods, including, but not limited to, validated instruments that assess global cognition (e.g., the Modified Mini Mental State Examination (3MS-E)), and specific domains such as visual and verbal memory (e.g., the Brief Visuospatial Memory Test (Revised) (BVMT-R) and the Hopkins Verbal Learning Test (Revised) (HVLT-R), respectively), language (e.g., the Generative Verbal Fluency Test (GVFT)) and executive function and attention (e.g., the Digit Span Test (DST)). Dementia due to AD is also defined by insidious onset and a history of worsening cognitive performance.

The criteria for `probable Alzheimer's disease' are described a National Institute of Aging-Alzheimer's Association workgroup (McKhann et al. 2011 Alzheimer's Dement, 7: 263-269). According to this workgroup, for people who first exhibit the core clinical characteristics of Alzheimer's disease dementia, evidence of biomarkers associated with the disease may enhance the certainty of the diagnosis.

In one embodiment, administration of an aminosterol composition is to a patient that is not lying down. In some embodiments, "lying down" is defined as the subject's body being predominantly flat, or in a horizontal, recumbent, or in prostrate position.

In another embodiment, administration of a therapeutically effective amount of an aminosterol to an AD subject results in improvement of one or more symptoms of AD or on one or more clinically accepted scoring metrics, by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%. The improvement may be measured by any of the art accepted methods discussed above.

### c. Multiple System Atrophy

Multiple system atrophy (MSA) is a progressive neurodegenerative disorder characterized by a combination of symptoms that affect both the autonomic nervous system (the part of the nervous system that controls involuntary action such as blood pressure or digestion) and movement. MSA, also known as Shy-Drager syndrome, is a neurodegenerative disorder characterized by tremors, slow movement, muscle rigidity, and postural instability (collectively known as parkinsonism) due to dysfunction of the autonomic nervous system, and ataxia. This is caused by progressive degeneration of neurons in several parts of the brain including the substantia nigra, striatum, inferior olivary nucleus, and cerebellum. There is no known cure for MSA and management is primarily supportive.

Progression of neurodegeneration can be measured using well known techniques. For example, an electroencephalogram (EEG) can be used as a biomarker for the presence and progression of a neurodegenerative disease (S. Morairty, 2013). Another exemplary technique that can be used to measure progression of neurodegeneration of MRI (Rocca et al. 2017).

A variety of neuroimaging techniques may be useful for the early diagnosis and/or measurement of progression of MSA. Examples of such techniques include but are not limited to neuroimaging, functional MRI, structural MRI, diffusion tensor imaging (DTI) (including for example diffusion tensor measures of anatomical connectivity), [18F]fluorodeoxyglucose (FDG) PET, agents that label amyloid, [18F]F-dopa PET, radiotracer imaging, volumetric analysis of regional tissue loss, specific imaging markers of abnormal protein deposition (e.g., for AD progression), multimodal imaging, and biomarker analysis (Jon Stoessl, 2012). Combinations of these techniques can also be used to measure disease progression.

For example, structural MRI can be used to measure atrophy of the hippocampus and entorhinal cortex in AD, as well as involvement of the lateral parietal, posterior superior temporal and medial posterior cingulate cortices. In frontotemporal dementias (FTD), structural MRI can show atrophy in frontal or temporal poles.

In one embodiment, administration of an aminosterol composition is to a patient that is not lying down. In some embodiments, "lying down" is defined as the subject's body being predominantly flat, or in a horizontal, recumbent, or in prostrate position.

In another embodiment, administration of a therapeutically effective amount of an aminosterol to an MSA patient results in improvement of one or more symptoms of MSA, by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%. Improvement can be measured using any clinically recognized tool or assessment, for example, Unified MSA Rating Scale (UMSARS), Scale for the Assessment and Rating of Ataxia (SARA), Berg Balance Scale (BBS), MSA Health-Related Quality of Life scale (MSA-QOL), or Scales for Outcomes in Parkinson's Disease-Autonomic questionnaire (SCOPA-AUT).

### d. Schizophrenia

Schizophrenia is a chronic progressive disorder that has at its origin structural brain changes in both white and gray matter. It is likely that these changes begin prior to the onset of clinical symptoms in cortical regions, particularly those concerned with language processing. Later, they can be detected by progressive ventricular enlargement. Current magnetic resonance imaging (MRI) technology can provide a valuable tool for detecting early changes in cortical atrophy and anomalous language processing, which may be predictive of who will develop schizophrenia.

A 2013 study of schizophrenia patients documented brain changes seen in MRI scans from more than 200 patients beginning with their first episode and continuing with scans at regular intervals for up to 15 years. The scans showed that people at their first episode had less brain tissue than healthy individuals. The findings suggest that those who have schizophrenia are being affected by something before they show outward signs of the disease.

While not wished to be bound by theory, it is theorized that administration of a therapeutically effective amount of an aminosterol composition to a schizophrenia patient may treat, prevent and/or slow the onset or progression of schizophrenia or any one or more symptoms thereof. In some embodiments, the administration may be oral - resulting in absorption in the ENS. In some embodiments, the administration may be intranasal - resulting in stimulation of neurogenesis, which has a positive impact on the loss of brain tissue characteristic of schizophrenia subjects.

In one embodiment of the disclosure, administration of a therapeutically effective amount of an aminosterol to a schizophrenia patient results in improvement of one or more symptoms as determined by a clinically recognized psychiatric symptom rating scale. Examples of such rating scales include for example, the Positive and Negative Syndrome Scale (PANSS), the Psychotic Symptom Rating Scales (PSYRATS), the Quality of Life Scale (QLS), the Schizophrenia Cognition Rating Scale (SCoRS), the Drug Attitude Inventory (DAI), and the Abnormal Involuntary Movement Scale (AIMS).

In another embodiment, administration of a therapeutically effective amount of an aminosterol to a schizophrenia patient results in improvement of one or more symptoms as determined by a clinically recognized psychiatric symptom rating scale, by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%. Improvement can be measured using any clinically recognized tool or assessment, for example, those recited above.

### e. Autism or Autism Spectrum Disorder

Autism, or autism spectrum disorder, refers to a range of conditions characterized by challenges with social skills, repetitive behaviors, speech and nonverbal communication, as well as by unique strengths and differences. There are many types of autism, caused by different combinations of genetic and environmental influences. Autism's most-obvious signs tend to appear between 2 and 3 years of age. In some cases, it can be diagnosed as early as 18 months. Some developmental delays associated with autism can be identified and addressed even earlier.

The Centers for Disease Control and Prevention (CDC) estimates autism's prevalence as 1 in 59 children in the United States. This includes 1 in 37 boys and 1 in 151 girls. Around one third of people with autism remain nonverbal. Around one third of people with autism have an intellectual disability. Certain medical and mental health issues frequently accompany autism. They include gastrointestinal (GI) disorders, seizures, sleep disturbances, attention deficit and hyperactivity disorder (ADHD), anxiety and phobias.

Experts are still uncertain about of all the causes of autism. In all likelihood, there are multiple causes. It appears that a number of different circumstances, including environmental, biologic, and genetic factors, set the stage for autism and make a child more likely to have the disorder. It is likely that genetics play a large factor in the development of autism. Identical twins are more likely to both be affected than twins who are fraternal (not genetically identical). In a family with one autistic child, the chance of having another child with autism is about 5 percent - or one in 20 - which is much higher than in the normal population. Research also has found that some emotional disorders (such as manic depression) occur more often in families of a child with autism.

A recent brain-tissue study suggests that children affected by autism have a surplus of synapses, or connections between brain cells. The excess is due to a slowdown in the normal pruning process that occurs during brain development. During normal brain development, a burst of synapse formation occurs in infancy. This is particularly pronounced in the cortex, which is central to thought and processing information from the senses. But by late adolescence, pruning eliminates about half of these cortical synapses. In addition, many genes linked to autism are known to affect the development or function of brain synapses. The study also found that the brain cells from individuals with autism were filled with damaged parts and deficient in signs of a normal breakdown pathway called "autophagy." Tang et al. 2014.

Thus, one embodiment of the disclosure is directed to methods of treating, preventing, and/or slowing the onset or progression of autism, the methods comprising administering a therapeutically effective amount of an aminosterol to the autism subject. In one embodiment, treatment results in improvement in one or more characteristics of autism. Such characteristics can be, for example, communication skills, social interaction, sensory sensitivity, and behavior. Improvement can be measured using any clinically recognized tool or assessment.

For example, the methods of the disclosure may show an improvement in one or more characteristics of autism, such as behavior, communication, mood, etc., as measured by a medically recognized scale. The improvement may be, for example, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%. The medically recognized scale may be selected from, for example, Childhood Autism Rating Scale (CARS), Autism Spectrum Rating Scales, or The Michigan Autism Spectrum Questionnaire.

### f. Other Neurodiseases

The methods and compositions of the disclosure may also be useful in treating, preventing, and/or slowing the onset or progression of a variety of other neurodiseases.

Huntington's disease (HD) is a fatal genetic disorder that causes the progressive breakdown of nerve cells in the brain. It deteriorates a person's physical and mental abilities during their prime working years and has no cure. Full-time care is required in the later stages of the disease. Symptoms of Huntington's disease most commonly become noticeable between the ages of 35 and 44 years, but they can begin at any age from infancy to old age. The most characteristic initial physical symptoms are jerky, random, and uncontrollable movements called chorea. Suicide is the cause of death in about 9% of cases. Death typically occurs 15 to 20 years from when the disease was first detected.

Progressive supranuclear palsy, also called Steele-Richardson-Olszewski syndrome, is an brain disorder that causes serious problems with walking, balance and eye movements. The disorder results from deterioration of cells in areas of the brain that control body movement and thinking. There is no known cure for PSP and management is primarily supportive.

Frontotemporal dementia (FTD) is a group of related conditions resulting from the progressive degeneration of the temporal and frontal lobes of the brain. These areas of the brain play a significant role in decision-making, behavioral control, emotion and language. The frontotemporal dementias (FTD) encompass six types of dementia involving the frontal or temporal lobes. They are: behavioral variant of FTD, semantic variant primary progressive aphasia, nonfluent agrammatic variant primary progressive aphasia, corticobasal syndrome, progressive supranuclear palsy, and FTD associated with motor neuron disease. Currently, there is no cure for FTD.

Vascular dementia, also known as multi-infarct dementia (MID) and vascular cognitive impairment (VCI), is dementia caused by problems in the supply of blood to the brain, typically a series of minor strokes, leading to worsening cognitive decline that occurs step by step. Risk factors for vascular dementia include age, hypertension, smoking, hypercholesterolemia, diabetes mellitus, cardiovascular disease, and cerebrovascular disease. Other risk factors include geographic origin, genetic predisposition, and prior strokes.

Amyotrophic lateral sclerosis (ALS), also known as motor neuron disease (MND), or Lou Gehrig's disease, is a specific disease which causes the death of neurons controlling voluntary muscles. ALS is characterized by stiff muscles, muscle twitching, and gradually worsening weakness due to muscles decreasing in size. This results in difficulty speaking, swallowing, and eventually breathing. The cause is not known in 90% to 95% of cases. The remaining 5-10% of cases are genetic. The underlying mechanism involves damage to both upper and lower motor neurons. No cure for ALS is known. The disease can affect people of any age, but usually starts around the age of 60 and in inherited cases around the age of 50. The average survival from onset to death is 2 to 4 years, although about 10% survive longer than 10 years.

Multiple sclerosis (MS) is a demyelinating disease in which the insulating covers of nerve cells in the brain and spinal cord are damaged. This damage disrupts the ability of parts of the nervous system to communicate, resulting in a range of signs and symptoms, including physical, mental, and sometimes psychiatric problems. Specific symptoms can include double vision, blindness in one eye, muscle weakness, trouble with sensation, or trouble with coordination. MS takes several forms, with new symptoms either occurring in isolated attacks (relapsing forms) or building up over time (progressive forms). Between attacks, symptoms may disappear completely; however, permanent neurological problems often remain, especially as the disease advances. While the cause is not clear, the underlying mechanism is thought to be either destruction by the immune system or failure of the myelin-producing cells. Proposed causes for this include genetics and environmental factors such as being triggered by a viral infection. There is no known cure for MS. Life expectancy is on average 5 to 10 years lower than that of an unaffected population. MS is the most common immune-mediated disorder affecting the central nervous system. In 2015, about 2.3 million people were affected globally, and in 2015 about 18,900 people died from MS, up from 12,000 in 1990.

Spinal muscular atrophy (SMA) is an inherited neuromuscular disorder characterized by loss of motor neurons and progressive muscle wasting, often leading to early death. The disorder is caused by a genetic defect in the SMN1 gene, which encodes SMN, a protein necessary for survival of motor neurons. Lower levels of the protein results in loss of function of neuronal cells in the anterior horn of the spinal cord and subsequent system-wide atrophy of skeletal muscles. SMA is the most common genetic cause of infant death. In December 2016, nusinersen became the first approved drug to treat SMA while several other compounds remain in clinical trials.

Friedreich's ataxia is an autosomal recessive inherited disease that causes progressive damage to the nervous system. It manifests in initial symptoms of poor coordination such as gait disturbance; it can also lead to scoliosis, heart disease and diabetes, but does not affect cognitive function. The ataxia of Friedreich's ataxia results from the degeneration of nervous tissue in the spinal cord, in particular sensory neurons essential (through connections with the cerebellum) for directing muscle movement of the arms and legs. The spinal cord becomes thinner and nerve cells lose some of their myelin sheath (the insulating covering on some nerve cells that helps conduct nerve impulses).

Progression of neurodegeneration can be measured using well known techniques. For example, an electroencephalogram (EEG) can be used as a biomarker for the presence and progression of a neurodegenerative disease. S. Morairty, "Detecting Neurodegenerative Diseases Before Damage Is Done," SRI International (July 26, 2013) (https://www.sri.com/blog/ detecting-neurodegenerative-diseases). Another exemplary technique that can be used to measure progression of neurodegeneration of MRI. Rocca et al., "The Role of T1-Weighted Derived Measures of Neurodegeneration for Assessing Disability Progression in Multiple Sclerosis," Front Neurol., 8:433 (Sept. 4, 2017).

A variety of neuroimaging techniques may be useful for the early diagnosis and/or measurement of progression of neurodegenerative disorders. Examples of such techniques include but are not limited to neuroimaging, functional MRI, structural MRI, diffusion tensor imaging (DTI) (including for example diffusion tensor measures of anatomical connectivity), [18F]fluorodeoxyglucose (FDG) PET, agents that label amyloid, [18F]F-dopa PET, radiotracer imaging, volumetric analysis of regional tissue loss, specific imaging markers of abnormal protein deposition (e.g., for AD progression), multimodal imaging, and biomarker analysis. Jon Stoessl, "Neuroimaging in the early diagnosis of neurodegenerative disease," Transl. Neurodegener., 1: 5 (2012). Combinations of these techniques can also be used to measure disease progression.

For example, structural MRI can be used to measure atrophy of the hippocampus and entorhinal cortex in AD, as well as involvement of the lateral parietal, posterior superior temporal and medial posterior cingulate cortices. In frontotemporal dementias (FTD), structural MRI can show atrophy in frontal or temporal poles. DTI can be used to show abnormal white matter in the parietal lobes of patients with dementia with Lewy bodies (DLB) as compared to AD. Functional MRI may reveal reduced frontal but increased cerebellar activation during performance of a working memory task in FTD compared to AD. In another example, [18F]fluorodeoxyglucose (FDG) PET can show reduced glucose metabolism in parietotemporal cortex in AD. *Id.*

In one embodiment of the disclosure, the progression or onset of a neurodegenerative disorder is slowed or prevented over a defined time period, following administration of a therapeutically effective amount of an aminosterol to a subject in need, as measured by a medically-recognized technique. For example, the progression or onset of a neurodegenerative disorder can be slowed by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%.

The progression of neurodegeneration may be measured qualitatively or quantitatively using any of the medically recognized tools described herein, for example, ADASCog, Mini-Mental State Exam (MMSE), Mini-cog test, Woodcock-Johnson Tests of Cognitive Abilities, Leiter International Performance Scale, Miller Analogies Test, Raven's Progressive Matrices, Wonderlic Personnel Test, IQ tests, or a computerized tested selected from Cantab Mobile, Cognigram, Cognivue, Cognision, and Automated Neuropsychological Assessment Metrics Cognitive Performance Test(CPT), Unified MSA Rating Scale (UMSARS), Scale for the Assessment and Rating of Ataxia (SARA), Berg Balance Scale (BBS), MSA Health-Related Quality of Life scale (MSA-QOL), or Scales for Outcomes in Parkinson's Disease-Autonomic questionnaire (SCOPA-AUT). Alternatively, neurodegeneration can be measured by measuring the levels of one or more biomarkers known in the art to indicate neurodegeneration using analytical techniques selected from the group consisting of, for example, high-performance liquid chromatography, liquid chromatography mass spectrometry, enzyme linked immunosorbent assay, protein immunoprecipitation, immunoelectrophoresis, Western blot, and protein immunostaining. Biomarkers indicating neurodegeneration are known to the skilled artisan and may include, for example, any of those in Beach et al. 2017, the entire disclosure of which is hereby incorporated by reference.

The period of time over which the progression or onset of a neurodegenerative disorder is measured can be for example, one or more months or one or more years, *e.g*., about 6 months, about 1 year, about 18 months, about 2 years, about 36 months, about 3, about 4, about 5, about 6, about 7, about 8, about 9, about 10, about 11, about 12, about 13, about 14, about 15, about 16, about 17, about 18, about 19, or about 20 years, or any amount of months or years in between the values of about 6 months to about 20 years or more.

In another embodiment of the disclosure, a neurodegenerative disorder may be positively impacted by administration of a therapeutically effective amount of an aminosterol. A "positive impact" includes for example slowing advancement of the condition, improving one or more symptoms, etc.

### 7. Blood Pressure, Cerebral Ischemic, and General Ischemic Disorders

Blood pressure (BP) correlates with ischemic disorders. Studies have been conducted showing that aminosterol administration to a subject with high blood pressure (HBP) or low blood pressure (LBP), results in normalization of the blood pressure; subjects with high blood pressure at pretreatment demonstrated lower blood pressure at post medication measurement, while subjects with low blood pressure at pretreatment demonstrated higher blood pressure at post medication measurement (data not shown).

Studies have also shown a correlation between abnormal αS pathology and ischemic disorders. For example, one study reported that post-stroke induction of αS mediates ischemic brain damage (Kim et al. 2016). Yet another study conducted a comparison of the amount of αS in ischemic stroke and PD subjects, with the results showing that the levels of oligomeric form of αS of red blood cells in ischemic stroke and PD patients were both significantly higher than that of healthy controls (Zhao et al., 2016). Finally, another study reported that cerebral ischemic injury leads to a reduction in αS and consequently causes serious brain damage (Kho, 2017).

The methods of the invention are also be useful in treating, preventing, and/or delaying the onset or progression of high blood pressure (HBP) and/or a related symptom, or low blood pressure (LBP) and/or a related symptom, where the HBP or LBP is correlated with αS pathology, and/or correlated with dysfunctional DA neurotransmission, also known as dopaminergic dysfunction, and wherein the HBP or LBP is also correlated with a cerebral or general ischemic disorder.

In some embodiments, the cerebral ischemic disorder comprises cerebral microangiopathy, intrapartal cerebral ischemia, cerebral ischemia during/after cardiac arrest or resuscitation, cerebral ischemia due to intraoperative problems, cerebral ischemia during carotid surgery, chronic cerebral ischemia due to stenosis of blood-supplying arteries to the brain, sinus thrombosis or thrombosis of cerebral veins, cerebral vessel malformations, or diabetic retinopathy.

In some embodiments, the general ischemic disorders comprises high blood pressure, high cholesterol, myocardial infarction, cardiac insufficiency, cardiac failure, congestive heart failure, myocarditis, pericarditis, perimyocarditis, coronary heart disease, angina pectoris, congenital heart disease, shock, ischemia of extremities, stenosis of renal arteries, diabetic retinopathy, thrombosis associated with malaria, artificial heart valves, anemias, hypersplenic syndrome, emphysema, lung fibrosis, or pulmonary edema.

### III. Aminosterols

U.S. Patent No. 6,962,909, entitled "Treatment of neovascularization disorders with squalamine," discloses various aminosterols, and this disclosure is specifically incorporated by reference with respect to its teaching of aminosterol compounds. Any aminosterol known in the art, including those described in U.S. Patent No. 6,962,909, can be used in the disclosed methods. In some embodiments, the aminosterol is aminosterol 1436 or a salt or derivative thereof, squalamine or a salt or derivative thereof, or a combination thereof.

Squalamine has broad spectrum antimicrobial activity and anti-angiogenic activity. Squalamine (ENT-01 in the examples) inhibits the formation of α-synuclein (αS) aggregates *in vitro* and *in vivo,* reverses motor dysfunction in the *C*. *elegans* αS model, and restores gastrointestinal motility in mouse models of PD. As shown in the examples herein, squalamine also increases transcription in the gut and is an effective treatment for wounds.

Useful aminosterol compounds comprise a bile acid nucleus and a polyamine, attached at any position on the bile acid, such that the molecule exhibits a net positive charge contributed by the polyamine.

Thus, in some embodiments, the disclosed methods comprise administering a therapeutically effective amount of a pharmaceutical composition comprising one or more aminosterols having the chemical structure of Formula I: 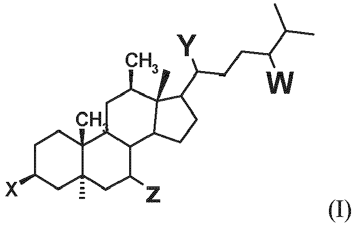 wherein,
**W** is 24S -OSO₃ or 24R -OSO₃;
**X** is 3β H₂N-(CH₂)₄-NH-(CH₂)₃-NH- or 3α H₂N-(CH₂)₄-NH-(CH₂)₃-NH-;
**Y** is 20R -CH₃; and
**Z** is 7α or 7β -OH.

In another embodiment of the invention, the aminosterol is one of the aminosterols **(1-8)** isolated from *Squalus acanthias:*

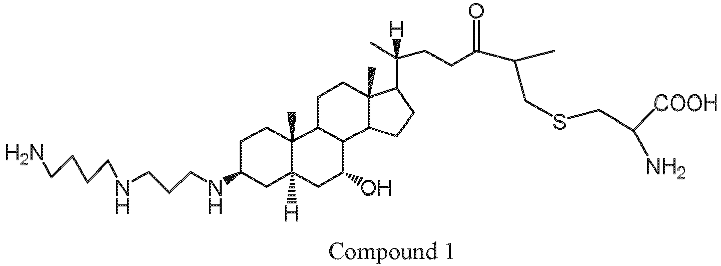

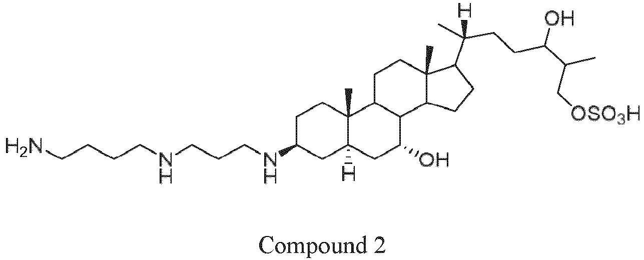

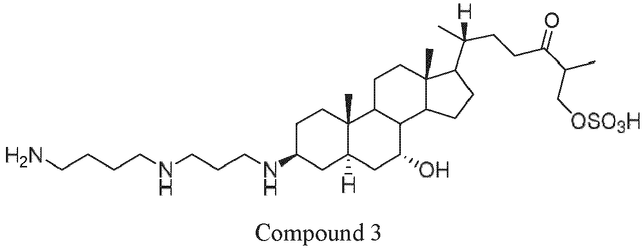

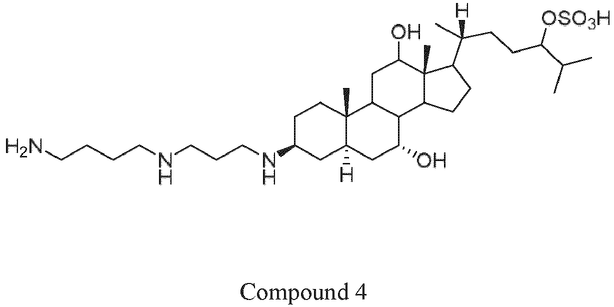

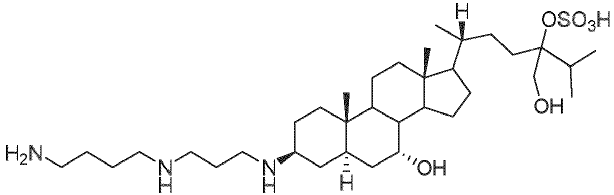

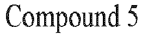

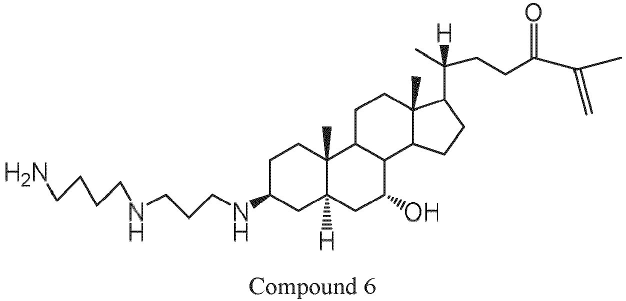

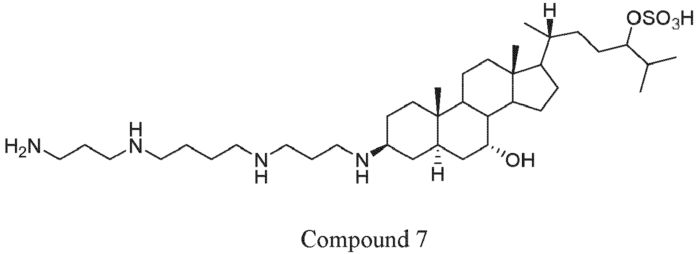

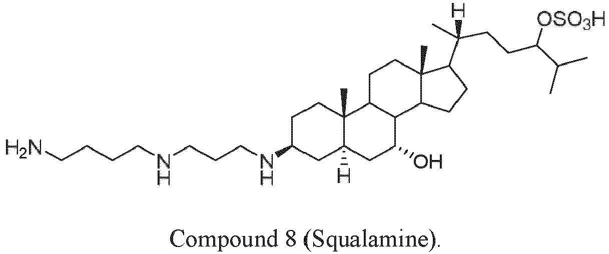

In another embodiment of the invention, the aminosterol is at least one compound from the following: 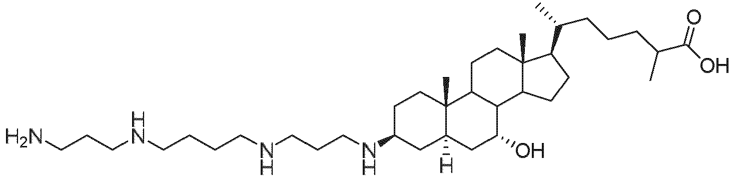 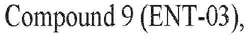 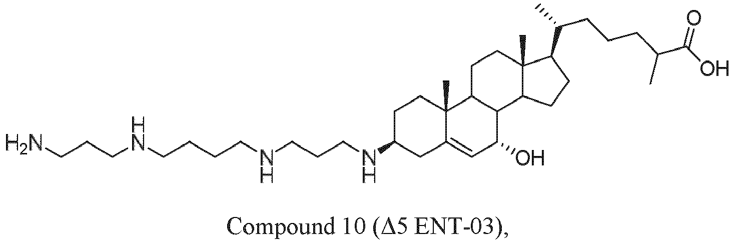 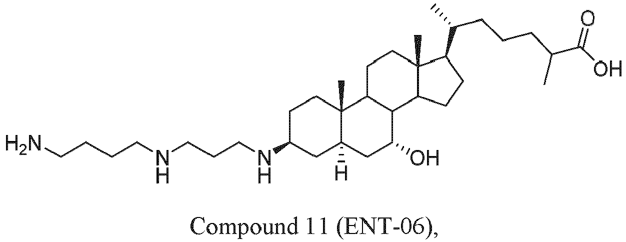 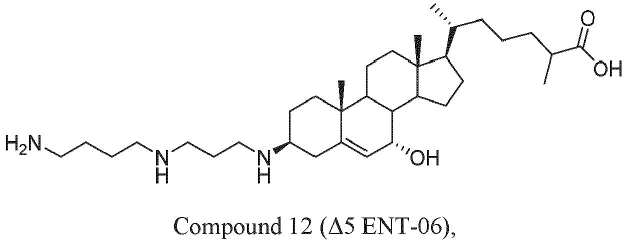 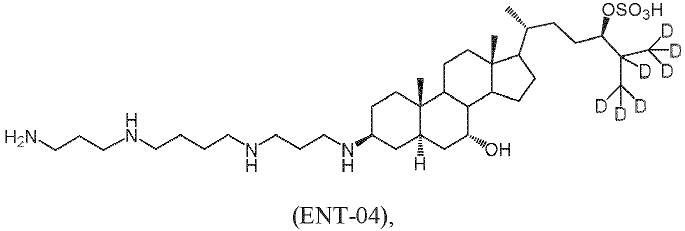 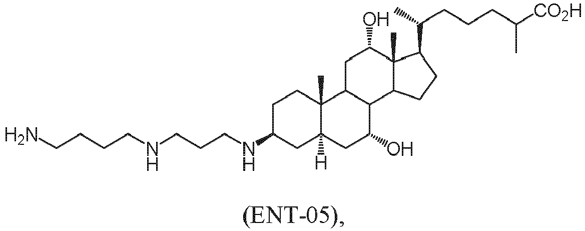 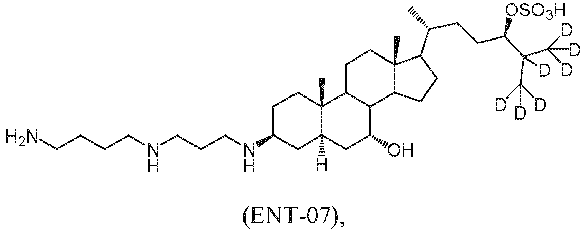 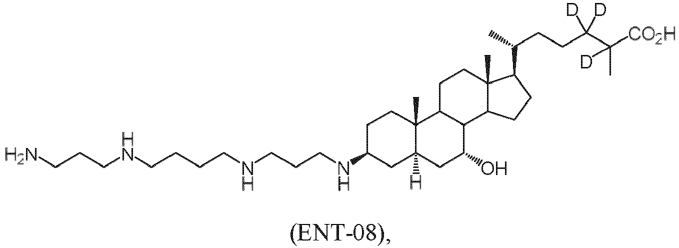 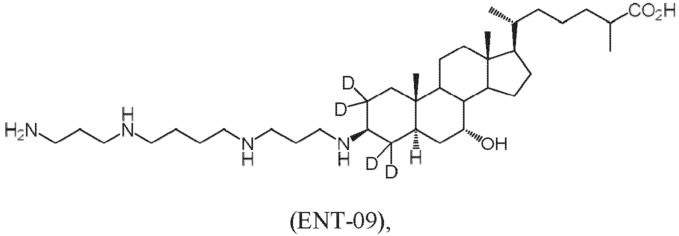 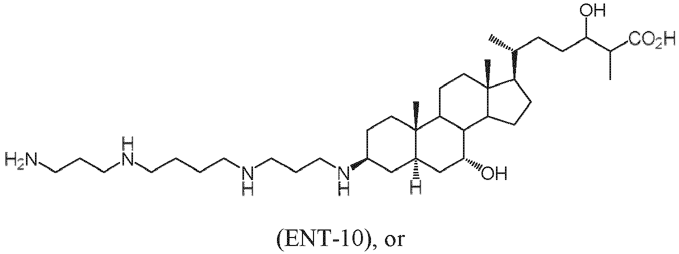 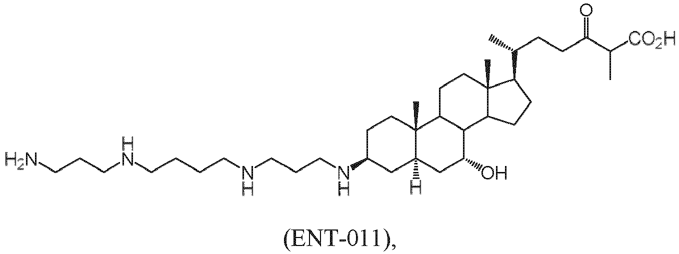 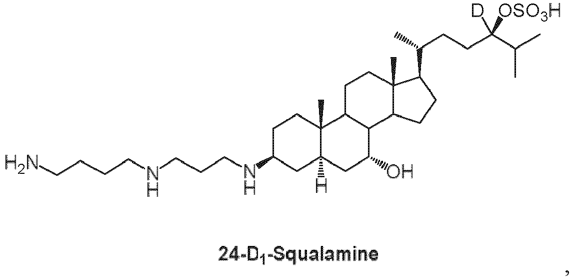 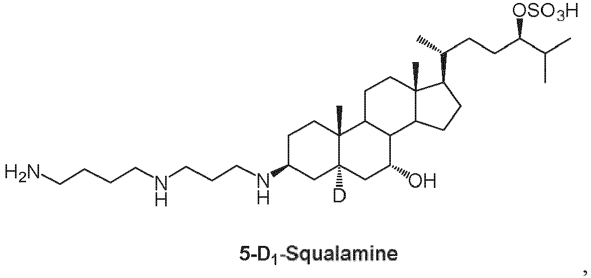 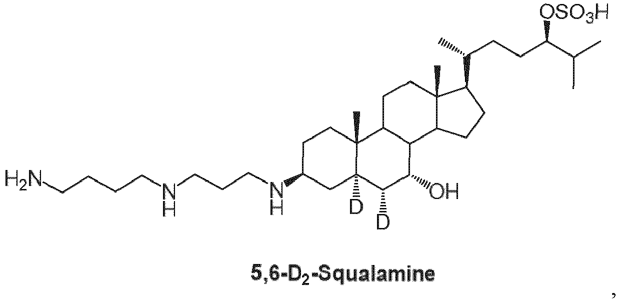 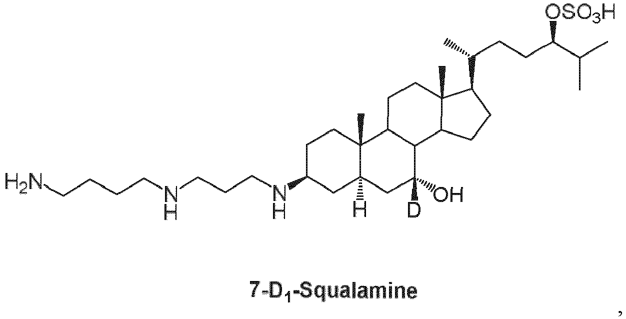 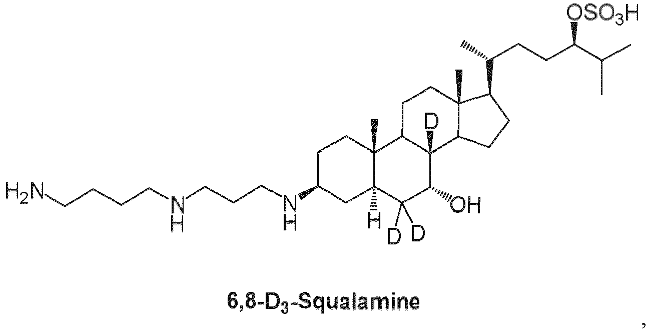 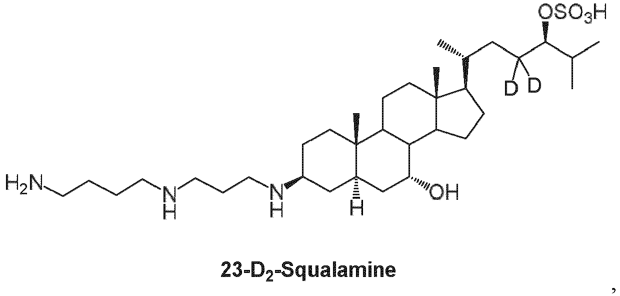 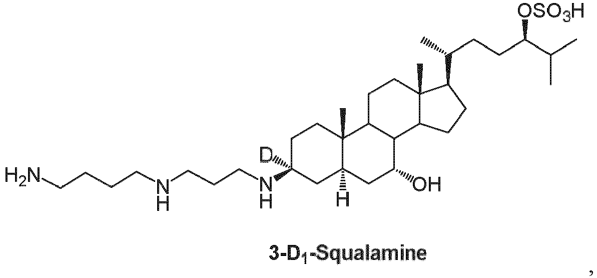 or

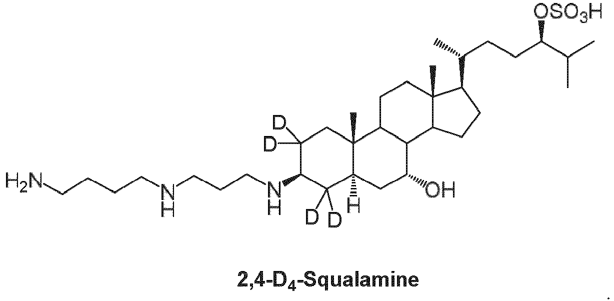

Variants or derivatives of known aminosterols, such as squalamine, aminosterol 1436, or an aminosterol isolated from *Squalus acanthias,* may be used in the disclosed methods.

In one embodiment, the aminosterol is aminosterol 1436 or a squalamine isomer. In yet another embodiment of the disclosure, the aminosterol is a derivative of squalamine or another naturally occurring aminosterol modified through medicinal chemistry to improve biodistribution, ease of administration, metabolic stability, or any combination thereof. In another embodiment, the squalamine or aminosterol is modified to include one or more of the following: (1) substitutions of the sulfate by a sulfonate, phosphate, carboxylate, or other anionic moiety chosen to circumvent metabolic removal of the sulfate moiety and oxidation of the cholesterol side chain; (2) replacement of a hydroxyl group by a non-metabolizable polar substituent, such as a fluorine atom, to prevent its metabolic oxidation or conjugation; and (3) substitution of various ring hydrogen atoms to prevent oxidative or reductive metabolism of the steroid ring system.

In yet another embodiment, the aminosterol comprises a sterol nucleus and a polyamine, attached at any position on the sterol, such that the molecule exhibits a net charge of at least + 1, the charge being contributed by the polyamine.

In yet another embodiment, the aminosterol comprises a bile acid nucleus and a polyamine, attached at any position on the bile acid, such that the molecule exhibits a net positive charge being contributed by the polyamine.

In some embodiments, an aminosterol composition is used in the methods of the disclosure and comprises: (a) at least one pharmaceutical grade aminosterol; and optionally (b) at least one phosphate selected from the group consisting of an inorganic phosphate, an inorganic pyrophosphate, and an organic phosphate. In some embodiments, the aminosterol is formulated as a weakly water soluble salt of the phosphate. In some embodiments, the phosphate is an inorganic polyphosphate, and the number of phosphates can range from about 3 (tripolyphosphate) to about 400, or any number in-between these two values. In other embodiments, the phosphate is an organic phosphate which comprises glycerol 2 phosphates.

In some embodiments, the aminosterol is selected from the group consisting of: (a) squalamine or a pharmaceutically acceptable salt or derivative thereof; (b) a squalamine isomer; (c) aminosterol 1436; (d) an aminosterol comprising a sterol or bile acid nucleus and a polyamine, attached at any position on the sterol or bile acid, such that the molecule exhibits a net charge of at least + 1, the charge being contributed by the polyamine; (e) an aminosterol which is a derivative of squalamine modified through medicinal chemistry to improve biodistribution, ease of administration, metabolic stability, or any combination thereof; (f) an aminosterol modified to include one or more of the following: (i) substitutions of the sulfate by a sulfonate, phosphate, carboxylate, or other anionic moiety chosen to circumvent metabolic removal of the sulfate moiety and oxidation of the cholesterol side chain; (ii) replacement of a hydroxyl group by a non-metabolizable polar substituent, such as a fluorine atom, to prevent its metabolic oxidation or conjugation; and (iii) substitution of various ring hydrogen atoms to prevent oxidative or reductive metabolism of the steroid ring system; (g) an aminosterol that can inhibit the formation of actin stress fibers in endothelial cells stimulated by a ligand known to induce stress fiber formation, having the chemical structure of Formula I (above); or (h) any combination thereof.

In some embodiments, the methods of the disclosure can employ a formulation of aminosterol 1436 as an insoluble salt of phosphate, polyphosphate, lactate, or an organic phosphate ester. In some embodiments, the methods of the disclosure can employ a formulation of aminosterol 1436 (Zasloff, Williams et al. 2001) as an insoluble salt of phosphate, polyphosphate, or an organic phosphate ester.

Any pharmaceutically acceptable salt of an aminosterol can be used in the methods of the disclosure. For example, a lactate salt, a phosphate salt or buffer, free base, succinate, phosphate, mesylate or other salt form associated with low mucosal irritation can be utilized in the methods and compositions of the disclosure.

### IV. Routes of Administration

Topical administration referred to herein may comprise topical administration of aminosterol compositions to the wound site of a cutaneous or mucosal wound. The wound site may comprise an area about 0%, about 5%, about 10%, about 15%, about 20%, about 25%, about 30%, about 40%, about 45%, about 50%, about 55%, about 60%, about 65%, about 70%, about 75%, about 80%, about 85%, about 90%, about 95%, about 100%, about 125%, about 150%, about 175%, about 200%, about 225%, about 250%, about 275%, about 300%, about 325%, about 350%, about 375%, or about 400% greater than the area of the wound. In one embodiment, the wound site completely encompasses the wound.

Topical administration may include administration to internal surfaces of mucosal or cutaneous tissue. For example, administration to the internal surface of the lungs, oral cavity, nasal cavity, esophageal cavity, sinus cavity, rectal cavity, vaginal cavity, or auditory cavity.

In one embodiment, topical administration comprises administration to the lungs. The internal surface of the lungs may receive aminosterol via bronchoscope or injection into the lungs of a mist, gas or otherwise airborne aminosterol composition. Ventilators or compressors may include a mask worn by the subject and may deliver vaporized or otherwise airborne aminosterol to the lungs. Metered-dose inhalers (MDSs), dry powder inhalers, nebulizers, and soft mist inhalers are well known in the art for the delivery of medication to the airways and may be used to deliver the aminosterol to the lungs.

**Non-topical route of administration in combination with a topical dosage form:** The topical administration of aminosterols may be complemented by the administration of one or more aminosterols by a non-topical route of administration. Non-topical routes of administration oral, intranasal, intravenous, intra-arterial, intradermal, intraperitoneal, intrathecal, intramuscular, epidural, intracerebral, intracerebroventricular, or any combination thereof. In some embodiments, aminosterols are administered via a non-topical route of administration selected from oral and/or intranasal.

It is appreciated that the aminosterol in any method herein, that is not administered topically, can be administered via any suitable route of administration, including but not limited to oral or intranasal delivery, injection (IP, IV, or IM) or a combination thereof. Other routes of administration include gastronomical (via gastric feeding tube) or directly to any segment of the digestive tract, for example, via duodenal feeding tube.

Further, co-administration of the aminosterol with injectable (e.g., IP, IV, IM) aminosterol formulations is also contemplated herein. For injectable dosage forms, the dosage form can comprise an aminosterol at a dosage of, for example, about 0.1 to about 20 mg/kg body weight. In other embodiments, the effective daily dosing amount is about 0.1, about 0.5, about 1, about 2, about 3, about 4, about 5, about 6, about 7, about 8, about 9, about 10, about 11, about 12, about 13, about 14, about 15, about 16, about 17, about 18, about 19, or about 20 mg/kg body weight.

The disclosure also encompasses methods of treatment using a combination of an aminosterol composition administered via one route, e.g., oral, with a second aminosterol composition, comprising the same or a different aminosterol, administered via a different route, e.g., intranasal. For example, in a method of the disclosure, squalamine can be administered orally and aminosterol 1436 can be administered IN.

Nasal administration may be accomplished via insufflation of solids, liquids or powders, inhalation of a gas, or via inhalation of a mist comprising the at least one aminosterol in a suitable carrier and optionally excipients. Suitable carriers and excipients are known to the skilled artisan and include buffers such as sodium phosphate, sodium citrate, and citric acid; solubilizers such as glycols, small quantities of alcohol, transcutol (diethylene glycol monoethyl ether), medium chain glycerides, labrasol (saturated polyglycolyzed C₈-C₁₀ glyceride), surfactants and cyclodextrins; preservatives such as parabens, phenyl ethyl alcohol, benzalkonium chloride, EDTA (ethylene diaminetetraaceticacid), and benzoyl alcohol; antioxidants such as sodium bisulfite, butylated hydroxytoluene, sodium metabisulfite and tocopherol; humectants such as glycerin, sorbitol and mannitol; surfactants such as polysorbet; bioadhesive polymers such as mucoadhesives; and penetration enhancers such as dimethyl sulfoxide (DMSO).

Nasal administration via inhalation of a mist may employ the use of metered-dose spray pumps. Typical volumes of aminosterol-comprising mist, delivered via a single pump of a metered-dose spray pump may be about 20-100 µl, 100-150 µl, or 150-200 µl. Such pumps offer high reproducibility of the emitted dose and plume geometry. The particle size and plume geometry can vary within certain limits and depend on the properties of the pump, the formulation, the orifice of the actuator, and the force applied.

The aminosterol can be administered via any pharmaceutically acceptable means, such as by injection (e.g., IM, IV, or IP), oral, pulmonary, intranasal, etc. Preferably, the aminosterol is administered orally, intranasally, or a combination thereof.

### V. Dosages and Dosing Period

The pharmaceutical composition comprising an aminosterol or a derivative or salt thereof can be administered for any suitable period of time, including as a maintenance dose for a prolonged period of time. Dosing can be done on an as needed basis using any pharmaceutically acceptable dosing regimen. Aminosterol dosing can be no more than 1x per day, once every other day, once every three days, once every four days, once every five days, once every six days, once a week, or divided over multiple time periods during a given day (e.g., twice daily).

In other embodiments, the aminosterol may be administered as a composition. The aminosterol composition can be administered: (1) as a single dose, or as multiple doses over a period of time; (2) at a maintenance dose for an indefinite period of time; (3) once, twice or multiple times; (4) daily, every other day, every 3 days, weekly, or monthly; (5) for a period of time such as about 1, about 2, about 3, or about 4 weeks, about 1, about 2, about 3, about 4, about 5, about 6, about 7, about 8, about 9, about 10, about 11, or about 12 months, about 1 year, about 1.5 years, about 2, about 2.5, about 3, about 3.5, about 4, about 4.5, about 5, about 5.5, about 6, about 6.5, about 7, about 7.5, about 8, about 8.5, about 9, about 9.5, about 10, about 10.5, about 11, about 11.5, about 12, about 12.5, about 13, about 13.5, about 14, about 14.5, about 15, about 15.5, about 16, about 16.5, about 17, about 17.5, about 18, about 18.5, about 19, about 19.5, about 20, about 20.5, about 21, about 21.5, about 22, about 22.5, about 23, about 23.5, about 24, about 24.5, or about 25 years, or (6) any combination of these parameters, such as daily administration for 6 months, weekly administration for 1 or more years, etc.

Yet another exemplary dosing regimen includes periodic dosing, where an effective dose can be administered once every about 1, about 2, about 3, about 4, about 5, about 6 days, or once weekly. In some embodiments, no food is taken after about 60 to about 90 minutes of oral administration of aminosterol.

The pharmaceutical composition may be applied in a single administration or in multiple administrations. The aminosterol composition can be applied for any suitable time period in one or more applications per day. For example, the aminosterol composition can be administered for at least once a week, at least twice a week, at least once a day, at least twice a day, multiple times daily, multiple times weekly, biweekly or any combination thereof. The aminosterol composition can be administered for a period of time of about one week to about 5 years or as long as it is necessary to maintain healthy skin or mucosa and as prescribed by a physician. Between applications, the application area may be washed to remove any residual composition.

Preferably, the aminosterol compositions are applied to the skin area in an amount of from about 0.001 mL/cm² to about 15.0 mL/ cm². An exemplary application amount and area is about 0.2 mL/cm², although any amount from about 0.001 mL/cm² up to about 15.0 mL/cm² can be applied. Other exemplary dosage amounts include, for example, about 0.001, about 0.002, about 0.003, about 0.004, about 0.005, about 0.006, about 0.007, about 0.008, about 0.009, about 0.010, about 0.015, about 0.02, about 0.025, about 0.03, about 0.035, about 0.04, about 0.045, about 0.05, about 0.055, about 0.06, about 0.065, about 0.07, about 0.075, about 0.08, about 0.085, about 0.09, about 0.095, about 0.1, about 0.15, about 0.2, about 0.25, about 0.3, about 0.35, about 0.4, about 0.45, about 0.5, about 0.55, about 0.6, about 0.65, about 0.7, about 0.75, about 0.8, about 0.85, about 0.9, about 0.95, about 1, about 2, about 3, about 4, about 5, about 6, about 7, about 8, about 9, about 10, about 11, about 12, about 13, about 14 or about 15 mL/ cm² of an aminosterol or a salt or derivative thereof.

Other dosage amounts include, but are not limited to, about 0.001, about 0.002, about 0.003, about 0.004, about 0.005, about 0.006, about 0.007, about 0.008, about 0.009, about 0.010, about 0.015, about 0.02, about 0.025, about 0.03, about 0.035, about 0.04, about 0.045, about 0.05, about 0.055, about 0.06, about 0.065, about 0.07, about 0.075, about 0.08, about 0.085, about 0.09, about 0.095, about 0.1, about 0.15, about 0.2, about 0.25, about 0.3, about 0.35, about 0.4, about 0.45, about 0.5, about 0.55, about 0.6, about 0.65, about 0.7, about 0.75, about 0.8, about 0.85, about 0.9, about 0.95, about 1, about 2, about 3, about 4, about 5, about 6, about 7, about 8, about 9, about 10, about 11, about 12, about 13, about 14 or about 15 g or mg /cm² of aminosterol or a salt or derivative thereof.

Following topical administration, the aminosterol compositions may be occluded or semi-occluded. Occlusion or semi-occlusion may be performed by overlaying a bandage, polyolefin film, impermeable barrier, or semi-impermeable barrier to the topical composition administered to the wound.

**Non-topical dosage amounts:** In some embodiments, the pharmaceutical composition is administered orally. Exemplary dosages of orally administered aminosterols include, but are not limited to, about 1, about 5, about 10, about 15, about 20, about 25, about 30, about 35, about 40, about 45, about 50, about 55, about 60, about 65, about 70, about 75, about 80, about 85, about 90, about 95, about 100, about 105, about 110, about 115, about 120, about 125, about 130, about 135, about 140, about 145, about 150, about 155, about 160, about 165, about 170, about 175, about 180, about 185, about 190, about 195, about 200, about 205, about 210, about 215, about 220, about 225, about 230, about 235, about 240, about 245, about 250, about 255, about 260, about 265, about 270, about 275, about 280, about 285, about 290, about 295, about 300, about 305, about 310, about 315, about 320, about 325, about 330, about 335, about 340, about 345, about 350, about 355, about 360, about 365, about 370, about 375, about 380, about 385, about 390, about 395, about 400, about 405, about 410, about 415, about 420, about 425, about 430, about 435, about 440, about 445, about 450, about 455, about 460, about 465, about 470, about 475, about 480, about 485, about 490, about 495, or about 500 mg/day.

In some embodiments, the pharmaceutical composition is administered intranasally. Intranasal dosages of an aminosterol are much lower than oral dosages of an aminosterol. Examples of such intranasal aminosterol low dosages include, but are not limited to, about 0.001 to about 6 mg, or any amount in-between these two values. For example, the low dosage of an intranasal administered aminosterol can be about 0.001, about 0.005, about 0.01, about 0.02, about 0.03, about 0.04, about 0.05, about 0.06, about 0.07, about 0.08, about 0.09, about 0.1, about 0.2, about 0.3, about 0.4, about 0.5, about 0.6, about 0.7, about 0.8, about 0.9, about 1, about 1.1, about 1.2, about 1.3, about 1.4, about 1.5, about 1.6, about 1.7, about 1.8, about 1.9, about 2, about 2.1, about 2.2, about 2.3, about 2.4, about 2.5, about 2.6, about 2.7, about 2.8, about 2.9, about 3, about 3.1, about 3.2, about 3.3, about 3.4, about 3.5, about 3.6, about 3.7, about 3.8, about 3.9, about 4, about 4.1, about 4.2, about 4.3, about 4.4, about 4.5, about 4.6, about 4.7, about 4.8, about 4.9, about 5, about 5.1, about 5.2, about 5.3, about 5.4, about 5.5, about 5.6, about 5.7, about 5.8, about 5.9, or about 6 mg/day.

### VI. Dosage Forms

The compositions may conveniently be presented in unit dosage form and may be prepared by any of the methods well known in the art of pharmacy. An exemplary oral dosage form is a tablet or capsule. An exemplary intranasal dosage form is a liquid or powder nasal spray. A nasal spray is designed to deliver drug to the upper nasal cavity, and can be a liquid or powder formulation, and in a dosage form such as an aerosol, liquid spray, or powder.

Pharmaceutically acceptable dosage forms for topical administration include, but are not limited to, ointments, gels, creams, liquids, pastes, suspensions, lotions, emulsions, lotions, gels, bioadhesive gels, sprays, aerosols, pastes, ointments, foams, solutions, or in the form of an article, for example, a stick, roll-on applicator, patch, lipstick, a paste tube; or carrier, such as a bandage, insert, powder, talc or other solid, including self adhering transdermal drug delivery systems (TDS) or transdermal patches.

The compositions may be formulated for immediate release, sustained release, controlled release, delayed release, or any combinations thereof, into the epidermis, dermis, or mucosa with minimum to no systemic absorption.

In some embodiments, the composition is formulated for delivery via a "patch" comprising a therapeutically effective amount aminosterol. In one embodiment, the patch provides a time release of aminosterol to the wound. In some embodiments, the patch provides time release of aminosterol to heathy skin. In one aspect, a patch for topical application is provided, comprising (a) a polymer matrix layer; and (b) at least one aminosterol dispersed within the polymer matrix layer. Such transdermal delivery systems or patches are well known in the art, see, for example, US5662926A, US5948433A, US6010715A, US5965154A, US6440454B1, and US5683712A, the entire disclosures of which are hereby incorporated by reference.

Typically, for a method of the present invention, aside from the content of the aminosterol composition used, a small amount of the composition (from about 1 ml to about 5 ml) is applied to exposed areas of skin from a suitable container or applicator, and, if necessary, the aminosterol composition is then spread over and/or rubbed into the skin using the hand, finger, or other suitable device. Each aminosterol composition disclosed herein is typically packaged in a container that is appropriate in view of the composition's viscosity and intended use by the consumer. For example, a lotion or fluid cream may be packaged in a bottle, roll-ball applicator, capsule, propellant-driven aerosol device, or a container fitted with a manually operated pump. A cream may simply be stored in a non-deformable bottle, or in a squeeze container, such as a tube or a lidded jar. A unit dose can be a packaged in a disposable pouch for application.

In some embodiments, oral administration of the aminosterols complements topical administration. An exemplary oral dosage form is a tablet or capsule. Oral dosage of an aminosterol can range from about 1 to about 500 mg/day, or any amount in-between these two values.

In instances where aerosol administration is appropriate, an aminosterol described herein, or a pharmaceutically acceptable salt or derivative thereof, can be formulated as an aerosol using standard procedures. The term "aerosol" includes any gas-borne suspended phase of a compound described herein which is capable of being inhaled into the bronchioles or nasal passages, and includes dry powder and aqueous aerosol, and pulmonary and nasal aerosols. Specifically, aerosol includes a gas-born suspension of droplets of a compound described herein, as may be produced in a metered dose inhaler or nebulizer, or in a mist sprayer. Aerosol also includes a dry powder composition of a composition of the present technology suspended in air or other carrier gas, which may be delivered by insufflation from an inhaler device, for example. See Ganderton & Jones, Drug Delivery to the Respiratory Tract (Ellis Horwood, 1987); Gonda, Critical Reviews in therapeutic Drug Carrier Systems, 6:273-313 (1990); and Raeburn et al,. Pharmacol. Toxicol. Methods, 27:143-159 (1992).

### VII. Composition Components

In some embodiments, the pharmaceutical composition comprises one or more pharmaceutically acceptable carriers, such as an aqueous carrier, buffer, and/or diluent. The aqueous carrier can be water or a buffer, including a physiologically compatible solution such as water or phosphate buffered saline. In some embodiments, a pharmaceutical composition disclosed herein further comprises a simple polyol compound, such as glycerin. Other examples of polyol compounds include sugar alcohols.

The aminosterol may be combined or coordinately administered with a suitable carrier or vehicle for administration. As used herein, the term "carrier" means a pharmaceutically acceptable solid or liquid filler, or diluent. A water-containing carrier can comprise pharmaceutically acceptable additives such as acidifying agents, alkalizing agents, antimicrobial preservatives, antioxidants, buffering agents, chelating agents, complexing agents, solubilizing agents, humectants, solvents, suspending and/or viscosity-increasing agents, tonicity agents, wetting agents, surfactants or other biocompatible materials. A tabulation of ingredients listed by the above categories can be found in the U.S. Pharmacopeia National Formulary, 1857-1859,

(1990). Some examples of the materials which can serve as pharmaceutically acceptable carriers are sugars, such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols, such as propylene glycol; polyols such as glycerin, sorbitol, mannitol and polyethylene glycol; esters such as ethyl oleate and ethyl laurate; agar; buffering agents such as magnesium hydroxide and aluminum hydroxide; alginic acid; pyrogen free water; isotonic saline; Ringer's solution, ethyl alcohol and phosphate buffer solutions, as well as other nontoxic compatible substances used in pharmaceutical formulations.

Wetting agents, emulsifiers and lubricants such as sodium lauryl sulfate and magnesium stearate, as well as coloring agents, release agents, coating agents, sweetening, flavoring and perfuming agents, preservatives and antioxidants can also be present in the compositions, according to the desires of the formulator. Examples of pharmaceutically acceptable antioxidants include water soluble antioxidants such as ascorbic acid, cysteine hydrochloride, sodium bisulfite, sodium metabisulfite, sodium sulfite and the like; oil-soluble antioxidants such as ascorbyl palmitate, butylated hydroxyanisole (BHA), butylated hydroxytoluene (BHT), lecithin, propyl gallate, alpha-tocopherol; or antioxidants including cetylpyridinium chloride, benzalkonium chloride, benzyl alcohol, chlorhexidine, imidazolidinyl urea, phenol, potassium sorbate, benzoic acid, bronopol, chlorocresol, paraben esters, phenoxyethanol, sorbic acid, ascorbic acid, ascorbyl palmitate, butylated hydroxyanisole, butylated hydroxytoluene, sodium ascorbate, sodium metabisulphite, edetic acid, semi-synthetic derivatives thereof, and combinations thereof and the like; and metal-chelating agents such as citric acid, ethylenediamine tetraacetic acid (EDTA), sorbitol, tartaric acid, phosphoric acid and the like.

The compositions can optionally comprise one or more solvents. Any suitable solvent can be used in the methods and formulations of the invention. Exemplary solvents include, but are not limited, to C₁-C₁₂ alcohols, isopropyl myristate, triacetin, N-methyl pyrrolidinone, aliphatic or aromatic alcohols, polyethylene glycols, ethanol, and propylene glycol. An example of an alcohol useful in the present invention includes, but is not limited to ethanol. Other short chain alcohols and/or amides may be used. Other solvents include dimethyl sulfoxide, dimethyl acetamide, and ethoxydiglycol. Mixtures of solvents can also be used in the pharmaceutical compositions used in the methods herein.

Pharmaceutical compositions according to the disclosure may also comprise one or more binding agents, filling agents, lubricating agents, suspending agents, pH adjusters, sweeteners, flavoring agents, effervescent agents, preservatives, buffers, wetting agents, disintegrants, surfactants, and other excipients. Such excipients are known in the art.

Suitable pH adjusters in the compositions include, but are not limited to, diethyanolamine, lactic acid, citric acid, hydrochloric acid, monoethanolamine, triethylanolamine, sodium hydroxide, sodium phosphate, semi-synthetic derivatives thereof, and combinations thereof.

Examples of surfactants include pharmaceutically acceptable ionic, non-ionic, anionic, cationic, and zwitterionic compounds or surfactants. In one embodiment, the surfactant comprises one or more of Tween family of surfactants (polyoxyethylene derivatives of sorbitan fatty acid esters; e.g., Tween 20, Tween 60, and Tween 80), nonphenol polyethylene glycol ethers, sorbitan esters (such as Span and Arlacel), glycerol esters (such as glycerin monostearate), polyethylene glycol esters (such as polyethylene glycol stearate), poloxamers or block polymers (such as Pluronics, e.g., Pluronic F68), acrylic polymers (such as Pemulen), ethoxylated fatty esters (such as Cremophor RH-40), ethoxylated alcohols (such as Brij), ethoxylated fatty acids, monoglycerides, silicon based surfactants, polysorbates, Tergitol NP-40 (Poly(oxy-1,2-ethanediyl), α -(4-nonylphenol)-.omega.-hydroxy, branched [molecular weight average 1980]), and Tergitol NP-70 (a mixed surfactant--AQ=70%).

Preferably, the individual surfactant molecules are free of cross-linkages. The surfactant is also preferably soluble in water. One or more surfactants may be used in the compositions of the methods of the invention. As used herein, the terms "stabilizer," "surface stabilizer," and "surfactant" are used interchangeably.

Examples of filling agents include lactose monohydrate, lactose anhydrous, and various starches; examples of binding agents include various celluloses and cross-linked polyvinylpyrrolidone, microcrystalline cellulose, such as Avicel^{®} PH101 and Avicel^{®} PH102, microcrystalline cellulose, and silicified microcrystalline cellulose (ProSolv SMCC^{™}). Suitable lubricants, including agents that act on the flowability of the powder to be compressed, may include colloidal silicon dioxide, such as Aerosil^{®} 200, talc, stearic acid, magnesium stearate, calcium stearate, and silica gel. Sweeteners may include any natural or artificial sweetener, such as sucrose, xylitol, sodium saccharin, cyclamate, aspartame, and acesulfame. Examples of flavoring agents are Magnasweet^{®} (trademark of MAFCO), bubble gum flavor, and fruit flavors, and the like. Examples of preservatives include potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quaternary compounds such as benzalkonium chloride.

Compositions of the disclosure also comprise squalene (or squalane), soybean oil (including super refined soybean oil), or medium chain triglycerides; the compositions can also comprise a combination of these compounds.

Squalene is a natural organic compound originally obtained for commercial purposes primarily from shark liver oil, though plant sources (primarily vegetable oils) are used as well. All plants and animals produce squalene, including humans. Squalane is a saturated form of squalene in which the double bonds have been eliminated by hydrogenation. Both squalene and squalane can be used in the compositions. Both naturally occurring and synthetic forms of squalene and squalane can be used in the compositions, including derivatives of squalene or squalane. Synthetic and naturally occurring squalenes and squalanes are collectively referred to herein as "squalene."

Medium-chain triglycerides (MCTs) are medium-chain (6 to 12 carbons) fatty acid esters of glycerol. Coconut oil is composed of approximately 66% medium-chain triglycerides. Other rich sources of MCTs include palm kernel oil and camphor tree drupes. The fatty acids found in MCTs are called medium-chain fatty acids. The names of the medium-chain fatty acids (and the corresponding number of carbons) found in MCTs are caproic acid (C₆), caprylic acid (Cs), capric acid (C₁₀) and lauric acid (C₁₂). MCTs are composed of a glycerol backbone and three of these fatty acids. The approximate ratios of these fatty acids in commercial MCT products derived from coconut oil are 2(C₆):55(C₈):42(C₁₀):1(C₁₂). MCTs are also known as 1,2,3-Propanetriol Trioctanoate, AC-1202, Capric Acid, Caproic Acid, Caprylic Acid, Caprylic Triglycerides, Lauric Acid, MCT, MCT's, Medium-Chain Triacylglycerols, Medium-Chain Triglycerides, Tricaprylin, Triglycérides à Chaine Moyenne, Triglicéridos de Cadena Media (TCMs), and Trioctanoin.

Soybean oil is a vegetable oil extracted from the seeds of the soybean (Glycine max), and is used in cosmetics as an emollient. Soybean oil contains 61% polyunsaturated fat and 24% monounsaturated fat, and consists of mostly glycerides of linoleic, oleic, linolenic and plamitic acids. Super refined soybean oil has been purified to remove impurities. While conventional soybean oil is pale yellow in appearance, super refined soybean oil is a water-white, odourless, neutral non-polar lipid with a low peroxide value. Examples of commercially available super refined soybean oil include that may be included in the composition include Cropure^{™} Soybean, Super Refined^{™} Soybean, Super Refined^{™} Soybean EP NP, Super Refined^{™} Soybean USP, Super Refined^{™} Soybean USP NP, and Super Refined^{™} Soybean USP JP NP.

In some embodiments, the topically administered pharmaceutical composition comprises one or more cosmetic agents. Cosmetic agents may be provided in different skin tones so as to match the subject's skin and conceal the topically administered composition and wound. Alternatively, cosmetic agents may render the topical composition clear so as to conceal the topically administered composition.

In some embodiments the cosmetic agent comprises a shine control agent. Such agents include, but are not limited to, silicas, magnesium aluminum silicates, talc, sericite and various organic copolymers. Some specific examples of the silicates and carbonates useful in this present invention are more fully explained in Van Nostrand Reinhold's Encyclopedia of Chemistry, 4h Ed. pp155, 169, 556, and 849 (1984). Synthetic versions of the shine control agents, particularly silicates, are preferred. Suitable synthetic carbonates are commercially available from Mallinckrodt or Whittaker, Clarke & Daniels. Examples of synthetic silicates useful in the compositions are Hubersorb 250 or Hubersorb 600 , available from JM Huber. Particularly effective shine control agents include silicates or carbonates that are formed by reaction of a carbonate or silicate with the alkali (IA) metals, alkaline earth (IIA) metals, or transition metals, and silicas (silicon dioxide). Preferred shine control agents are selected from the group consisting of calcium silicates, amorphous silicas, calcium, carbonates, magnesium carbonates, zinc carbonates, and combinations thereof.

In some embodiments, the composition comprises (a) one or more aminosterols amounting to about 0.01 wt. % to about 10 wt. % of the total composition; (b) water; (c) glycerin at about 10 wt. % to about 30 wt. %; (d) decyl glucoside at about 2.5 wt. % to about 17.5 wt. %; (e) lauryl N-methyl glucamide at about 1 wt. % to about 15 wt %; (f) propionic acid at about 0.5 wt. % to about 5 wt. %; (g) isoleucine at about 0.2 wt. % to about 5 wt. %; and polyethylene glycol distearate at about 0.1 wt. % to about 5 wt. %.

In some embodiments, the composition comprises (a) one or more aminosterols amounting to about 0.05 wt. % to about 1 wt. % of the total composition; (b) water; (c) glycerin at about 15 wt. % to about 25 wt. %; (d) decyl glucoside at about 7.5 wt. % to about 12.5 wt. %; (e) lauryl N-methyl glucamide at about 5 wt. % to about 10 wt %; (f) propionic acid at about 2.5 wt. % to about 3.5 wt. %; (g) isoleucine at about 0.5 wt. % to about 3 wt. %; and polyethylene glycol distearate at about 0.6 wt. % to about 1 wt. %.

In some embodiments, the composition comprises (a) one or more aminosterols amounting to about 0.1 wt. % of the total composition; (b) water; (c) glycerin at about 20 wt. %; (d) decyl glucoside at about 10 wt. %; (e) lauryl N-methyl glucamide at about 7 wt. %; (f) propionic acid at about 3 wt. %; (g) isoleucine at about 1.5 wt. %; and polyethylene glycol distearate at about 0.8 wt. %.

Any pharmaceutical used for administration can be sterile. Sterility is readily accomplished by, for example, filtration through sterile filtration membranes (e.g., 0.2 micron membranes), heat sterilization, or sterilization via exposure to radiation. Any pharmaceutically acceptable sterility method can be used in the compositions of the disclosure.

The pharmaceutical composition comprising an aminosterol, a derivative, or salts thereof will be formulated and dosed in a fashion consistent with good medical practice, taking into account the clinical condition of the individual patient, the method of administration, the scheduling of administration, and other factors known to practitioners.

### A. Additional Active Agents

In some embodiments, the pharmaceutical composition comprising an aminosterol is administered in combination with at least one additional active agent to achieve either an additive or synergistic effect. The additional active agent may administered via a method selected from the group consisting of (a) concomitantly; (b) as an admixture; (c) separately and simultaneously or concurrently; and (d) separately and sequentially. The additional active agent may be a component of the pharmaceutical composition comprising the aminosterol, a component of a second pharmaceutical composition not comprising an aminosterol, or separately administered to the subject via any route of administration disclosed herein.

In some embodiments, the presence of at least one non-aminosterol additional active agent is to merely enhance the anti-aging and anti-wrinkle properties of the aminosterol composition. In another embodiment, the additional active agent comprises at least one non-aminosterol active agent for rendering the composition suitable as a cosmetic preparation. In one embodiment, the additional active agent comprises an anti-aging agent selected from vitamin E, vitamin A, vitamin C, vitamin B and derivatives thereof; a retinoid; an antioxidant; a plant extract or mixtures thereof.

In one embodiment, additional active agents are compounds that provide benefits to the skin and/or provide desirable properties to a composition formulated as a cosmetic or medicinal preparation. The non-aminosterol active agent can be a drug substance or a non-drug substance. Examples of non-drug active agents include, but are not limited to, skin lightening agents, tanning agents, skin conditioning agents, skin protectants, emollients and humectants, and sunscreen actives.

Examples non-aminosterol active agents useful in the compositions, such as a non-aminosterol active drug substance or an active cosmetic substance, include, but are not limited to, Botulinum toxin type A (Botox^{®}), a retinoid (e.g., vitamin A derivatives, retinol, retinal, tretinoin (retinoic acid, Renova^{®}, Retin-A^{®}), isotretinoin, alitretinoin, etretinate, acitretin, tazarotene (Avage^{®}, Tazorac^{®}), bexarotene and Adapalene), alpha hydroxy acids, beta hydroxy acids, poly hydroxy acids, hydroxyl acids, kinetin, coenzyme Q10, copper peptides, tea extracts (e.g., green, black and oolong tea extracts), antioxidants (e.g., ascorbic acid (vitamin C), glutathione, melatonin, tocopherols, α-tocopherol, tocotrienols (vitamin E), lipoic acid, uric acid, carotenes, ubiquinone (coenzyme Q), thioredoxin, Polyphenolic antioxidants (resveratrol, flavonoids), and carotenoids), colloidal oatmeal (Aveeno^{®}), soybean extract, or any mixture thereof. In one aspect, the antioxidant comprises an alpha hydroxy acid elected from lactic acid (e.g., DL lactic acid), glycolic acid, citric acid, malic acid, ascorbic acid, tartaric acid, or a combination thereof In one aspect, the antioxidant comprises a beta hydroxy acid selected from beta hydroxybutyric acid, beta phenyl lactic acid, DL lactic acid or a combination thereof

The additional active agent may be a sunscreen. Representative sunscreen drugs are active ingredients that absorb, reflect, or scatter radiation in the UV range at wavelengths from 290 to 400 nanometers. Specific examples include benzophenone-3 (oxybenzone), benzophenone-4 (sulisobenzone), benzophenone-8 (dioxybenzone), butyl methoxydibenzoylmethane (Avobenzone), DEA-methoxycinnamate (diethanolamine methoxycinnamate), ethyl dihydroxypropyl PABA (ethyl 4-[bis(hydroxypropyl)] aminobenzoate), ethylhexyl dimethyl PABA (Padimate O), ethylhexyl methoxycinnamate (octyl methoxycinnamate), ethylhexyl salicylate (octyl salicylate), homosalate, menthyl anthranilate (Meradimate), octocrylene, PABA (aminobenzoic acid), phenylbenzimidazole sulfonic acid (Ensulizole), TEA-salicylate (trolamine salicylate), titanium dioxide, and zinc oxide. One skilled in the art will appreciate that other sunscreen agents may be used in the compositions and preparations of the present invention.

The non-aminosterol active agent may comprise an antifungal. In one embodiment, the active agent comprises an antifungal selected from clotrimazole, tolnaftate, terbinafine hydrochloride or mixtures thereof. In one embodiment, the active agent comprises a analgesic or an anesthetic selected from benzocaine, lidocaine, novacaine, menthol, phenol, camphor, methyl salicylate or mixtures thereof.

The non-aminosterol active agent may comprise an antibiotic. In one embodiment, the active agent comprises an antibiotic selected from sulfacetamide sodium/sulfur, erythromycin, silver sulfadiazine, bacitracin, neomycin, polymyxin b, retapamulin, mupirocin, ozenoxacin, and combinations thereof.

As noted above, the additional active agent may comprise skin conditioning agents. Such agents comprise substances that enhance the appearance of dry or damaged skin, as well as materials that adhere to the skin to reduce flaking, restore suppleness, and generally improve the appearance of skin. Representative examples of skin conditioning agents include acetyl cysteine, N-acetyl dihydrosphingosine, acrylates/behenyl acrylate/dimethicone acrylate copolymer, adenosine, adenosine cyclic phosphate, adensosine phosphate, adenosine triphosphate, alanine, albumen, algae extract, allantoin and derivatives, aloe barbadensis extracts, aluminum PCA, amyloglucosidase, arbutin, arginine, azulene, bromelain, buttermilk powder, butylene glycol, caffeine, calcium gluconate, capsaicin, carbocysteine, carnosine, beta-carotene, casein, catalase, cephalins, ceramides, chamomilla recutita (matricaria) flower extract, cholecalciferol, cholesteryl esters, coco-betaine, coenzyme A, corn starch modified, crystallins, cycloethoxymethicone, cysteine DNA, cytochrome C, darutoside, dextran sulfate, dimethicone copolyols, dimethylsilanol hyaluronate, DNA, elastin, elastin amino acids, epidermal growth factor, ergocalciferol, ergosterol, ethylhexyl PCA, fibronectin, folic acid, gelatin, gliadin, beta-glucan, glucose, glycine, glycogen, glycolipids, glycoproteins, glycosaminoglycans, glycosphingolipids, horseradish peroxidase, hydrogenated proteins, hydrolyzed proteins, jojoba oil, keratin, keratin amino acids, and kinetin.

Other examples of skin conditioning agents include, but are not limited to, lactoferrin, lanosterol, lauryl PCA, lecithin, linoleic acid, linolenic acid, lipase, lysine, lysozyme, malt extract, maltodextrin, melanin, methionine, mineral salts, niacin, niacinamide, oat amino acids, oryzanbl, palmitoyl hydrolyzed proteins, pancreatin, papain, PEG, pepsin, phospholipids, phytosterols, placental enzymes, placental lipids, pyridoxal 5-phosphate, quercetin, resorcinol acetate, riboflavin, RNA, saccharomyces lysate extract, silk amino acids, sphingolipids, stearamidopropyl betaine, stearyl palmitate, tocopherol, tocopheryl acetate, tocopheryl linoleate, ubiquinone, vitis vinifera (grape) seed oil, wheat amino acids, xanthan gum, and zinc gluconate. Skin conditioning agents, other than those listed above, may also be used, as is readily appreciated by those skilled in the art. In one embodiment, the skin conditioning agent comprises an anti-acne agent, such as benzoyl peroxide, salicylic acid, a topical retinoid, a tetracycline, an erythromycin or a mixture thereof. In one aspect, the topical retinoid comprises retinol, tretionin, adapalene, isotretionin, azelaic acid, motretinide, tazarotene or a mixture thereof.

In some embodiments, the pharmaceutical composition comprising an aminosterol is administered with a wound healing promotor selected from an anabolic agent such as human growth hormone, insulin-like growth factor, insulin, or testosterone and related derivatives; an appetite promoter such as megestrol acetate, dronabinol, tetrahydrocannabinol, mirtazapine, cyproheptadine, clozapine, olanzapine, risperidone, and quetiapine; a selective estrogen receptor modulator such as tamoxifen and raloxifene. The wound healing promotor may be within the pharmaceutical composition or administered separately from the aminosterol composition.

### B. Stability of Compositions

The compositions can be stable when exposed to room temperature (e.g., about 25°C) and up to about 50°C. In other embodiments, the compositions are stable when exposed to a temperature selected from the group consisting of greater than about 50°C, about 55°C or greater than about 55°C, about 60°C or greater than about 60°C, about 65°C or greater than about 65°C, about 70°C or greater than about 70°C, about 75°C or greater than about 75°C, about 80°C or greater than about 80°C, or about 85°C or greater than about 85°C. The compositions may be stable at any of the preceding temperatures for about 1 month to about 6 months, about 6 months to about 12 months, about 12 months to about 24 months, about 24 months to about 3 years, about 3 years to about 6 years, about 6 years to about 12 years, or greater than about 12 years. In addition, the compositions can be stable when exposed to an elevated temperature of 50°C for a duration of 1 month and room temperature storage of 25°C for up to 3 years.

Exemplary thermostable surfactants and/or stabilizers may be in the composition. These include, but are not limited to, (1) sorbitan esters, such as Spans and Arlacel, (2) block polymers, such as Pluronics, (3) acrylic polymers, such as Pemulen, and (4) ethoxylated fatty esters, such as Cremophor RH-40.

### C. Kits and Drug Containers

Aminosterol formulations or compositions used in the methods of the disclosure may be packaged together with, or included in a kit along with instructions, a drug label, or a package insert. Such instructions or package inserts may address dosing protocol, recommended storage conditions, such as time, temperature and light, taking into account the shelf-life of the aminosterol or derivatives or salts thereof. Such instructions or package inserts may also address the particular advantages of the aminosterol or derivatives or salts thereof, such as the ease of storage for formulations that may require use in the field, outside of controlled hospital, clinic or office conditions.

The disclosure also provides methods employing a pharmaceutical pack or kit comprising one or more containers filled with one or more aminosterol pharmaceutical compositions disclosed herein. The kits may include, for instance, containers filled with an appropriate amount of an aminosterol pharmaceutical composition, either as a powder or a tablet, to be dissolved or mixed with a carrier composition, or as a sterile solution. Associated with such container(s) can be a notice in the form prescribed by a governmental agency regulating the manufacture, use or sale of pharmaceuticals or biological products, which notice reflects approval by the agency of manufacture, use or sale for human administration.

In addition, the aminosterol or a derivative or salt thereof may be employed in conjunction with other therapeutic compounds.

In one aspect, a drug container is provided. The drug container comprising a label and an aminosterol or a salt or derivative thereof, for the treatment of a disease or condition, or a symptom thereof in a subject, wherein the label requires that the subject not be lying down when the aminosterol is administered to the subject. The label may containing an adhesive back face and may be affixed to the drug container.

The label may contain written or pictorial instructions. In one embodiment, the label requires that the subject be sitting down or standing up during administration of the aminosterol. In some embodiments, the label requires the subject be not lying down, sitting, or standing, after aminosterol administration for a time period of about 1 minute to about 3 hours. In some embodiments, the time period is about 1 minute to about 10 min, about 10 min to about 20 min, about 20 min to about 30 min, about 30 min to about 40 min, about 40 min to about 50 min, about 50 min to about 60 min, about 60 min to about 70 min, about 70 min to about 80 min, about 80 min to about 90 min, about 90 min to about 100 min, about 100 min to about 110 min, about 110 min to about 120 min, about 120 min to about 130 min, about 130 min to about 140 min, about 140 min to about 150 min, about 150 min to about 160 min, about 160 min to about 170 min, or about 170 min to about 180 min.

In some embodiments, the label may indicate the condition or symptom which the aminosterol is used to treat. In some embodiments, (a) the symptom is selected from the group consisting of constipation, hallucinations, cognitive impairment, and inflammation; (b) the symptom is correlated with a synucleopathy, a neurodegenerative disease, a neurological disease or disorder, a psychological and/or behavior disorder, or a cerebral or general ischemic disorder or condition; (c) the disease or condition is a synucleopathy, neurodegenerative disease, or neurological disease or disorder; (d) the disease or condition is a psychological and/or behavior disorder; or (e) the disease or condition is a cerebral or general ischemic disorder or condition.

In another embodiment, (a) the synucleopathy, neurodegenerative disease, or neurological disease or disorder is selected from the group consisting of Parkinson's disease, Alzheimer's disease, schizophrenia, multiple system atrophy, Lewy body dementia, dementia with Lewy bodies, Huntington's Disease, Multiple Sclerosis, Amyotorphic Lateral Sclerosis, Friedreich's ataxia, vascular dementia, spinal muscular atrophy, supranuclear palsy, progressive nuclear palsy, frontotemporal dementia, progressive nuclear palsy, Guadeloupian Parkinsonism, spinocerebellar ataxia, parkinsonism, traumatic brain injury, degenerative processes associated with aging, and dementia of aging; (b) the psychological or behavior disorder is selected from the group consisting of depression, autism, autism spectrum disorder, down syndrome, Gaucher's disease, Krabbe's disease, lysosomal conditions affecting glycosphingolipid metabolism, ADHD, agitation, anxiety, delirium, irritability, illusion and delusions, amnesia, apathy, bipolar disorder, disinhibition, aberrant motor and obsessive-compulsive behaviors, addiction, cerebral palsy, epilepsy, major depressive disorder, and sleep disorders such as REM sleep behavior disorder (RBD), sleep fragmentation, REM behavior disorder, circadian rhythm dysfunction, sleep apnea, and cognitive impairment; or (c) the cerebral or general ischemic disorder or condition is selected from the group consisting of microangiopathy, intrapartum, cerebral ischemia, cerebral ischemia during/after cardiac arrest or resuscitation, cerebral ischemia due to intraoperative problems, cerebral ischemia during carotid surgery, chronic cerebral ischemia due to stenosis of blood-supplying arteries to the brain, sinus thrombosis or thrombosis of cerebral veins, cerebral vessel malformations, diabetic retinopathy, high cholesterol, myocardial infarction, cardiac insufficiency, cardiac failure, congestive heart failure, myocarditis, pericarditis, perimyocarditis, coronary heart disease, angina pectoris, congenital heart disease, shock, ischemia of extremities, stenosis of renal arteries, diabetic retinopathy, thrombosis associated with malaria, artificial heart valves, anemias, hypersplenic syndrome, emphysema, lung fibrosis, erectile dysfunction, cardiac conduction defects, high blood pressure, low blood pressure, and pulmonary edema.

In other aspects, a kit comprising a nasal spray device as described herein is used in the methods. In one aspect, the kit may comprise one or more devices as disclosed herein, comprising a disclosed low dose aminosterol composition, wherein the device is sealed within a container sufficient to protect the device from atmospheric influences. The container may be, for example, a foil, or plastic pouch, particularly a foil pouch, or heat sealed foil pouch. Suitable containers sufficient to adequately protect the device will be readily appreciated by one of skill in the art.

In one aspect, the kit may comprise one or more drug containers (i.e., a roll-on applicator, a spray pump, a lipstick, etc.) comprising the aminosterol composition, as disclosed herein, wherein the drug containers may be sealed within a first protective packaging, or a second protective packaging, or a third protective packaging, that protects the physical integrity of the drug containers. One or more of the first, second, or third protective packaging may comprise a foil pouch. The kit may further comprise instructions for use of the article. In one aspect, the kit contains two or more drug containers.

In one aspect, the kit may comprise a drug containers as disclosed herein, and may further comprise instructions for use. The instructions may be present on a drug label. The drug label may comprise and adhesive back face affixed to the container. In one aspect, the instructions may comprise visual aid/pictorial and/or written directions to an administrator of the composition.

### D. Methods of Making Compositions

Compositions of the present disclosure may be prepared by mixing one or more aminosterols with one or more of the composition components disclosed herein (see, for example, Section II.E). In one embodiment, the composition can be made by a preparation method comprising the following steps: (a) providing the one or more aminosterol and one or more composition components; and (b) mixing the aminosterol and the one or more composition components at room temperature without the use of heat until complete dissolution of the aminosterol compound. In one aspect, room temperature is between about 20° C. to 25° C.

In one embodiment, the composition can be made by a method comprising the following steps: (a) providing one or more aminosterol and one or more composition components; and (b) mixing the aminosterols and the one or more composition components with heat until complete dissolution of the aminosterols. In one aspect, the aminosterols and the one or more compounds are mixed at temperatures above about 25° C., at temperatures above about 35° C., 45° C., 55° C., 65° C., 75° C., 85° C. or 95° C.

In some embodiments, the preparation method further comprises mixing additional composition components after dissolution of the aminosterol composition in the composition components of steps (a) and (b).

In one embodiment, the aminosterols comprise, based on the total weight of the composition, from about 0.01 percent to about 5 percent, from about 0.05 percent to about 1 percent, from about 0.075 percent to 0.5 percent, from about 0.09 percent to 0.15 percent, or about 0.1 percent of the composition's weight.

In the methods of the disclosure, including compositions, the composition can be made by a preparation method comprising the following steps: (a) providing one or more aminosterols, a solubility enhancing agent and one or more composition components; (b) dissolving the aminosterols into a solution comprising a solubility enhancing agent; and (c) mixing the solution of step (b) into the one or more composition components until complete dissolution of the aminosterols. In one aspect, the solubility enhancing agent is selected from the group consisting of an ethanol, a polyol or a mixture thereof. The polyol can be propylene glycol, glycerol and/or polyethylene glycol. In one aspect, the solution is an aqueous solution. In some embodiments, the preparation method further comprises mixing additional composition components with the mixture formed in step (c).

### E. Patient Populations

The disclosed compositions can be used on a range of subjects or patients, including human and non-human animals, including mammals, as well as immature and mature animals, including human children and adults. In some embodiments, the subject may be suffering from a an impediment to normal cutaneous or mucosal wound healing.

The impediment to normal cutaneous wound healing may be the result of poor oxygenation, infection, presence of a foreign body near the wound site, venous insufficiency, old-age, hormone imbalance, stress, ischemia, diabetes, keloids, fibrosis, hereditary healing disorders, jaundice, uremia, obesity, alcoholism, smoking, cancer, AIDS, and poor nutrition. In some embodiments, impediment to normal wound healing is due to a medication taken by the subject. Examples of medications that impede wound healing include, glucocorticoid steroids, non-steroidal anti-inflammatory drugs, and chemotherapy.

In embodiments disclosed herein relating to prevention, particular patient populations may be selected based on being "at risk for" the development of one or more disorders. For example, genetic markers of Alzheimer's disease (e.g. APOE4) or family history may be used as signs to identify subjects likely to develop Alzheimer's disease. Thus, in some embodiments relating to disorders for which certain genetic or hereditary signs are known, prevention may involve first identifying a patient population based on one of the signs. Alternatively, certain symptoms are considered early signs of particular disorders. For example, constipation is considered an early sign of Parkinson's disease. Thus, in some embodiments relating to Parkinson's disease, a patient population may be selected for being "at risk" for developing Parkinson's disease based on age and experiencing constipation. An exemplary population is young adults between the ages of about 20 and about 40 experiencing constipation characterized by less than 3 bowel movements per week. These patients can be targeted and monitored for prevention of Parkinson's disease onset. Further genetic or hereditary signs may be used to refine the patient population.

### VIII. Definitions

The following definitions are provided to facilitate understanding of certain terms used throughout this specification.

Technical and scientific terms used herein have the meanings commonly understood by one of ordinary skill in the art, unless otherwise defined. Any suitable materials and/or methodologies known to those of ordinary skill in the art can be utilized in carrying out the methods described herein.

As used in the description of the disclosure and the appended claims, the singular forms "a", "an" and "the" are used interchangeably and intended to include the plural forms as well and fall within each meaning, unless the context clearly indicates otherwise. Also, as used herein, "and/or" refers to and encompasses any and all possible combinations of one or more of the listed items, as well as the lack of combinations when interpreted in the alternative ("or").

As used herein the term "aminosterol" refers to an amino derivative of a sterol. Non-limiting examples of suitable aminosterols for use in the composition and methods disclosed herein are aminosterol 1436, squalamine, aminosterols isolated from *Squalus acanthias*, and isomers, salts, and derivatives each thereof.

The term "administering" as used herein includes prescribing for administration as well as actually administering, and includes physically administering by the subject being treated or by another.

The term "about" will be understood by persons of ordinary skill in the art and will vary to some extent depending upon the context in which it is used. If there are uses of the term which are not clear to persons of ordinary skill in the art given the context in which it is used, "about" will mean up to plus or minus 10% of the particular term. For example, in some embodiments, it will mean plus or minus 5% of the particular term. Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number, which, in the context in which it is presented, provides the substantial equivalent of the specifically recited number.

As used herein "subject," "patient," or "individual" refers to any subject, patient, or individual, and the terms are used interchangeably herein. In this regard, the terms "subject," "patient," and "individual" includes mammals, and, in particular humans. When used in conjunction with "in need thereof," the term "subject," "patient," or "individual" intends any subject, patient, or individual having or at risk for a symptom, disorder, or wound.

As used herein, the phrase "therapeutically effective," "pharmaceutically effective," or "effective" in context of a "dose" or "amount" means a dose or amount that provides the specific pharmacological effect for which the compound or compounds are being administered. It is emphasized that a therapeutically effective amount will not always be effective in achieving the intended effect in a given subject, even though such dose is deemed to be a therapeutically effective amount by those of skill in the art. For convenience only, exemplary dosages are provided herein. Those skilled in the art can adjust such amounts in accordance with the methods disclosed herein to treat a specific subject suffering from a specified symptom, disorder, or wound. The therapeutically effective amount may vary based on the route of administration and dosage form.

The terms "treatment," "treating," or any variation thereof includes reducing, ameliorating, or eliminating (i) a symptom, disorder, or wound and/or (ii) one or more symptoms or effects of a symptom, disorder, or wound.

The terms "prevention," "preventing," or any variation thereof includes reducing, ameliorating, or eliminating the risk of developing (i) a symptom, disorder, or wound and/or (ii) one or more symptoms or a symptom, disorder, or wound.

### EXAMPLES

### Example 1: Gut rejuvenation using aminosterols

The purpose of this example was to evaluate the effect of topical application of an aminosterol to gut mucosal tissue.

The present example evaluated the impact of oral dosing of squalamine (ENT-01) on the gastrointestinal (GI) tissue of old mice (78 weeks). As ENT-01 exhibits minimal absorption (less than 0.3% in blood), oral administration results in topical administration to gut tissue. After 2 weeks of dosing the animals were euthanized, and the GI tracts sectioned into stomach, duodenum, jejunum, ileum, caecum, colon, and rectum. The stomach tissues were then sent for histology, and the transcriptomes analyzed by RNAseq.

The dosing schedule used to determine the effect of orally administered squalamine and MSI-1436 on the GI tracts of young and old mice was as follows. Male C57Bl/6 mice, aged 20 and 78 weeks, were obtained from Jackson labs. Animals were exposed to 12hr light dark cycles and provided Teklad standard mouse diet and water ad lib. Animals were assigned to the treatment groups shown in Table 2.

| **Table 2** | | | | | |
|---|---|---|---|---|---|
| **Group** | **Dose Level*** (mg/kg/day) | **Concentration** (mg/ml) | **Dose Volume** (ml/kg) | **Age on Day 1** (weeks) | **Number of Animals** |
| | | | | | Male |
| 1. Control (vehicle) | 0 | 0 | 100 | 20 | 5 |
| 2. Squalamine | 40 | 4 | 100 | 20 | 5 |
| 3. MSI-1436 | 40 | 4 | 100 | 20 | 5 |
| 4. Control (vehicle) | 0 | 0 | 100 | 78 | 5 |
| 5. Squalamine | 40 | 4 | 100 | 78 | 5 |
| 6. MSI-1436 | 40 | 4 | 100 | 78 | 5 |

Animals were dosed once daily by oral gavage in the morning for a total of 14 days. Animals were fasted 3-4 hours prior to dosing and 1 hour following. The test article was disolved in 0.5% hydroxypropylcellulose in water. On day 15 the animals were euthanized by CO₂ asphyxiation, necropsied, and tissues prepared for histology and RNAseq analysis.

The GI tracts of the animals were sectioned into stomach, duodenum, jejunum, ileum, caecum, colon, and rectum. The tissues were then sent for histology, and the transcriptomes analyzed by RNAseq.

Aging involves a depletion of gene expression in the gut. Comparison of the images showing mucosal tissue in the stomach of a young mouse (20 week, Fig. 1A) versus an old mouse (78 week, Fig. 1B) shows a reduced thickness of the mucosal layer in the older specimen. This reduction in mucosa is associated with a reduced RNA transcriptome in the stomach in aged mice (78 weeks) vs. young mice (20 weeks), see Table 3 below, which shows the respective mRNA amounts in young and old mouse stomach.

Table 3 shows a significant *decrease* in expression of certain genes as the mouse ages. Universally, *every single gene tested* shows a significant decrease in expression in aged tissue as compared to young tissue. This decrease in expression is observed for genes having a known epithelial barrier function as well as for genes associated with muscle tissue.

| **Table 3: Respective mRNA amounts in young and old mouse stomach** | | | |
|---|---|---|---|
| | **Young** | **Old** | **P Value** |
| **Epithelial Barrier Functions** | | | |
| caspase_14 | 9.228 | 0.835 | 8.45E-10 |
| collagen_type_XVII_alpha_1 | 9.835 | 2.804 | 2.07E-04 |
| Corneodesmosin | 12.75 | 4.295 | 6.74E-04 |
| Cornifelin | 23.74 | 6.442 | 3.01E-05 |
| cystatin_E/M | 9.41 | 1.551 | 5.68E-07 |
| Dermokine | 117.5 | 23.86 | 1.34E-07 |
| desmocollin_1 | 16.09 | 4.175 | 2.6E-05 |
| desmoglein_1_beta | 9.592 | 2.625 | 1.41E-04 |
| Filaggrin | 21.67 | 2.207 | 3.81E-11 |
| gap_junction_protein_beta_4 | 1.882 | 0.239 | 2.52E-04 |
| gap_junction_protein_beta_6 | 2.125 | 0.298 | 2.11E-04 |
| H19_imprinted_maternally_expressed_transcript | 39.95 | 1.909 | 1.59E-17 |
| Hornerin | 13.6 | 3.519 | 3.23E-05 |
| kallikrein_related-peptidase_7_(chymotryptic_stratum_ | 10.14 | 0.656 | 1.33E-11 |
| keratin_1 | 332.6 | 69.79 | 1.72E-07 |
| keratin_10 | 337.7 | 45.93 | 8.03E-11 |
| keratinocyte_differentiation_ associated_protein | 184.6 | 37.58 | 1.21E-07 |
| keratinocyte_expressed_proline-rich | 12.93 | 2.267 | 3.87E-07 |
| late_cornified_envelope_1A1 | 17.85 | 2.625 | 1.42E-08 |
| late_cornified_envelope_1A2 | 23.43 | 4.056 | 6.01E-08 |
| late_cornified_envelope_1B | 9.774 | 1.491 | 2.05E-07 |
| late_cornified_envelope_1C | 10.93 | 1.73 | 1.90E-07 |
| late_cornified_envelope_1E | 5.767 | 1.074 | 1.23E-05 |
| late_cornified_envelope_1F | 8.742 | 1.551 | 1.67E-06 |
| late_cornified_envelope_1G | 8.378 | 1.611 | 4.59E-06 |
| late_cornified_envelope_1H | 5.282 | 1.312 | 2.14E-04 |
| late_cornified_envelope_1I | 8.682 | 1.074 | 3.37E-08 |
| late_cornified_envelope_1J | 5.16 | 0.895 | 1.01E-05 |
| late_cornified_envelope_1L | 4.553 | 0.596 | 1.82E-06 |
| late_cornified_envelope_1M | 16.76 | 1.133 | 5.22E-13 |
| late_cornified_envelope_3C | 5.525 | 1.491 | 3.92E-04 |
| late_cornified_envelope_3E | 5.889 | 1.491 | 3.92E-04 |
| late_cornified_ envelope_3F | 10.81 | 3.281 | 2.66E-04 |
| lectin_galactose_binding_soluble_7 | 139.3 | 25.77 | 2.70E-08 |
| Loricrin | 275.3 | 45.75 | 3.37E-09 |
| Sciellin | 11.11 | 3.221 | 1.56E-04 |

| **Muscle Tissue** | | | |
|---|---|---|---|
| Myoglobin | 47.9 | 2.923 | 4.10E-16 |
| myosin_binding_protein_C_slow-type | 15.06 | 1.133 | 4.01E-12 |
| myosin_heavy_polypeptide_1_skeletal_muscle | 73.46 | 2.982 | 3.47E-20 |
| myosin_heavy_polypeptide_8_skeletal_muscle | 90.46 | 3.34 | 2.67E-21 |
| myosin_light_chain_phosphorylatable_fast_ske | 30.23 | 4.235 | 1.42E-09 |
| myosin_light_polypeptide_3 | 26.77 | 1.312 | 2.72E-16 |
| myozenin_1 | 6.617 | 0.418 | 3.87E-10 |
| myozenin_2 | 4.31 | 0.239 | 7.95E-09 |
| titin-cap | 17.36 | 0.418 | 3.66E-18 |

As shown in Table 4 below, mRNA levels for all of the genes in the table showed a significant increase after treatment with squalamine. This suggests that squalamine, and by extension structurally related aminosterols, have a rejuvenating effect in the mucosa of the gut.

| **Table 4: respective mRNA amounts in old mice versus old mice treated with squalamine (ENT-01)** | | | | |
|---|---|---|---|---|
| | **Old** | **Old+E nt-01** | **P Value** | **% increase** |
| **Epithelial Barrier Functions** | | | | |
| caspase_14 | 0.795 | 6.077 | 2.25E-07 | 664% |
| collagen_type_XVII_alpha_1 | 2.668 | 9.509 | 1.43E-04 | 256% |
| Corneodesmosin | 4.087 | 7.933 | 4.10E-02 | 94% |
| Cornifelin | 6.131 | 22.17 | 4.07E-05 | 261% |
| cystatin_E/M | 1.476 | 8.721 | 6.89E-07 | 491% |
| Dermokine | 22.71 | 88.45 | 5.65E-06 | 290% |
| desmocollin_1 | 3.974 | 8.102 | 2.83E-02 | 104% |
| desmoglein_1_beta | 2.498 | 7.089 | 2.14E-03 | 184% |
| Filaggrin | 2.1 | 16.32 | 2.05E-09 | 671% |
| gap_junction_protein_beta_4 | 0.227 | 1.294 | 3.15E-03 | 470% |
| gap_junction_protein_beta_6 | 0.284 | 1.575 | 1.59E-03 | 455% |
| H19_imprinted_maternally_expressed_transcr ipt | 1.817 | 13.73 | 5.79E-09 | 656% |
| Hornerin | 3.349 | 11.82 | 1.09E-04 | 253% |
| kallikrein_related-peptidase_7_(chymotryptic_stratum | 0.624 | 5.908 | 3.74E-08 | 847% |
| keratin_1 | 66.42 | 348.9 | 3.28E-08 | 425% |
| keratin_10 | 43.71 | 310.3 | 1.56E-10 | 610% |
| keratinocyte_differentiation_associated_protei n | 35.77 | 181.2 | 7.27E-08 | 407% |
| keratinocyte_expressed_proline-rich | 2.157 | 7.99 | 1.36E-04 | 270% |
| late_cornified_envelope_1A1 | 2.498 | 10.97 | 1.03E-05 | 339% |
| late_cornified_envelope_1A2 | 3.86 | 17.78 | 2.08E-06 | 361% |
| late_cornified_envelope_1B | 1.419 | 8.102 | 1.33E-06 | 471% |
| late_cornified_envelope_1C | 1.646 | 7.202 | 3.26E-05 | 338% |
| late_cornified_envelope_1E | 1.022 | 4.501 | 1.17E-04 | 340% |
| late_cornified_envelope_1F | 1.476 | 6.47 | 3.92E-05 | 338% |
| late_cornified_envelope_1G | 1.533 | 4.557 | 2.78E-03 | 197% |
| late_cornified_envelope_1H | 1.249 | 4.164 | 1.38E-03 | 233% |
| late_cornified_envelope_1I | 1.022 | 7.708 | 8.29E-08 | 654% |
| late_cornified_envelope_1J | 0.852 | 3.376 | 6.62E-04 | 296% |
| late_cornified_envelope_1L | 0.568 | 2.644 | 5.13E-04 | 365% |
| late_cornified_envelope_1M | 1.079 | 8.158 | 6.04E-08 | 656% |
| late_cornified_envelope_3C | 1.419 | 3.263 | 2.79E-02 | 130% |
| late_cornified_envelope_3E | 1.419 | 4.332 | 2.50E-03 | 205% |
| late_cornified_envelope_3F | 3.122 | 9.115 | 1.15E-03 | 192% |
| lectin_galactose_binding_soluble_7 | 24.52 | 142.5 | 7.67E-09 | 481% |
| Loricrin | 43.54 | 209.5 | 1.63E-07 | 381% |
| Sciellin | 3.066 | 9.79 | 4.25E-04 | 219% |

| **Muscle Tissue** | | | | |
|---|---|---|---|---|
| Myoglobin | 2.782 | 17.72 | 2.49E-08 | 437% |
| myosin binding_protein C slow-type | 1.079 | 3.173 | 1.42E-03 | 209% |
| myosin_heavy_polypeptide_1_skeletal_muscl e | 2.838 | 11.03 | 4.18E-05 | 289% |

| **Table 4: respective mRNA amounts in old mice versus old mice treated with squalamine (ENT-01)** | | | | |
|---|---|---|---|---|
| | **Old** | **Old+E nt-01** | **P Value** | **% increase** |
| myosin_heavy_polypeptide_8_skeletal_muscl e | 3.179 | 14.97 | 2.20E-06 | 371% |
| myosin_light_chain_phosphorylatable_fast_s ke | 4.031 | 15.92 | 1.79E-05 | 295% |
| myosin_light_polypeptide_3 | 1.249 | 10.58 | 4.49E-09 | 747% |
| myozenin_1 | 0.397 | 2.476 | 1.19E-04 | 524% |
| myozenin_2 | 0.227 | 1.519 | 8.72E-04 | 341% |
| titin-cap | 0.397 | 3.376 | 3.15E-06 | 298% |

The results detailed in Tables 3 and 4 are dramatic. Specifically, topical administration or application of an aminosterol to mucosal tissue resulted in increased expression of *every single Epithelial Barrier gene and muscle tissue gene measured.* Further, the increases in protein expression were significant and dramatic, and ranged from a low of a 94% increase in protein expression to a high of an 847% increase in protein expression upon topical exposure to an aminosterol.

### Example 2: Topical pharmaceutical composition preparation

The purpose of the example was to describe preparation of an exemplary topical dosage form of an aminosterol.

A carrier solution was prepared by mixing water, glycerin, decyl glucoside, lauryl N-methyl glucamide, propionic acid, isoleucine, and PEG distearate to arrive at a carrier solution having the following weight percentages of each component: water (57.7%), glycerin (20%), decyl glucoside (10%), lauryl N-methyl glucamide (7%), propionic acid (3%), isoleucine (1.5%), and PEG distearate (0.8%).

Squalamine lactate was then dissolved into the carrier solution to arrive at a 0.1% squalamine by weight composition. In another variation, squalamine lactate was dissolved into the carrier solution to arrive at a 0.4% squalamine by weight composition.

These compositions can be used in any suitable dosage form, such as an aerosol spray, nasal spray, aqueous pump, etc. for use in the methods described herein.

### Example 3: Topical treatment of wound using an aminosterol composition

The purpose of this example was to evaluate the effectiveness of topical application of an aminosterol composition to wound healing.

The 0.4 wt % squalamine-comprising composition described in Example 2 was applied to a three-week old deep knee abrasion for a human subject.

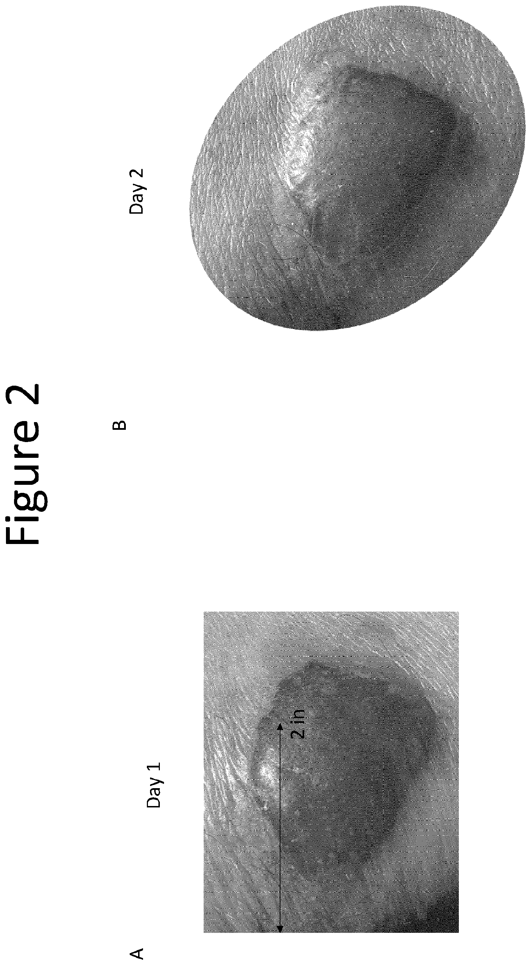

Fig. 2A shows a photo of the wound at Day 1 of treatment. The abrasion was initially scabbed over and included a warm, deep pink epithelialized surface.

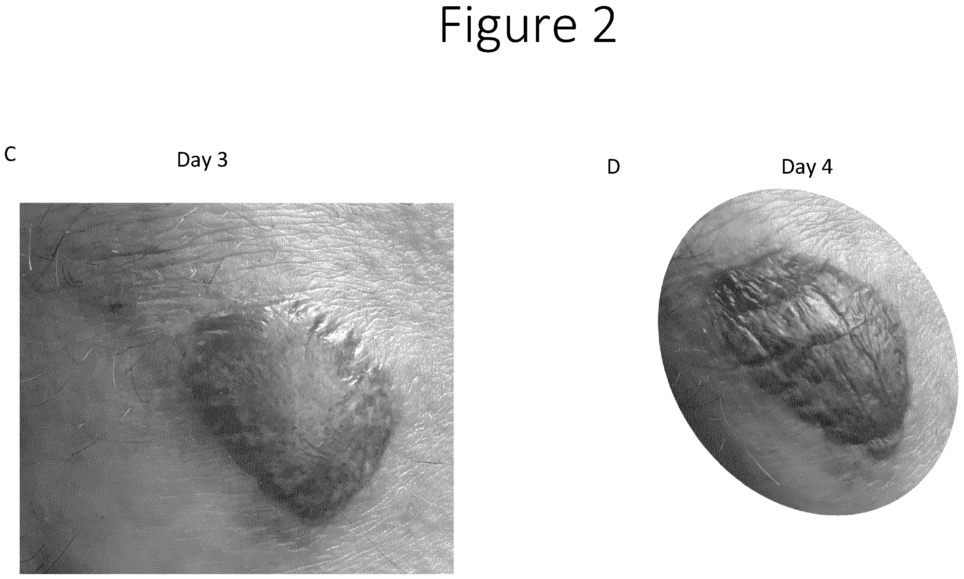

The squalamine comprising solution was sprayed on the abrasion. Within 24 hours of the first treatment (Day 2), a blister formed (see Fig. 2B). At 48 hours (Fig. 2C) and 72 hours (Fig. 2D) following the first treatment, the blister hardened and the wound continued healing.

These results demonstrate a dramatic rate of increased healing of a cutaneous wound following topical treatment with an aminosterol composition.

### Example 4: Aminosterol Dosage and Body Position

The purpose of this example was to evaluate the effectiveness of different dosing protocols relating to the physical position of a patient at the time of dosing and for a period of time following dosing. The drug administered was squalamine (ENT-01, which is a synthetic derivative of squalamine).

For the Phase 2(b) study, 47 patients were evaluated: 19 patients were administered 75 mg/daily (or three 25 mg pills) and 25 were administered 150 mg/daily (or four 25 mg pills) (3 patients withdrew from the study). The patients were enrolled at 24 different clinical sites. The subjects were all diagnosed with Parkinson's disease. The term of the study was 25 days for each patient at the indicated dosage.

Subjects were evaluated based upon the ability of the dosage to result in an increase in complete spontaneous bowel movements (CSBM). About 80% of the 29 patients with assessable bowel movements responded to drug treatment. For the 15 patients with assessable improvement in Mini-Mental State Exam (MMSE) scores, 5/15 (33%) showed an improvement of 3 or more points; 3/15 (20%) showed an improvement of 4 or more points; 2/15 (13%) showed an improvement of 5 or more points; 1/15 showed an improvement of 6 or more points. Patients also showed a reduction in hallucinations (including a complete elimination of hallucinations in several patients, as well as in increase in total sleep time (along with a decrease in sleep fragmentation).

Drug related adverse events are summarized in Table 5 below (n, %)

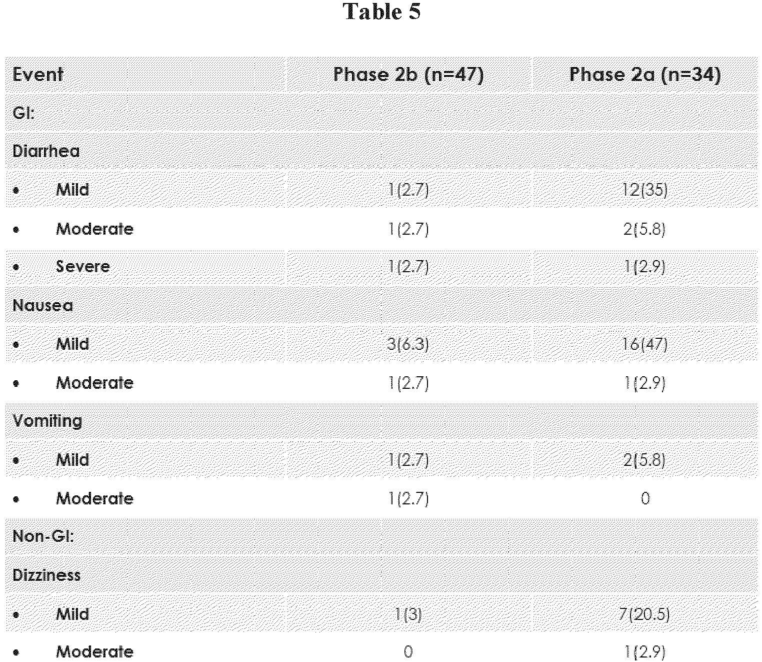

Unrelated drug adverse events are summarized in Table 6 below (n, %).

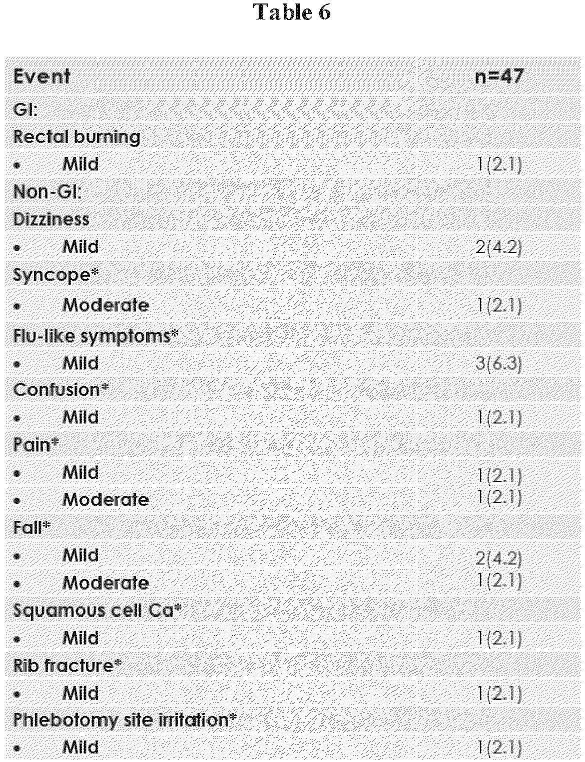

It was surprisingly found that patients taking the squalamine dose while in a sitting or standing position (as compared to lying down) had an *about 50% reduction* in severity and/or occurrence of adverse events. Further, patients taking the squalamine dose while in a sitting or standing position (as compared to lying down) also showed qualitative improvements in bowel function, MMSE scores, the amount and quality of sleep, as well as other tested neurological indices (e.g., using the UPDRS).

### References

Aarsland et al., "Neuropsychiatric symptoms in patients with Parkinson's disease and dementia: frequency, profile and associated care giver stress,"J. Neurol. Neurosurg. Psychiatry, 78:36-42 (2007).
Abbott et al., "Frequency of bowel movements and the future risk of Parkinson's disease," Neurology, 57(3):456-462 (2001).
Albert et al., "The Diagnosis of Mild Cognitive Impairment Due to Alzheimer's Disease: Recommendations from the National Institute on Aging-Alzheimer's Association Workgroups on Diagnostic Guidelines for Alzheimer's Disease," Alzheimer's & Dementia, 7(3):270-279 (2011).
Andresen et al., "Effect of 5 days linaclotide on transit and bowel function in females with constipation-predominant irritable bowel syndrome," Gastroenterology, 133:761-8 (2007).
Antonio-Rubio et al., "Abnormal thermography in Parkinson's disease," Parkinsonism Relat. Disord., 21:852-7 (2015).
Auyeung et al., "Ten year survival and outcomes in a prospective cohort of new onset Chinese Parkinson's disease patients," J. Neurol. Neurosurg. Psychiatry, 83:607-11 (2012).
Beach at al Neurol Ther. 2017 Jul; 6(Suppl 1): 5-13
Berg et al., "MDS Research Criteria for Prodromal Parkinson's Disease," Mov. Disord., 30:1600-1611 (2015).
Bhargava et al., "A phase I and pharmacokinetic study of squalamine, a novel antiangiogenic agent, in patients with advanced cancers," Clin. Cancer Res.,7:3912-9 (2001).
Braak et al., "Idiopathic Parkinson's disease: possible routes by which vulnerable neuronal types may be subject to neuroinvasion by an unknown pathogen," J. Neural. Transm. (Vienna), 110:517-36 (2003).
Braak et al., "Staging of brain pathology related to sporadic Parkinson's disease," Neurobiol. Aging, 24:197-211 (2003).
Braak et al., "Gastric alpha-synuclein immunoreactive inclusions in Meissner's and Auerbach's plexuses in cases staged for Parkinson's disease-related brain pathology," Neuroscience Letters, 396:67-72 (2006).
Breen et al., "Sleep and circadian rhythm regulation in early Parkinson disease," JAMA Neurol., 71:589-95 (2014).
Breen, D.P. & Lang, A.E., "Tracking the Course of Prodromal Parkinson's Disease," Brain, 140:259-262 (2017).
Chang et al., "A Meta-Analysis of Genome-Wide Association Studies Identifies 17 New Parkinson's Disease Risk Loci," Nat. Genet., 49:1511-1516 (2017).
Cordell et al., "Alzheimer's Association Recommendations for Operationalizing the Detection of Cognitive Impairment During the Medicare Annual Wellness Visit in a Primary Care Setting," Alzheimer's & Dementia, 9(2):141-150 (2013).
Darweesh et al., "Trajectories of Prediagnostic Functioning in Parkinson's Disease," Brain, 140:429-441 (2017).
Diederich etal., "Hallucinations in Parkinson disease," Nat. Rev. Neurol., 5:331-42 (2006).
Fahn S ER, Members of the UPDRS Development Committee. UNIFIED PARKINSON'S DISEASE RATING SCALE. Florham Park, NJ: Macmillan Health Care Information (1987).
Frank et al., "Psychometric validation of a constipation symptom assessment questionnaire," Scand. J. Gastroenterol., 34:870-7 (1999).
Friedman JH, Akbar U., "Psychosis in Parkinson's disease: unexplained observations in a seemingly simple model," Expert Rev. of Neurotherapeutics,16:595-6 (2016).
Gjerstad et al., "Excessive daytime sleepiness in Parkinson disease: is it the drugs or the disease?" Neurology, 67:853-8 (2006).
Goetz CG, Stebbins GT., "Risk factors for nursing home placement in advanced Parkinson's disease," Neurology, 43:2227-9 (1995).
Hao et al., "A Phase I and pharmacokinetic study of squalamine, an aminosterol angiogenesis inhibitor," Clin. Cancer Res., 9:2465-71 (2003).
Heaton et al., "Defecation frequency and timing, and stool form in the general population: a prospective study," Gut, 33:818-24 (1992).
Hilton, D., Stephens, M., Kirk, L. et al. "Accumulation of α-synuclein in the bowel of patients in the pre-clinical phase of Parkinson's disease," Acta. Neuropathol., 127:235 (2014).
Holmqvist et al., "Direct evidence of Parkinson pathology spread from the gastrointestinal tract to the brain in rats," Acta Neuropathol., 128:805-20 (2014).
Hughes et al., "Accuracy of clinical diagnosis of idiopathic Parkinson's disease: a clinico-pathological study of 100 cases," J. Neurol. Neurosurg. Psychiatry, 55:181-4 (1992).
Hughes et al., "Associations of Probable REM Sleep Behavior Disorder, Constipation, and Hyposmia with PD" (2017), in Movement Disorder Society: Proceedings of the International Congress of Parkinson's Disease and Movement Disorders; Marsili et al., 2018. Diagnostic Criteria for Parkinson's Disease: From James Parkinson to the Concept of Prodromal Disease. Front. Neurol., Online 23 Mar. 2018.
Jack et al., "Introduction to the Recommendations from the National Institute on Aging-Alzheimer's Association Workgroups on Diagnostic Guidelines for Alzheimer's Disease," Alzheimer's & Dementia, 7(3):257-262 (2011).
Jennings et al., "Hyposmic and Dopamine Transporter-Deficit Prodromal Cohort," JAMA Neurol., 74:933-940 (2017).
Jorm, A.F., "The Informant Questionnaire on Cognitive Decline in the Elderly (IQCODE): A Review," International Psychogeriatrics, 16:1-19 (2004).
Kim et al., "Poststroke Induction of α-Synuclein Mediates Ischemic Brain Damage," J Neurosci 36(26):7055-65 (2016).
Kirkevold, O. & Selbaek, G., "The Agreement Between the MMSE and IQCODE Tests in a Community-Based Sample of Subjects Aged 70 Years or Older Receiving In-Home Nursing: An Explorative Study," Dement Geriatr. Cogn. Dis. Extra, 5(1):32-41 (2015).
Koh et al., "Cerebral ischemic injury decreases α-synuclein expression in brain tissue and glutamate-exposed HT22 cells," Lab Anim Res. 33(3):244-250 (2017).
Lee, et al. "An instrument for the assessment of diarrhoeal severity based on a longitudinal community-based study." BMJ Open 2014
Lewis SJ, Heaton KW., "Stool form scale as a useful guide to intestinal transit time," Scand. J. Gastroenterol., 32:920-4 (1997).
Lin et al., "Risk of Parkinson's disease following severe constipation: a nationwide population-based cohort study," Parkinsonism Relat. Disord., 20:1371-5 (2014).
Madrid-Navarro et al., "Multidimensional Circadian Monitoring by Wearable Biosensors in Parkinson's Disease," Front. Neurol., 9:157 (2018).
Mahlknecht et al., "Prodromal Parkinson's Disease as Defined per MDS Research Criteria in the General Elderly Community," Mov. Disord., 31:1405-1408 (2016).
Marquis et al., "Development and validation of the Patient Assessment of Constipation Quality of Life questionnaire," Scand. J. Gastroenterol., 40:540-51 (2005).
Marsili et al., "Diagnostic Criteria for Parkinson's Disease: From James Parkinson to the Concept of Prodromal Disease," Front. Neurol., Online 23 Mar. 2018.
Mearin et al., "Bowel Disorders," Gastroenterology, 150(6):1393-1407 (2016).
McKhann et al., "The Diagnosis of Dementia Due to Alzheimer's Disease: Recommendations from the National Institute on Aging-Alzheimer's Association Workgroups on Diagnostic Guidelines for Alzheimer's Disease," Alzheimer's & Dementia, 7(3):263-269 (2011).
S. Morairty, "Detecting Neurodegenerative Diseases Before Damage Is Done," SRI International (July 26, 2013) (https://www.sri.com/blog/detecting-neurodegenerative-diseases).
Ondo et al., "Daytime sleepiness and other sleep disorders in Parkinson's disease," Neurology, 57:1392-6 (2001).
Ondo et al., "Placebo-controlled trial of lubiprostone for constipation associated with Parkinson disease," Neurology, 78:1650-4 (2012).
Ortiz-Tudela et al., "Ambulatory circadian monitoring (ACM) based on thermometry, motor activity and body position (TAP): a comparison with polysomnography," Physiol. Behav., 126:30-8 (2014).
Pagano, G., "Imaging in Parkinson's Disease," Clin. Med., 16:371-375 (2016).
Palsetia et al., "The Clock Drawing Test versus Mini-mental Status Examination as a Screening Tool for Dementia: A Clinical Comparison," Indian J. Psychol. Med., 40:1-10 (2018).
Papapetropoulos et al., "A questionnaire-based (UM-PDHQ) study of hallucinations in Parkinson's disease," BMC Neurol., 8:21 (2008).
Perni et al., "A natural product inhibits the initiation of alpha-synuclein aggregation and suppresses its toxicity," PNAS, USA, 114:E1009-E17 (2017).
Perni et al., "Multistep Inhibition of α-Synuclein Aggregation and Toxicity in Vitro and in Vivo by Trodusquemine," ACS Chem Biol., 13(8):2308-2319 (2018).
Phillips et al., "Alpha-synuclein-immunopositive myenteric neurons and vagal preganglionic terminals: autonomic pathway implicated in Parkinson's disease?" Neuroscience, 153:733-50 (2008).
Plassman et al., "Prevalance of Cognitive Impairment Without Dementia in the United States," Ann. Intern. Med., 148(6):427-434 (2009).
Postuma et al., "The New Definition and Diagnostic Criteria of Parkinson's Disease," Lancet Neurol., 15:546-548 (2016).
Postuma et al., "The New Definition and Diagnostic Criteria of Parkinson's Disease," Lancet Neurol. 15:546-548 (2016).
Rocca et al., "The Role of T1-Weighted Derived Measures of Neurodegeneration for Assessing Disability Progression in Multiple Sclerosis," Front Neurol., 8:433 (Sept. 4, 2017).
Sarabia et al., "Circadian rhythm of wrist temperature in normal-living subjects A candidate of new index of the circadian system," Physiol. Behav., 95:570-80 (2008).
Shehata et al., "Neuronal stimulation induces autophagy in hippocampal neurons that is involved in AMPA receptor degradation after chemical long-term depression," J. Neurosci., 32:10413-22 (2012).
Jon Stoessl, "Neuroimaging in the early diagnosis of neurodegenerative disease," Transl. Neurodegener., 1: 5 (2012).
Steer et al., "Use of the Beck Depression Inventory-II with depressed geriatric inpatients," Behav. Res. Ther., 38:311-8 (2000).
Stiasny-Kolster et al., "The REM sleep behavior disorder screening questionnaire--a new diagnostic instrument," Movement disorders : Official J. of the Movement Dis. Soc., 22:2386-93 (2007).
Stolzenberg et al. , "A Role for Neuronal Alpha-Synuclein in Gastrointestinal Immunity," J. Innate Immun., 9:456-63 (2017).
Svensson et al., "Does vagotomy reduce the risk of Parkinson's disease: The authors reply" Ann. Neurol., 78:1012-3 (2015).
Tang et al., "Loss of mTOR-Dependent Macroautophagy Causes Autistic-like Synaptic Pruning Deficits," Neuron, 83(5):1131-1143 (2014).
Videnovic A, Golombek D., "Circadian Dysregulation in Parkinson's Disease," Neurobiol. Sleep Circadian Rhythms, 2:53-8 (2017).
West et al., "Squalamine increases vagal afferent firing frequency in aging mice," J. of the Canadian Association of Gatroenterology, 1 (2018).
Wimo et al., "The worldwide economic impact of dementia 2010," Alzheimer's Dement., 9: 1-11 (2013).
Yun et al., "Identification of squalamine in the plasma membrane of white blood cells in the sea lamprey, Petromyzon marinus" December 2007 The Journal of Lipid Research, 48, 2579-2586.
Zahodne et al., "Mood and motor trajectories in Parkinson's disease: multivariate latent growth curve modeling," Neuropsychology, 26:71-80 (2012).
Zhao et al., "A comparative study of the amount of α-synuclein in ischemic stroke and Parkinson's disease," Neurol Sci. 37(5):749-54 (2016).
Zinsmeister et al., "Pharmacodynamic and clinical endpoints for functional colonic disorders: statistical considerations," Dig. Dis. Sci., 58:509-18 (2013).
Learning About Parkinson's Disease, NIH Nat'l Human Genome Research Inst., https://www.genome.gov/10001217/learning-about-parkinsons-disease/ (last updated Mar. 14, 2014); Parkinson Disease, NIH U.S. Nat'l Library of Med.: Genetics Home Reference, https://ghr.nlm.nih.gov/condition/parkinson-disease#genes (last updated July 3, 2018).

While certain embodiments have been illustrated and described, it should be understood that changes and modifications can be made therein in accordance with ordinary skill in the art without departing from the technology in its broader aspects as defined in the following claims.

The embodiments, illustratively described herein may suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the claimed technology. Additionally, the phrase "consisting essentially of" will be understood to include those elements specifically recited and those additional elements that do not materially affect the basic and novel characteristics of the claimed technology. The phrase "consisting of" excludes any element not specified.

The present disclosure is not to be limited in terms of the particular embodiments described in this application. Many modifications and variations can be made without departing from its spirit and scope, as will be apparent to those skilled in the art. Functionally equivalent methods and compositions within the scope of the disclosure, in addition to those enumerated herein, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled. It is to be understood that this disclosure is not limited to particular methods, reagents, compounds, or compositions, which can of course vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof, inclusive of the endpoints. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member.

All publications, patent applications, issued patents, and other documents referred to in this specification are herein incorporated by reference as if each individual publication, patent application, issued patent, or other document was specifically and individually indicated to be incorporated by reference in its entirety. Definitions that are contained in text incorporated by reference are excluded to the extent that they contradict definitions in this disclosure.

Other embodiments are set forth in the following .
1. A method of treating, preventing, and/or slowing the onset or progression of a disease or condition, or a symptom thereof, amenable to treatment with an aminosterol or a salt or derivative thereof in a subject in need, comprising administering to the subject a therapeutically effective amount of at least one aminosterol or a salt or derivative thereof,
   wherein the subject is not lying down.
2. A method of treating, preventing, and/or slowing the onset or progression of a disease or condition, or a symptom thereof, amenable to treatment with an aminosterol or a salt or derivative thereof in a subject in need, comprising administering to the subject a therapeutically effective amount of at least one aminosterol or a salt or derivative thereof while the subject is not lying down.
3. A method of treating, preventing, and/or slowing the onset or progression of a disease or condition, or a symptom thereof, amenable to treatment with an aminosterol or a salt or derivative thereof in a subject in need, comprising requiring the subject to not lie down and then administering to the subject a therapeutically effective amount of at least one aminosterol or a salt or derivative thereof.
4. The method of any one of embodiments 1-3, wherein the subject remains not lying down:
   (a) for about 5 minutes after administration of the at least one aminosterol or a salt or derivative thereof, and/or
   (b) for about 15 minutes after administration of the at least one aminosterol or a salt or derivative thereof; and/or
   (c) for about 30 minutes after administration of the at least one aminosterol or a salt or derivative thereof; and/or
   (d) for about 45 minutes after administration of the at least one aminosterol or a salt or derivative thereof; and/or
   (e) for about 60 minutes after administration of the at least one aminosterol or a salt or derivative thereof; and/or
   (f) for about 5 minutes to about 30 minutes after administration of the at least one aminosterol or a salt or derivative thereof; and/or
   (g) for about 30 to about 60 minutes after administration of the at least one aminosterol or a salt or derivative thereof.
5. The method of any one of embodiments 1-4, wherein the subject experiences fewer side effects, or one or more side effects are reduced in severity, as compared to a subject administered the at least one aminosterol or a salt or derivative thereof, that is lying down.
6. The method of embodiment 5, wherein the side effect is selected from the group consisting of gastrointestinal discomfort, diarrhea, nausea, vomiting, and dizziness.
7. The method of embodiment 5 or 6, wherein the severity of the side effect is reduced by about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%.
8. The method of any one of embodiments 1-7, wherein "lying down" is defined as the subject's body is predominantly flat, or in a horizontal, recumbent, or prostrate position.
9. The method of any one of embodiments 1-8, wherein the subject is standing up.
10. The method of any one of embodiments 1-8, wherein the subject is sitting down.
11. The method of any one of embodiments 1-10, wherein the aminosterol or a salt or derivative thereof is taken on an empty stomach, optionally within two hours of the subject waking.
12. The method of any one of embodiments 1-11, wherein no food is consumed after about 60 to about 90 minutes of administration of the at least one aminosterol or a salt or derivative thereof.
13. The method of any one of embodiments 1-12, wherein the therapeutically effective amount of the at least one aminosterol or a salt or derivative thereof comprises: (a) about 0.1 to about 20 mg/kg body weight of the subject; (b) about 0.1 to about 15 mg/kg body weight of the subject; (c) about 0.1 to about 10 mg/kg body weight of the subject; (d) about 0.1 to about 5 mg/kg body weight of the subject; (e) about 0.1 to about 2.5 mg/kg body weight of the subject; (f) about 0.001 to about 500 mg/day.; (g) about 0.001 to about 250 mg/day; (h) about 0.001 to about 125 mg/day; (i) about 0.001 to about 50 mg/day; (j) about 0.001 to about 25 mg/day; or (k) about 0.001 to about 10 mg/day.
14. The method of any one of embodiments 1-13, further comprising:
   (a) determining a dose of an aminosterol or a salt or derivative thereof for the subject, wherein the aminosterol dose is determined based on the effectiveness of the aminosterol dose in improving or resolving the symptom of the disease or condition,
   (b) followed by administering the aminosterol dose to the subject for a defined period of time, wherein the method comprises:
      (i) identifying the symptom;
      (ii) identifying a starting aminosterol dose for the subject; and
      (iii) administering an escalating dose of the aminosterol or a salt or derivative thereof to the subject over a defined period of time until an effective dose for the symptom is identified, wherein the effective dose is the aminosterol dose where improvement or resolution of the symptom is observed, and fixing the aminosterol dose at that level for that particular symptom in that particular subject.
15. The method of any one of embodiments 1-14, wherein administering comprises: (a) administration selected from the group consisting of oral, nasal, sublingual, buccal, rectal, vaginal, intravenous, intra-arterial, intradermal, intraperitoneal, intrathecal, intramuscular, epidural, intracerebral, intracerebroventricular, transdermal, pulmonary, inhalation, or any combination thereof; (b) nasal administration; (c) oral administration; or (d) non-oral administration.
16. The method of embodiment 14 or 15, wherein:
   (a) the aminosterol or a salt or derivative thereof is administered orally and the dose of the aminosterol or a salt or derivative thereof for the subject following escalation is fixed at a range of from about 1 mg up to about 500 mg;
   (b) the aminosterol or a salt or derivative thereof is administered orally and the starting aminosterol dosage ranges from about 1 mg up to about 150 mg/day;
   (c) the aminosterol or a salt or derivative thereof is administered orally and the dose of the aminosterol or a salt or derivative thereof for the subject following escalation is fixed at a range of from about 25 mg up to about 500 mg/day;
   (d) the aminosterol or a salt or derivative thereof is administered orally and the dosage of the aminosterol or a salt or derivative thereof is escalated in about 25 mg increments;
   (e) the aminosterol or a salt or derivative thereof is administered intranasally and the starting aminosterol dosage ranges from about 0.001 mg to about 3 mg/day;
   (f) the aminosterol or a salt or derivative thereof is administered intranasally and the dose of the aminosterol or a salt or derivative thereof for the subject following escalation is fixed at a range of from about 0.001 mg up to about 6 mg/day;
   (g) the aminosterol or a salt or derivative thereof is administered intranasally and the dose of the aminosterol or a salt or derivative thereof for the subject following escalation is a dose which is subtherapeutic when given orally or by injection;
   (h) the aminosterol or a salt or derivative thereof is administered intranasally and the dosage of the aminosterol or a salt or derivative thereof is escalated in increments of about 0.1, about 0.2, about 0.25, about 0.3, about 0.35, about 0.4, about 0.45, about 0.5, about 0.55, about 0.6, about 0.65, about 0.7, about 0.75, about 0.8, about 0.85, about 0.9, about 0.95, about 1, about 1.1, about 1.2, about 1.3, about 1.4, about 1.5, about 1.6, about 1.7, about 1.8, about 1.9, or about 2 mg;
   (i) the dosage of the aminosterol or a salt or derivative thereof is escalated every about 3 to about 5 days;
   (j) the dose of the aminosterol or a salt or derivative thereof is escalated every about 1, about 2, about 3, about 4, about 5, about 6, about 7, about 8, about 9, about 10, about 11, about 12, about 13, or about 14 days;
   (k) the dose of the aminosterol or a salt or derivative thereof is escalated about 1x/week, about 2x/week, about every other week, or about 1x/month;
   (l) the fixed dose of the aminosterol or a salt or derivative thereof is administered once per day, every other day, once per week, twice per week, three times per week, four times per week, five times per week, six times per week, every other week, or every few days;
   (m) the fixed dose of the aminosterol or a salt or derivative thereof is administered for a first defined period of time of administration, followed by a cessation of administration for a second defined period of time, followed by resuming administration upon recurrence of the symptom and/or a different related symptom;
   (n) the fixed aminosterol dose is incrementally reduced after the fixed dose of aminosterol or a salt or derivative thereof has been administered to the subject for a defined period of time;
   (o) the fixed aminosterol dose is varied plus or minus a defined amount to enable a modest reduction or increase in the fixed dose;
   (p) the fixed aminosterol dose is varied plus or minus a defined amount to enable a modest reduction or increase in the fixed dose, wherein the fixed aminosterol dose is increased or decreased by about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20%; and/or (q) the starting dose of the aminosterol or a salt or derivative thereof is higher if the symptom is severe.
17. The method of any one of embodiments 14-16, wherein:
   (a) the improvement or resolution of the symptom is measured quantitatively or qualitatively by one or more medically-recognized techniques, scales or tools; and/or
   (b) the improvement in the symptom is at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100%.
18. The method of any one of embodiments 14-16, wherein the symptom is selected from the group consisting of: (a) at least one non-motor aspect of experiences of daily living as defined by Part I of the Unified Parkinson's Disease Rating Scale selected from the group consisting of cognitive impairment, hallucinations and psychosis, depressed mood, anxious mood, apathy, features of dopamine dysregulation syndrome, sleep problems, daytime sleepiness, pain, urinary problems, constipation problems, lightheadedness on standing, and fatigue; (b) at least one motor aspect of experiences of daily living as defined by Part II of the Unified Parkinson's Disease Rating Scale selected from the group consisting of speech, saliva and drooling, chewing and swallowing, eating tasks, dressing, hygiene, handwriting, turning in bed, tremors, getting out of a bed, a car, or a deep chair, walking and balance, and freezing; (c) at least one motor symptom identified in Part III of the Unified Parkinson's Disease Rating Scale selected from the group consisting of speech, facial expression, rigidity, finger tapping, hand movements, pronation-supination movements of hands, toe tapping, leg agility, arising from chair, gait, freezing of gait, postural stability, posture, body bradykinesia, postural tremor of the hands, kinetic tremor of the hands, rest tremor amplitude, and constancy of rest tremor; (d) at least one motor complication identified in Part IV of the Unified Parkinson's Disease Rating Scale selected from the group consisting of time spent with dyskinesias, functional impact of dyskinesias, time spent in the off state, functional impact of fluctuations, complexity of motor fluctuations, and painful off-state dystonia; (e) constipation; (f) depression; (g) cognitive impairment; (h) sleep problem, sleep disorder, or sleep disturbance; (i) circadian rhythm dysfunction; (j) hallucinations; (k) fatigue; (l) REM disturbed sleep; (m) REM behavior disorder; (n) erectile dysfunction; (o) apnea; (p) postural hypotension; (q) correction of blood pressure or orthostatic hypotension; (r) nocturnal hypertension; (s) regulation of temperature; (t) improvement in breathing or apnea; (u) correction of cardiac conduction defect; (v) amelioration of pain; (w) restoration of bladder sensation and urination; (x) urinary incontinence; (y) control of nocturia; (z) schizophrenia; (aa) neurodegeneration, or a neurodegenerative or neurological disorder; (bb) autism; and/or (cc) an inflammatory disease or condition caused by excessive expression or concentration of alpha-synuclein in the subject.
19. The method of any one of embodiments 14-18, wherein the symptom is constipation, or optionally wherein the subject suffers from or is at risk of developing constipation, and wherein:
   (a) the method results in treating, preventing, and/or delaying the progression and/or onset of constipation in the subject; and/or
   (b) the aminosterol causes the subject to have a bowel movement; and/or
   (c) the method results in an increase in the frequency of bowel movement in the subject; and/or
   (d) the method results in an increase in the frequency of bowel movement in the subject and the increase in the frequency of bowel movement is defined as: (i) an increase in the number of bowel movements per week of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%; and/or (ii) a percent decrease in the amount of time between each successive bowel movement selected from the group consisting of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%; and/or
   (e) the fixed escalated aminosterol dose for constipation is defined as the aminosterol dose that results in a complete spontaneous bowel movement (CSBM) within 24 hours of dosing on at least 2 of 3 days at a given dose; and/or
   (f) as a result of the method the subject has the frequency of bowel movement recommended by a medical authority for the age group of the subject; and/or
   (g) the starting aminosterol dose is determined by the severity of the constipation, wherein: (i) if the average complete spontaneous bowel movement (CSBM) or spontaneous
   bowel movement (SBM) is one or less per week, then the starting aminosterol dose is at least about 150 mg; and/or (ii) if the average CSBM or SBM is greater than one per week, then the starting aminosterol dose is about 75 mg or less.
20. The method of any one of embodiments 14-18, wherein the symptom is depression, or optionally wherein the subject suffers from is or at risk of developing depression, and wherein:
   (a) the method results in treating, preventing, and/or delaying the progression and/or onset of depression in the subject; and/or
   (b) the method results in improvement in the subject's depression, as measured by one or more clinically-recognized depression rating scales; and/or
   (c) the method results in improvement in the subject's depression, as measured by one or more clinically-recognized depression rating scales and the improvement is in one or more depression characteristics selected from the group consisting of mood, behavior, bodily functions such as eating, sleeping, energy, and sexual activity, and/or episodes of sadness or apathy; and/or
   (d) the method results in improvement in the subject's depression, as measured by one or more clinically-recognized depression rating scales, and the improvement a subject experiences following treatment is about 5, about 10, about 15, about 20, about 25, about 30, about 35, about 40, about 45, about 50, about 55, about 60, about 65, about 70, about 75, about 80, about 85, about 90, about 95 or about 100%.
21. The method of any one of embodiments 14-18, wherein the symptom is cognitive impairment, or optionally wherein the subject suffers from is or at risk of developing cognitive impairment, and wherein:
   (a) the method results in treating, preventing, and/or delaying the progression and/or onset of cognitive impairment in the subject; and/or
   (b) progression or onset of the cognitive impairment is slowed, halted, or reversed over a defined period of time following administration of the aminosterol or a salt or derivative thereof, as measured by a medically-recognized technique; and/or
   (c) the cognitive impairment is positively impacted by the administration of the aminosterol or a salt or derivative thereof, as measured by a medically-recognized technique; and/or
   (d) the cognitive impairment is positively impacted by the administration of the aminosterol or a salt or derivative thereof, as measured by a medically-recognized technique and the positive impact on and/or progression of cognitive decline is measured quantitatively or qualitatively by one or more techniques selected from the group consisting of ADASCog, Mini-Mental State Exam(MMSE), Mini-cog test, Woodcock-Johnson Tests of Cognitive Abilities, Leiter International Performance Scale, Miller Analogies Test, Raven's Progressive Matrices, Wonderlic Personnel Test, IQ tests, or a computerized tested selected from Cantab Mobile, Cognigram, Cognivue, Cognision, and Automated Neuropsychological Assessment Metrics Cognitive Performance Test (CPT); and/or
   (e) the progression or onset of cognitive impairment is slowed, halted, or reversed by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%, as measured by a medically-recognized technique.
22. The method of any one of embodiments 14-18, wherein the symptom is a sleep problem, sleep disorder, or sleep disturbance, or optionally wherein the subject suffers from is or at risk of developing a sleep problem, sleep disorder, or sleep disturbance, and wherein:
   (a) the method results in treating, preventing, and/or delaying the progression and/or onset of the sleep problem, sleep disorder, or sleep disturbance in the subject; and/or
   (b) the sleep problem, sleep disorder, or sleep disturbance comprises a delay in sleep onset, sleep fragmentation, REM-behavior disorder, sleep-disordered breathing including snoring and apnea, day-time sleepiness, micro-sleep episodes, narcolepsy, circadian rhythm dysfunction, REM disturbed sleep, or any combination thereof;
   (c) the sleep problem, sleep disorder, or sleep disturbance comprises REM-behavior disorder, which comprises vivid dreams, nightmares, and acting out the dreams by speaking or screaming, or fidgeting or thrashing of arms or legs during sleep;
   (d) administration of the aminosterol decreases the occurrence of at least one symptom of the sleep disorder or disturbance;
   (e) the method results in a positive change in the sleeping pattern of the subject; wherein the positive change is defined as: (i) an increase in the total amount of sleep obtained of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%; and/or (ii) a percent decrease in the number of awakenings during the night selected from the group consisting of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%;
   (f) as a result of the method the subject obtains the total number of hours of sleep recommended by a medical authority for the age group of the subject;
   (g) the method results in a positive change in the sleeping pattern of the subject, and optionally wherein the positive change is defined as: (i) an increase in the total amount of sleep obtained of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%; and/or (ii) a percent decrease in the number of awakenings during the night selected from the group consisting of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%;
   (h) the sleep disorder comprises a loss of diurnal rhythm (Circadian rhythm), and optionally wherein the loss of diurnal rhythm is caused by: (i) dysfunction of the suprachiasmatic nucleus, and optionally wherein the aminosterol reverses the dysfunction of the suprachiasmatic nucleus, restores the diurnal rhythm, and treats the sleep disorder; (ii) dysfunction of the enteric nervous system, and optionally wherein the aminosterol reverses the dysfunction of the enteric nervous system, restores the diurnal rhythm, and treats the sleep disorder; (iii) dysfunction of the olfactory nervous system, and optionally wherein the aminosterol reverses the dysfunction of the olfactory system, restores the diurnal rhythm, and treats the sleep disorder; (iv) dysfunction of circadian rhythm caused by visual loss, and optionally wherein the aminosterol reverses the dysfunction of the circadian rhythm caused by visual loss; (v) dysfunction of circadian rhythm caused by jet lag, and optionally wherein the aminosterol reverses the dysfunction of the circadian rhythm caused by jet lag; and/or (vi) dysfunction of circadian rhythm caused by night-shift work, and optionally wherein the aminosterol reverses the dysfunction of the circadian rhythm caused by night-shift work; and/or
   (i) wherein the sleep disorder is associated with a neurodegenerative disorder, and wherein: (i) treating the sleep disorder prevents or delays the onset or progression of the neurodegenerative disorder; and/or (ii) the neurodegenerative disorder is selected from the group consisting of Alzheimer's disease, Parkinson's disease, Lewy Body dementia, frontotemporal dementia, supranuclear palsy, multi-system atrophy, Parkinsonism, amyotrophic lateral sclerosis (ALS), Huntington's Disease, schizophrenia, Friedreich's ataxia, Multiple sclerosis (MS), spinal muscular atrophy, progressive nuclear palsy, degenerative processes associated with aging, dementia of aging, Guadeloupian Parkinsonism, spinocerebellar ataxia, and vascular dementia.
23. The method of any one of embodiments 14-18, wherein the symptom is schizophrenia, or optionally wherein the subject is at risk of developing, or is suffering from, schizophrenia, and wherein:
   (a) the method results in treating, preventing, and/or delaying the progression and/or onset of schizophrenia in the subject; and/or
   (b) the method results in improvement in one or more schizophrenia characteristics or behaviors, as measured using a clinically recognized rating scale; and/or
   (c) the method results in improvement in one or more schizophrenia characteristics or behaviors, and wherein the schizophrenia characteristics or behaviors are selected from the group consisting of unclear or confusing thinking, reduced social engagement, reduced emotional expression, abnormal social behavior, failure to understand reality, lack of motivation, and hearing voices that others do not hear, as measured using a clinically-recognized scale; and/or
   (d) the method results in improvement in one or more schizophrenia characteristics or
   behaviors, and wherein the improvement a subject experiences in one or more schizophrenia characteristics or behaviors following treatment is about 5, about 10, about 15, about 20, about 25, about 30, about 35, about 40, about 45, about 50, about 55, about 60, about 65, about 70, about 75, about 80, about 85, about 90, about 95 or about 100%.
24. The method of any one of embodiments 14-18, wherein the symptom is hallucinations, or optionally wherein the subject suffers from, is or at risk of developing, hallucinations, and wherein:
   (a) the method results in treating, preventing, and/or delaying the progression and/or onset of hallucinations in the subject; and/or
   (b) the hallucinations comprise a visual, auditory, tactile, gustatory or olfactory hallucination; (b) the hallucinations are the result of: (i) a neurodegenerative or neurological disorder, and optionally the neurodegenerative or neurological disorder is selected from the group consisting of Alzheimer's disease, Parkinson's disease, Lewy Body dementia, frontotemporal dementia, supranuclear palsy, multi-system atrophy, Parkinsonism, amyotrophic lateral sclerosis (ALS), Huntington's disease, schizophrenia, Friedreich's ataxia, Multiple sclerosis (MS), spinal muscular atrophy, progressive nuclear palsy, degenerative processes associated with aging, dementia of aging, Guadeloupian Parkinsonism, spinocerebellar ataxia, and vascular dementia; (ii) a neurodegenerative or neurological disorder, wherein the neurodegenerative or neurological disorder is the result of a sleep disorder; a focal brain lesion; a focal brain lesion which is occipital lobe lesions or temporal lobe lesions; a temporal lobe lesion selected from the group consisting of lesions of the uncinate gyrus, cerebral peduncles, and substantia nigra; a diffuse involvement of the cerebral cortex; a diffuse involvement of the cerebral cortex caused by a viral infectious disease; a diffuse involvement of the cerebral cortex caused by a viral infectious disease, wherein the viral infectious disease is selected from the group consisting of acute metabolic encephalopathies, encephalitis, and meningitis; a diffuse involvement of the cerebral cortex caused by a cerebral vasculitis condition; a diffuse involvement of the cerebral cortex caused by a cerebral vasculitis condition, wherein the cerebral vasculitis condition is caused by an autoimmune disorder; a bacterial or viral infection, or a systemic vasculitis; and/or a diffuse involvement of the cerebral cortex caused by a cerebral vasculitis condition, wherein the cerebral vasculitis condition is caused by an autoimmune disorder which is Systemic Lupus Erythematosus (SLE); (ii) a psychiatric disorder, and optionally the psychiatric disorder is selected from the group consisting of bipolar disorder, borderline personality disorder, depression (mixed), dissociative identity disorder, generalized anxiety disorder, major depression, obsessive compulsive disorder, post-traumatic stress disorder, psychosis (NOS), schizoaffective disorder, and schizophrenia; (iii) a neurological disorder; (iv) a brain tumor; (v) a sensory loss, and optionally wherein the sensory loss is visual, auditory, gustatory, tactile, or olfactory; and/or (vi) dysfunction of the enteric nervous system; and/or
   (c) the method results in a decreased number or severity of hallucinations of the subject, and optionally wherein the decrease in number or severity in hallucinations is defined as a reduction in occurrences or severity of hallucinations selected from the group consisting of by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, and about 100%.; and/or; (d) the method results in the subject being hallucination-free.
25. The method of embodiment 24, wherein the aminosterol: (a) reverses dysfunction caused by the neurodegenerative or neurological disorder and treats and/or prevents the hallucination; (b) reverses dysfunction caused by the psychiatric disorder and treats and/or prevents the hallucination; (c) reverses dysfunction caused by the sensory loss and treats and/or prevents the hallucination; and/or (d) reverses dysfunction of the enteric nervous system and treats and/or prevents the hallucination.
26. The method of any one of embodiments 14-18, wherein the symptom is neurodegeneration, or optionally wherein the subject is at risk for developing, or is suffering from, neurodegeneration, and wherein:
   (a) the method results in treating, preventing, and/or delaying the progression and/or onset of neurodegeneration in the subject; and/or
   (b) the neurodegeneration is age-related; and/or
   (c) the neurodegeneration is correlated with age-related dementia; and/or
   (d) the neurodegeneration is correlated with a neurodisease; and/or
   (e) the neurodegeneration is correlated with one or more conditions or diseases selected from the group consisting of Alzheimer's disease, Parkinson's disease, Lewy body dementia, frontotemporal dementia, supranuclear palsy, multi-system atrophy, Parkinsonism, amyotrophic lateral sclerosis (ALS), Huntington's disease, schizophrenia, Friedreich's ataxia, Multiple sclerosis (MS), spinal muscular atrophy, progressive nuclear palsy, degenerative processes associated with aging, dementia of aging, Guadeloupian Parkinsonism, spinocerebellar ataxia, and vascular dementia; and/or
   (f) progression or onset of the neurodegeneration is slowed, halted, or reversed over a defined period of time following administration of the fixed escalated dose of the aminosterol or a salt or derivative thereof, as measured by a medically-recognized technique, optionally wherein the positive impact and/or progression of neurodegeneration is measured quantitatively or qualitatively by one or more techniques selected from the group consisting of electroencephalogram (EEG), neuroimaging, functional MRI, structural MRI, diffusion tensor imaging (DTI), [18F]fluorodeoxyglucose (FDG) PET, agents that label amyloid, [18F]F-dopa PET, radiotracer imaging, volumetric analysis of regional tissue loss, specific imaging markers of abnormal protein deposition, multimodal imaging, and biomarker analysis; and/or
   (g) the neurodegeneration is positively impacted by the fixed escalated dose of the aminosterol or a salt or derivative thereof, as measured by a medically-recognized technique, and optionally wherein the positive impact and/or progression of neurodegeneration is measured quantitatively or qualitatively by one or more techniques selected from the group consisting of electroencephalogram (EEG), neuroimaging, functional MRI, structural MRI, diffusion tensor imaging (DTI), [18F]fluorodeoxyglucose (FDG) PET, agents that label amyloid, [18F]F-dopa PET, radiotracer imaging, volumetric analysis of regional tissue loss, specific imaging markers of abnormal protein deposition, multimodal imaging, and biomarker analysis; and/or
   (h) the progression or onset of neurodegeneration is slowed, halted, or reversed by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%, as measured by a medically-recognized technique.
27. The method of any one of embodiments 14-18, wherein the symptom is autism or autism spectrum disorder or a related symptom, or optionally wherein the subject is at risk for developing, or is suffering from, autism or autism spectrum disorder, and wherein:
   (a) the method results in treating, preventing, and/or delaying the progression and/or onset of autism or autism spectrum disorder in the subject; and/or
   (b) the method results in improvement: (i) in one or more of the subject's autism or autism spectrum disorder characteristics or behaviors, as measured by a clinically-recognized rating scale; (ii) in one or more autism or autism spectrum disorder characteristics or behaviors selected from the group consisting of social skills, repetitive behaviors, speech, nonverbal communication, sensory sensitivity, behavior, social interaction, and communication skills, as measured using a clinically-recognized scale; and/or (iii) wherein the improvement a subject experiences following treatment in one or more autism or autism spectrum disorder characteristics or behaviors is about 5, about 10, about 15, about 20, about 25, about 30, about 35, about 40, about 45, about 50, about 55, about 60, about 65, about 70, about 75, about 80, about 85, about 90, about 95 or about 100%.
28. The method of any one of embodiments 14-18, wherein the symptom is an inflammatory disease or condition caused by excessive expression or concentration of alpha-synuclein in the subject, or optionally wherein the subject suffers from, is or at risk of developing, an inflammatory disease or condition caused by excessive expression or concentration of alpha-synuclein in the subject, and wherein:
   (a) the method results in treating, preventing, and/or delaying the progression and/or onset of the inflammatory disease or condition caused by excessive expression or concentration of alpha-synuclein in the subject; and/or
   (b) the method results in a decrease in intensity of inflammation, blood levels of inflammatory markers, inflammatory markers in tissue, number of inflammatory cells in tissue, or any combination thereof, as compared to a control or as compared to the qualitative or quantitative amount from the same patient or subject prior to treatment;
   (c) the method results in a decrease in concentration of alpha-synuclein in the subject, and optionally wherein the decrease in alpha-synuclein concentration in is measured qualitatively, quantitatively, or semi-quantitatively by one or more methods selected from the group consisting of: (i) first determining the concentration of alpha-synuclein in a tissue sample from the subject prior to treatment, followed by (aa) after treatment determining the alpha-synuclein concentration in the same tissue type from the same subject; or (bb) after treatment comparing the alpha-synuclein concentration in the same tissue type to a control; (ii) measuring the intensity of inflammation over time; (iii) measuring the amount of inflammatory markers over time; (iv) measuring the amount of inflammatory markers in blood, plasma, or tissue over time, either qualitatively or quantitatively; (v) measuring the amount of one or more inflammatory marker cytokines in blood, plasma, or tissue over time, either qualitatively or quantitatively; (vi) measuring the amount of one or more plasma markers of inflammation such as TNF, IL-8, or CRP in blood, plasma, or tissue over time, either qualitatively or quantitatively; and (vii) measuring the amount of inflammatory cells in blood, plasma, or tissue over time, either qualitatively or quantitatively;
   (d) the method results in a decrease in concentration of alpha-synuclein in the subject, wherein the decrease is about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%;
   (e) the method is applied to a patient population susceptible to excessive expression of alpha-synuclein, resulting in an excessive or high concentration of alpha-synuclein.
29. The method of any one of embodiments 14-28, wherein each defined period of time is independently selected from the group consisting of about 1 day to about 10 days, about 10 days to about 30 days, about 30 days to about 3 months, about 3 months to about 6 months, about 6 months to about 12 months, and greater than about 12 months.
30. A method of treating a cutaneous or mucosal wound in a subject in need comprising topically administering to the wound a therapeutically effective amount of a pharmaceutical composition comprising at least one aminosterol or a salt or derivative thereof
31. The method of embodiment 30, wherein the wound is an abrasion, a laceration, a puncture wound, an avulsion, or any combination thereof.
32. The method of embodiment 30 or 31, wherein:
   (a) the wound is present at a surgical site; and/or
   (b) the wound is the result of trauma; and/or
   (c) the trauma is a blast injury or gun shot wound; and/or
   (d) the wound is a burn wound.
33. The method of embodiment 32, wherein the burn would is a first, second, third, or fourth degree burn wound.
34. The method of embodiment 32 or 33, wherein the burn wound is a first-degree burn wound, and wherein the wound is substantially healed in less than about 10, less than about 9.5, less than about 9, less than about 8.5, less than about 8, less than about 7.5, less than about 7, less than about 6.5, less than about 6, less than about 5.5, less than about 5, less than about 4.5, less than about 4, less than about 3.5, or less than about 3 days.
35. The method of embodiment 32 or 33, wherein the burn wound is a second-degree burn wound, and wherein the wound is substantially healed in less than about 3, less than about 2.5, less than about 2, less than about 2.5, or less than about 1 week.
36. The method of any one of any one of embodiments 32, 33 or 35, wherein the burn wound is a second-degree burn wound, and wherein the wound is substantially healed in less than about 21, less than about 20, less than about 19, less than about 18, less than about 17, less than about 16, less than about 15, less than about 14, less than about 13, less than about 12, less than about 11, less than about 10, less than about 9, less than about 8, or less than about 7 days.
37. The method of any one of embodiments 30-36, wherein the rate or amount of healing of the wound is increased as compared to a similar wound not administered a composition comprising at least one aminosterol or salt or derivative thereof
38. The method of embodiment 37, wherein the wound healing is measured quantitatively or qualitatively by wound closure or by reference to a clinically recognized scale or tool.
39. The method of embodiment 37 or 38, wherein the method results in at least about 5%, at least about 10%, about 15%, about 20%, about 25%, about 30%, about 40%, about 45%, about 50%, about 55%, about 60%, about 65%, about 70%, about 75%, about 80%, about 85%, about 90%, about 95%, or about 100% increase in the rate or amount of wound healing, as compared to a similar wound not topically treated with a composition comprising at least one aminosterol or salt or derivative thereof.
40. The method of any one of embodiments 30-39, wherein the method reduces the severity and/or incidence of one or more complications or side effects associated with wounds, including but not limited to burn wounds, as compared to that observed in the absence of aminosterol administration.
41. The method of embodiment 40, wherein the complication or side effect is selected from the group consisting of a microbial infection, blood loss, shock, formation of scar tissue, hypothermia, hypovolemia, shock, pulmonary dysfunction, abdominal or extremity compartment syndrome, and cardiac failure in the subject,.
42. The method of embodiment 40 or 41, wherein the incidence and/or severity of the complication or side effect is decreased by about 5%, about 10%, about 15%, about 20%, about 25%, about 30%, about 40%, about 45%, about 50%, about 55%, about 60%, about 65%, about 70%, about 75%, about 80%, about 85%, about 90%, about 95%, or about 100%, as compared to a similar wound not topically treated with a composition comprising at least one aminosterol or salt or derivative thereof, as measured quantitatively or qualitatively by reference to a clinically recognized scale or tool.
43. The method of any one of embodiments 30-42, wherein the subject is a member of a patient population characterized by an impediment to normal cutaneous wound healing.
44. The method of embodiment 43, wherein the subject is diabetic.
45. The method of any one of embodiments 30-44, wherein the wound is accompanied by a microbial infection.
46. The method of embodiment 45, wherein the microbial infection is a bacterial, viral or yeast infection.
47. The method of embodiment 46, wherein the microbial infection is *Staphylococcus* spp., *Staphylococcus aureus,* methicillin-resistant *Staphylococcus aureus, Pseudomonas* spp., *Pseudomonas aeruginosa, Enterococcus* spp., *vancomycin-resistant Enterococcus, Pseudomonas* spp., *Acinetobacter* spp., non-albicans *Candida* spp., *or Aspergillus* spp.
48. The method of any one of embodiments 30-47, wherein the pharmaceutical composition is administered in conjunction with a skin graft.
49. The method of any one of embodiments 30-48, wherein the pharmaceutical aminosterol composition is administered in conjunction with acellular or cellular dermal matrices.
50. The method of any one of embodiments 30-49, wherein the pharmaceutical aminosterol composition is administered to a cutaneous wound.
51. The method of any one of embodiments 30-49, wherein the topical pharmaceutical aminosterol composition is administered to a non-cutaneous wound.
52. A method of rejuvenating or improving the health of mucosal tissue in a subject in need comprising topically administering to the mucosal tissue a therapeutically effective amount of a pharmaceutical composition comprising at least one aminosterol or a salt or derivative thereof.
53. The method of embodiment 52, wherein the method results in an increased thickness of the mucosal tissue, as compared to the same type and age of mucosal tissue not treated with an aminosterol composition.
54. The method of embodiment 53, wherein the thickness of the mucosal tissue is increased by about 5%, about 10%, about 15%, about 20%, about 25%, about 30%, about 40%, about 45%, about 50%, about 55%, about 60%, about 65%, about 70%, about 75%, about 80%, about 85%, about 90%, about 95%, or about 100%, and.
55. The method of embodiment 53 or 54, wherein the increase is measured quantitatively or qualitatively by reference to a clinically recognized scale or tool.
56. The method of any one of embodiments 52-55, wherein the method results in increasing the expression in the mucosal tissue of one or more proteins having an epithelial barrier function.
57. The method of embodiment 56, wherein the mRNA or gene correlated with protein expression is selected from the group consisting of caspase 14, collagen type XVII alpha 1, corneodesmosin, cornifelin, cystatin E/M, dermokine, desmocollin 1, desmoglein 1 beta, filaggrin, gap junction protein beta 4, gap junction protein beta 6, H19 imprinted maternally expressed transcript, hornerin, kallikrein related-peptidase 7 (chymotryptic stratum, keratin 1, keratin 10, keratinocyte differentiation associated protein, keratinocyte expressed proline-rich, late cornified envelope 1A1, late cornified envelope 1A2, late cornified envelope 1B, late cornified envelope 1C, late cornified envelope 1E, late cornified envelope 1F, late cornified envelope 1G, late cornified envelope 1H, late cornified envelope 11, late cornified envelope 1J, late cornified envelope 1L, late cornified envelope 1M, late cornified envelope 3C, late cornified envelope 3E, late cornified envelope 3F, lectin galactose binding soluble 7, Loricrin, and sciellin.
58. The method of any one of embodiments 52-57, wherein the method results in increasing the expression in the mucosal tissue of one or more proteins having a muscle tissue related function.
59. The method of any one of embodiments 56-58, wherein the mRNA or gene correlated with protein expression is selected from the group consisting of myoglobin, myosin binding protein C slow-type, myosin heavy polypeptide 1 skeletal muscle, myosin heavy polypeptide 8 skeletal muscle, myosin light chain phosphorylatable fast ske, myosin light polypeptide 3, myozenin 1, myozenin 2, and titin-cap.
60. The method of any one of embodiments 56-59, wherein protein expression is increased by at least about 90%.
61. The method of any one of embodiments 56-60, wherein protein expression is increased by at least about 100%, at least about 200%, at least about 250%, at least about 300%, at least about 350%, at least about 400%, at least about 450%, at least about 500%, at least about 550%, at least about 600%, at least about 650%, at least about 700%, at least about 750%, at least about 800%, at least about 850%, at least about 900%, at least about 950%, or at least about 1000%.
62. The method of any one of embodiments 30-61, wherein:
   (a) the pharmaceutical aminosterol composition is formulated into any pharmaceutically acceptable dosage form; and/or
   (b) the pharmaceutical aminosterol composition is emulsified in a gel matrix; and/or
   (c) the pharmaceutical aminosterol composition is formulated into a nasal spray, aerosol, spray, gel, solution, cream, ointment, eye drops, or a transdermal patch.
63. The method of any one of embodiments 30-62, wherein the pharmaceutical aminosterol composition is administered to a mucosal surface.
64. The method of any one of embodiments 30-63, wherein the aminosterol dosage:
   (a) ranges from about 1 to about 500 mg/day;
   (b) ranges from about 0.001 mL/cm² to about 15.0 mL/ cm²;
   (c) ranges from about 0.001 g/cm² to about 15.0 g/ cm²; or
   (d) ranges from about 0.001 g/cm² to about 15.0 g/ cm².
65. The method of any one of embodiments 1- 64, wherein the aminosterol or the pharmaceutical composition comprising an aminosterol is administered in combination with at least one additional active agent to achieve either an additive or synergistic effect.
66. The method of embodiment 65, wherein the additional active agent is administered via a method selected from the group consisting of concomitantly; as an admixture; separately and simultaneously or concurrently; and/or separately and sequentially.
67. The method of embodiment 65 or 66, wherein: (a) the additional active agent is a different aminosterol from that administered in the method of any one of embodiments 1-84; (b) the method comprises administering a first aminosterol which is aminosterol 1436 or a salt or derivative thereof intranasally and administering a second aminosterol which is squalamine or a salt or derivative thereof administered orally.
68. The method of any one of embodiments 1-67, further comprising orally or intranasally administering to the subject a non-topical pharmaceutical composition comprising the same or a different aminosterol to that administered in the method of any of embodiments 1-67, or a salt or derivative thereof.
69. The method of embodiment 68, wherein:
   (a) the non-topical pharmaceutical composition is administered orally and comprises an aminosterol or a salt or derivative thereof at about 10 mg up to about 500 mg, in single or divided doses; or
   (b) the non-topical pharmaceutical composition is administered intranasally and comprises an aminosterol at about 0.001 mg to about 6 mg, in single or divided doses.
70. The method of embodiment 68 or 69, wherein each dose of a non-topical pharmaceutical composition comprising an aminosterol or a salt or derivative thereof administered orally is taken on an empty stomach, optionally within two hours of the subject waking.
71. The method of embodiment 70, wherein no food is taken after about 60 to about 90 minutes of taking the non-topical aminosterol dose.
72. The method of any one of embodiments 1- 71, wherein the aminosterol or a salt or derivative thereof is a pharmaceutically acceptable grade of at least one aminosterol or a pharmaceutically acceptable salt or derivative thereof.
73. The method of any one of embodiments 1-72, wherein the aminosterol or a salt or derivative thereof is: (a) isolated from the liver of *Squalus acanthias*; (b) squalamine; (c) a squalamine isomer; (d) a squalamine salt; (e) a phosphate salt of squalamine; (f) aminosterol 1436; (g) an aminosterol 1436 isomer; (h) an aminosterol 1436 salt; (i) a phosphate salt of aminosterol 1436; (j) comprises a sterol nucleus and a polyamine attached at any position on the sterol, such that the molecule exhibits a net charge of at least + 1; (k) comprises a bile acid nucleus and a polyamine, attached at any position on the bile acid, such that the molecule exhibits a net charge of at least + 1; (l) a derivative modified to include one or more of the following: (i) substitutions of the sulfate by a sulfonate, phosphate, carboxylate, or other anionic moiety chosen to circumvent metabolic removal of the sulfate moiety and oxidation of the cholesterol side chain; (ii) replacement of a hydroxyl group by a non-metabolizable polar substituent, such as a fluorine atom, to prevent its metabolic oxidation or conjugation; and (iii) substitution of one or more ring hydrogen atoms to prevent oxidative or reductive metabolism of the steroid ring system; (m) a derivative of squalamine modified through medical chemistry to improve bio-distribution, ease of administration, metabolic stability, or any combination thereof; (n) aminosterol 1436; (o) the phosphate salt of aminosterol 1436; and/or (p) a synthetic aminosterol.
74. The method of any one of embodiments 1-73, wherein the aminosterol is administered as a composition comprising one or more of the following: (a) an aqueous carrier; (b) a buffer; (c) a sugar; and/or (d) a polyol compound.
75. The method of any one of embodiments 1-74, wherein the aminosterol is a phosphate salt.
76. The method of any one of embodiments 1-75, wherein the aminosterol is selected from the group consisting of: 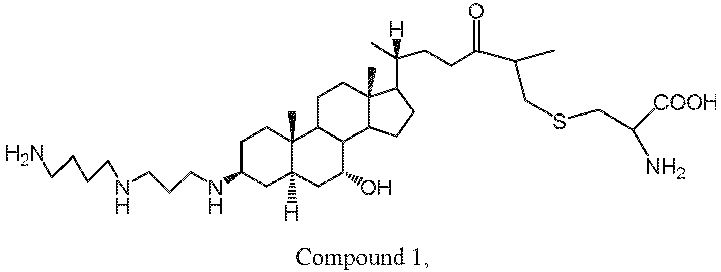 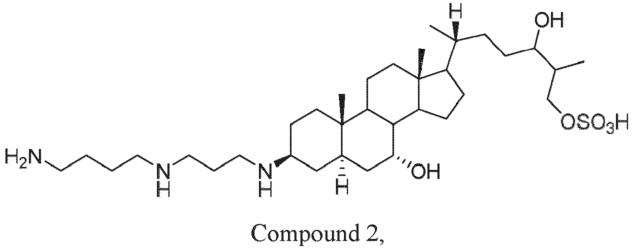 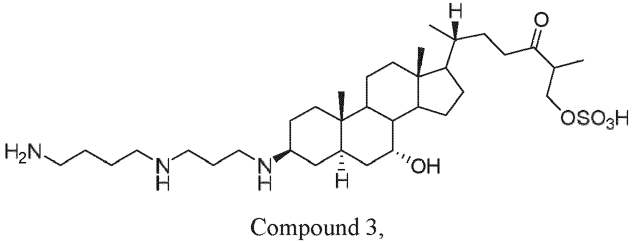 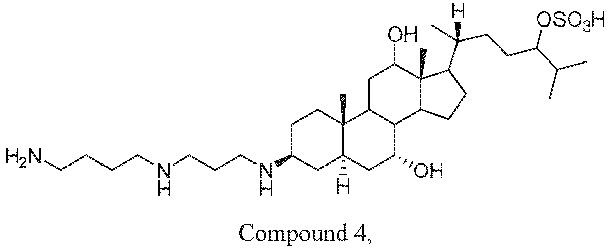 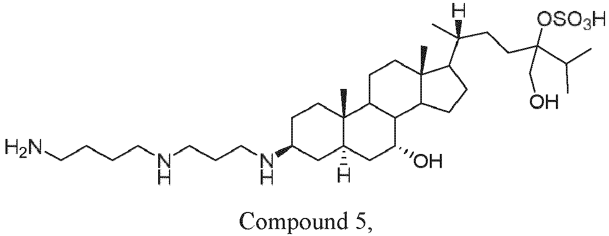 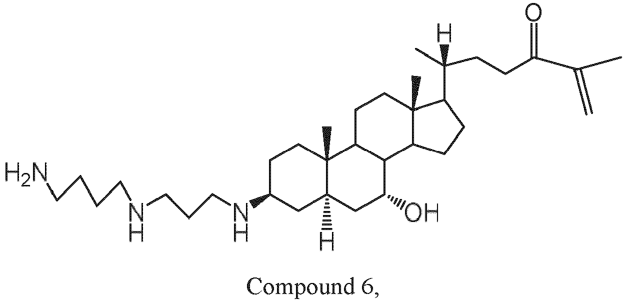 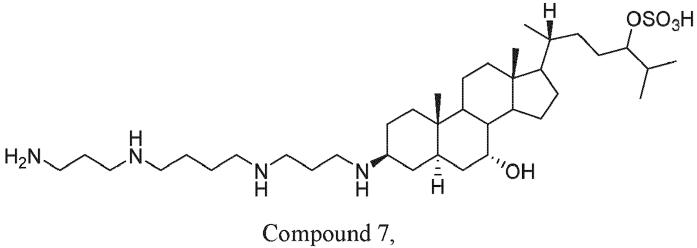 and 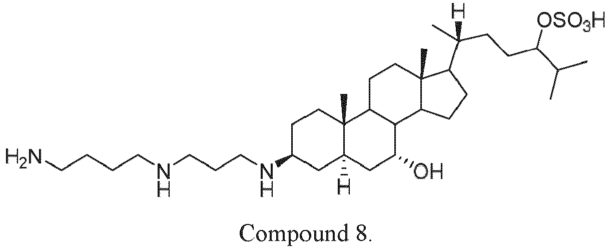
77. The method of any one of embodiments 1-75, wherein the aminosterol is selected from the group consisting of: 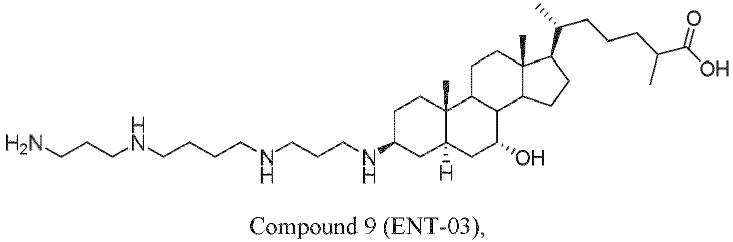 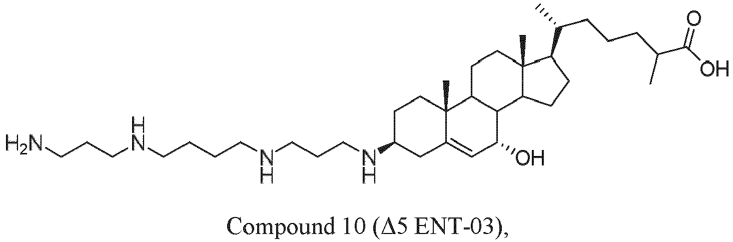 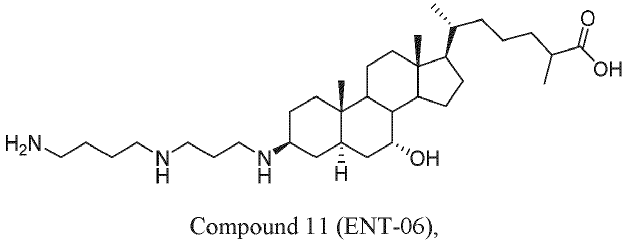 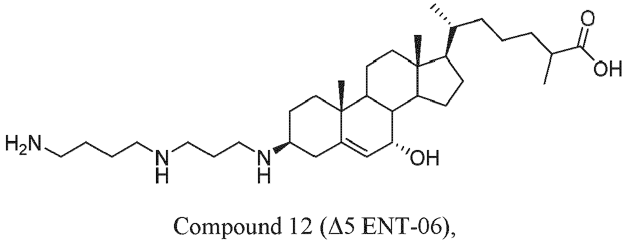 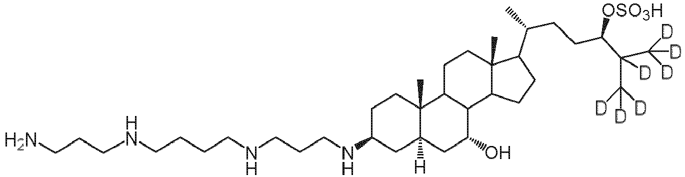 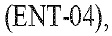 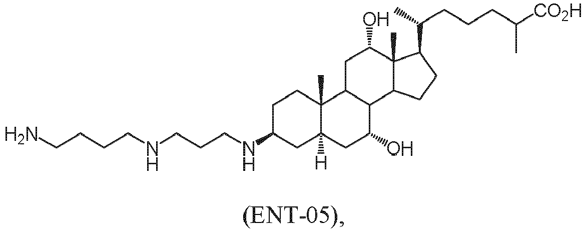 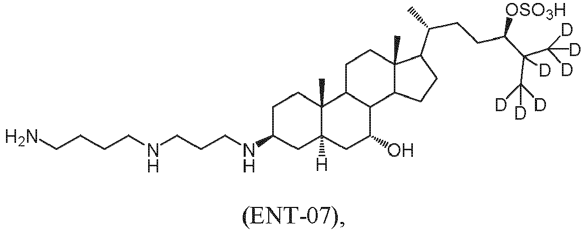 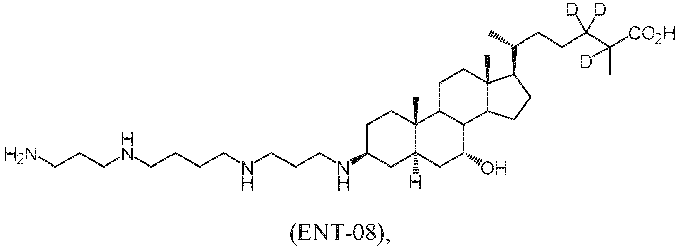 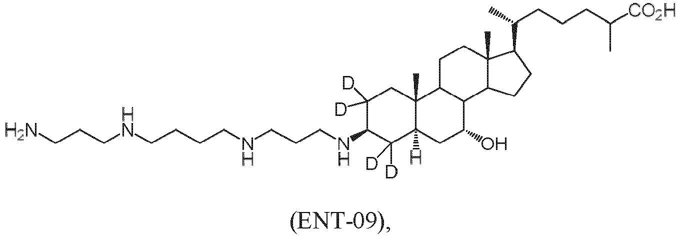 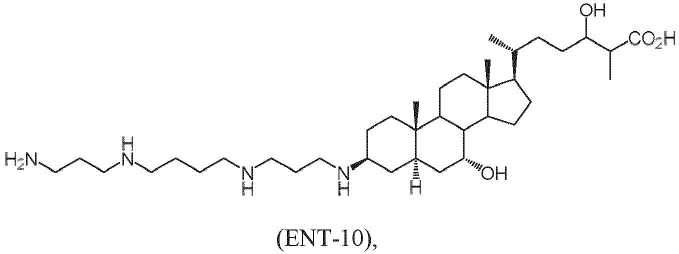 or 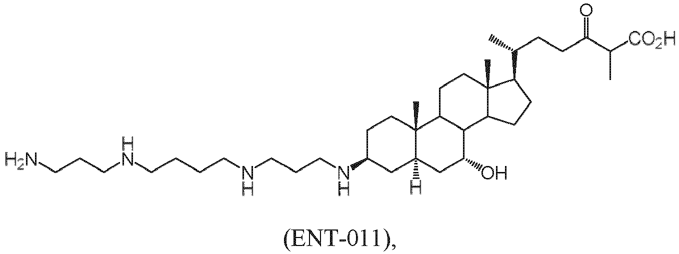 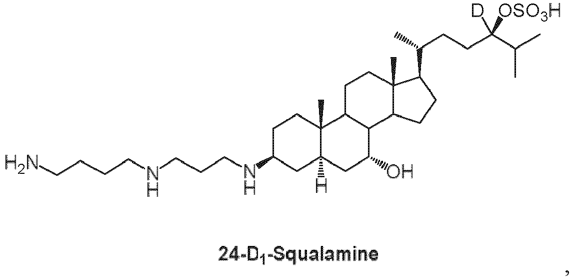 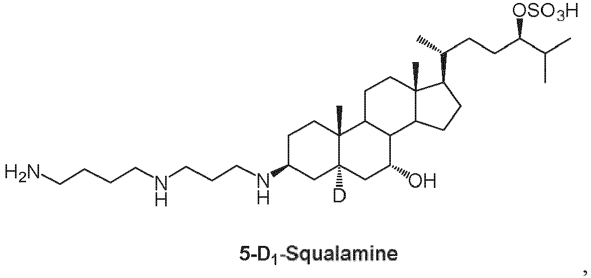 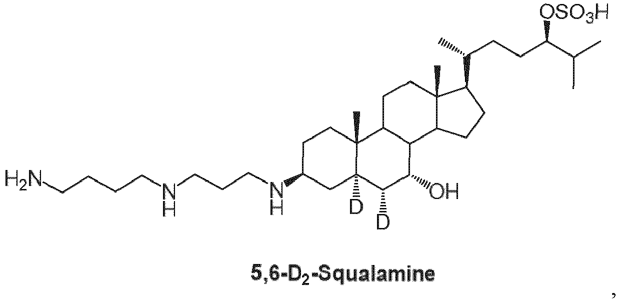 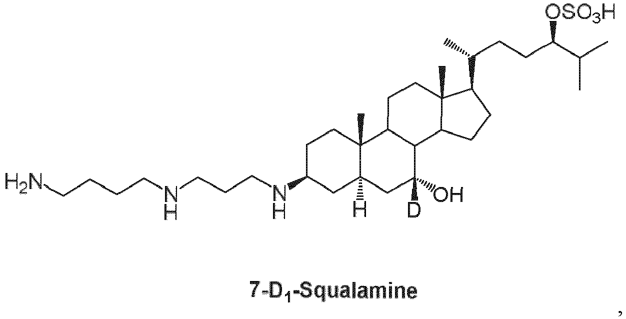 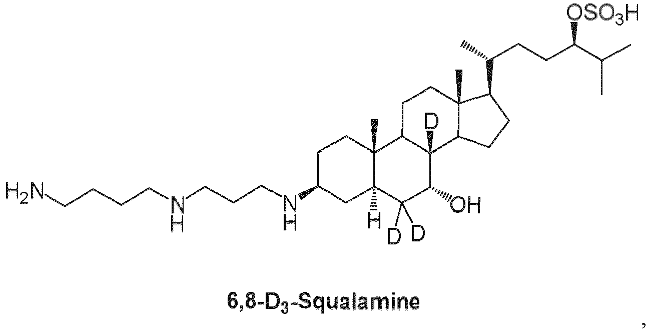 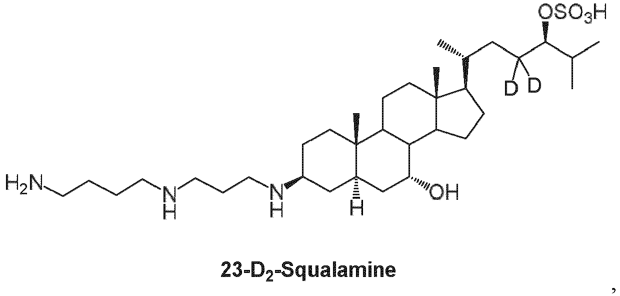 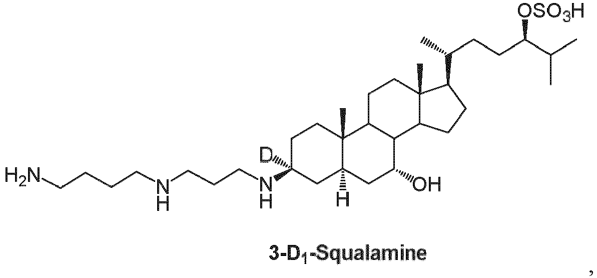 or 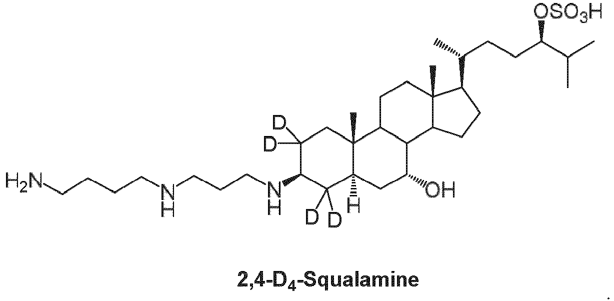
78. The method of any one of embodiments 1-77, wherein the aminosterol is administered as a pharmaceutical aminosterol composition further comprising one or more of the following: (a) at least one aqueous carrier; (b) at least one buffer; (c) at least one sugar; and/or (d) at least one polyol compound.
79. The method of any one of embodiments 1-78, wherein the subject is a human.
80. A drug container comprising a label and at least one aminosterol or a salt or derivative thereof, for the treatment of a disease or condition, or a symptom thereof in a subject, wherein the label requires that the subject not be lying down when the aminosterol is administered to the subject.
81. The drug container of embodiment 80, wherein the label requires that the subject is standing up or sitting.
82. The drug container of embodiment 80 or 81, wherein the label requires that the administration comprises oral administration.
83. The drug container of any one of embodiments 80-82, wherein:
   (a) the label requires that the subject is standing up or sitting for about 5 minutes after administration of the at least one aminosterol or a salt or derivative thereof; and/or
   (b) the label requires that the subject is standing up or sitting for about 15 minutes after administration of the at least one aminosterol or a salt or derivative thereof; and/or
   (c) the label requires that the subject is standing up or sitting for about 30 minutes after administration of the at least one aminosterol or a salt or derivative thereof; and/or
   (d) the label requires that the subject is standing up or sitting for about 45 minutes after administration of the at least one aminosterol or a salt or derivative thereof; and/or
   (e) the label requires that the subject is standing up or sitting for about 60 minutes after administration of the at least one aminosterol or a salt or derivative thereof.
84. The drug container of any one of embodiments 80-83, wherein: (a) the symptom is selected from the group consisting of constipation, hallucinations, cognitive impairment, and inflammation; (b) the symptom is correlated with a synucleopathy, a neurodegenerative disease, a neurological disease or disorder, a psychological and/or behavior disorder, or a cerebral or general ischemic disorder or condition; (c) the disease or condition is a synucleopathy, neurodegenerative disease, or neurological disease or disorder; (d) the disease or condition is a psychological and/or behavior disorder; or (e) the disease or condition is a cerebral or general ischemic disorder or condition.
85. The drug container of embodiment 84, wherein: (a) the synucleopathy, neurodegenerative disease, or neurological disease or disorder is selected from the group consisting of Parkinson's disease, Alzheimer's disease, schizophrenia, multiple system atrophy, Lewy body dementia, dementia with Lewy bodies, Huntington's Disease, Multiple Sclerosis, Amyotorphic Lateral Sclerosis, Friedreich's ataxia, vascular dementia, spinal muscular atrophy, supranuclear palsy, progressive nuclear palsy, frontotemporal dementia, progressive nuclear palsy, Guadeloupian Parkinsonism, spinocerebellar ataxia, parkinsonism, traumatic brain injury, degenerative processes associated with aging, and dementia of aging; or (b) the psychological or behavior disorder is selected from the group consisting of depression, autism, autism spectrum disorder, down syndrome, Gaucher's disease, Krabbe's disease, lysosomal conditions affecting glycosphingolipid metabolism, ADHD, agitation, anxiety, delirium, irritability, illusion and delusions, amnesia, apathy, bipolar disorder, disinhibition, aberrant motor and obsessive-compulsive behaviors, addiction, cerebral palsy, epilepsy, major depressive disorder, and sleep disorders such as REM sleep behavior disorder (RBD), sleep fragmentation, REM behavior disorder, circadian rhythm dysfunction, sleep apnea, and cognitive impairment; or (c) the cerebral or general ischemic disorder or condition is selected from the group consisting of microangiopathy, intrapartum, cerebral ischemia, cerebral ischemia during/after cardiac arrest or resuscitation, cerebral ischemia due to intraoperative problems, cerebral ischemia during carotid surgery, chronic cerebral ischemia due to stenosis of blood-supplying arteries to the brain, sinus thrombosis or thrombosis of cerebral veins, cerebral vessel malformations, diabetic retinopathy, high cholesterol, myocardial infarction, cardiac insufficiency, cardiac failure, congestive heart failure, myocarditis, pericarditis, perimyocarditis, coronary heart disease, angina pectoris, congenital heart disease, shock, ischemia of extremities, stenosis of renal arteries, diabetic retinopathy, thrombosis associated with malaria, artificial heart valves, anemias, hypersplenic syndrome, emphysema, lung fibrosis, erectile dysfunction, cardiac conduction defects, high blood pressure, low blood pressure, and pulmonary edema.

## Claims

1. An aminosterol compound, or a pharmaceutically acceptable salt, solvate, prodrug, enantiomer, or derivative thereof, having a structure selected from the group consisting of:
(a) 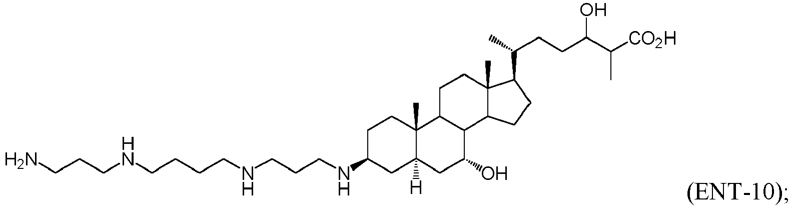
(b) 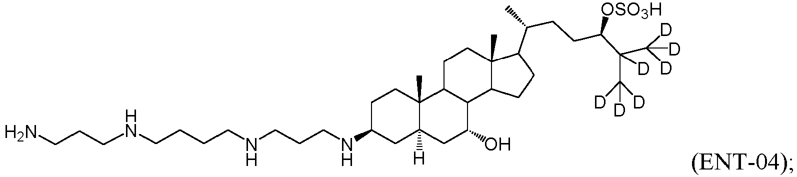
(c) 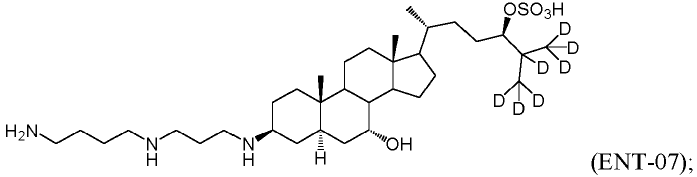
(d) 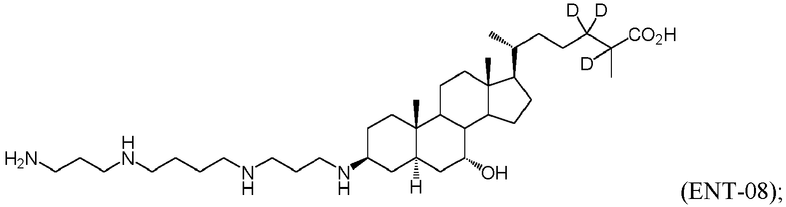
(e) 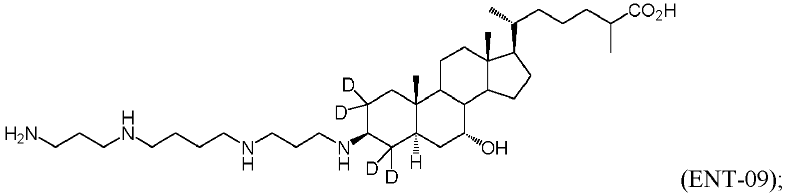 or
(f)

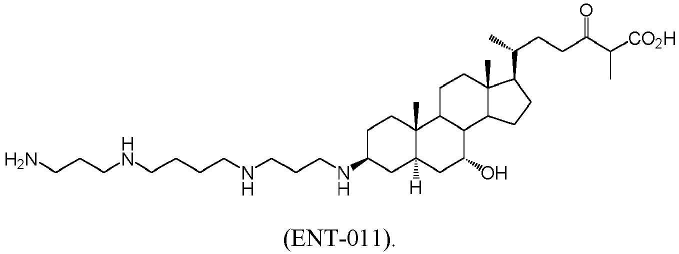

2. An aminosterol of claim 1 which is a phosphate salt.

3. A pharmaceutical composition comprising the aminosterol compound, or a pharmaceutically acceptable salt, solvate, prodrug, enantiomer, or derivative thereof, of claim 1 or 2 and further comprising one or more pharmaceutically acceptable carriers.

4. The pharmaceutical composition of claim 3 formulated into any pharmaceutically acceptable dosage form.

5. The pharmaceutical composition of claim 4, wherein the dosage form is an oral dosage form.

6. The pharmaceutical composition of any one of claims 2-5 further comprising at least one additional active agent.

7. A pharmaceutical composition comprising the aminosterol compound, or a pharmaceutically acceptable salt, solvate, prodrug, enantiomer, or derivative thereof, of claim 1, or the pharmaceutical composition of any one of claims 2-6, for use in a method of treating, preventing, and/or slowing the onset or progression of a disease or condition, or a symptom thereof, in a subject in need.

8. The pharmaceutical composition for use of claim 7, wherein the aminosterol is administered in a therapeutically effective amount which comprises:
(a) about 0.1 to about 20 mg/kg body weight of the subject;
(b) about 0.1 to about 15 mg/kg body weight of the subject;
(c) about 0.1 to about 10 mg/kg body weight of the subject;
(d) about 0.1 to about 5 mg/kg body weight of the subject;
(e) about 0.1 to about 2.5 mg/kg body weight of the subject;
(f) about 0.001 to about 500 mg/day;
(g) about 0.001 to about 250 mg/day;
(h) about 0.001 to about 125 mg/day;
(i) about 0.001 to about 50 mg/day;
(j) about 0.001 to about 25 mg/day;
(k) about 0.001 to about 10 mg/day; or
(l) about 1 to about 500 mg/day.

9. The pharmaceutical composition for use of claim 7 or 8, wherein the composition is administered orally and wherein the composition is taken on an empty stomach, optionally wherein no food is consumed for about 60 to about 90 minutes following administration.

10. The pharmaceutical composition for use of any one of claims 7-9, wherein an improvement in the disease or condition, or a symptom thereof, is measured quantitatively or qualitatively by one or more medically-recognized techniques, scales or tools; and/or the improvement in the symptom is at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100%.

11. The pharmaceutical composition for use of any one of claims 7-10, wherein the disease or disorder is selected from the group consisting of:
(a) a synucleopathy, neurodegenerative disease, or neurological disease or disorder, and optionally wherein the synucleopathy, neurodegenerative disease, or neurological disease or disorder is selected from the group consisting of Parkinson's disease, Alzheimer's disease, schizophrenia, multiple system atrophy, Lewy body dementia, dementia with Lewy bodies, Huntington's Disease, Multiple Sclerosis, Amyotorphic Lateral Sclerosis, Friedreich's ataxia, vascular dementia, spinal muscular atrophy, supranuclear palsy, progressive nuclear palsy, frontotemporal dementia, progressive nuclear palsy, Guadeloupian Parkinsonism, spinocerebellar ataxia, parkinsonism, traumatic brain injury, degenerative processes associated with aging, and dementia of aging;
(b) the disease or condition is a psychological and/or behavior disorder, and optionally wherein the psychological or behavior disorder is selected from the group consisting of depression, autism, autism spectrum disorder, down syndrome, Gaucher's disease, Krabbe's disease, lysosomal conditions affecting glycosphingolipid metabolism, ADHD, agitation, anxiety, delirium, irritability, illusion and delusions, amnesia, apathy, bipolar disorder, disinhibition, aberrant motor and obsessive-compulsive behaviors, addiction, cerebral palsy, epilepsy, major depressive disorder, and sleep disorders such as REM sleep behavior disorder (RBD), sleep fragmentation, REM behavior disorder, circadian rhythm dysfunction, sleep apnea, and cognitive impairment;
(c) the disease or condition is a cerebral or general ischemic disorder or condition, and optionally wherein the cerebral or general ischemic disorder or condition is selected from the group consisting of microangiopathy, intrapartum, cerebral ischemia, cerebral ischemia during/after cardiac arrest or resuscitation, cerebral ischemia due to intraoperative problems, cerebral ischemia during carotid surgery, chronic cerebral ischemia due to stenosis of blood-supplying arteries to the brain, sinus thrombosis or thrombosis of cerebral veins, cerebral vessel malformations, diabetic retinopathy, high cholesterol, myocardial infarction, cardiac insufficiency, cardiac failure, congestive heart failure, myocarditis, pericarditis, perimyocarditis, coronary heart disease, angina pectoris, congenital heart disease, shock, ischemia of extremities, stenosis of renal arteries, diabetic retinopathy, thrombosis associated with malaria, artificial heart valves, anemias, hypersplenic syndrome, emphysema, lung fibrosis, erectile dysfunction, cardiac conduction defects, high blood pressure, low blood pressure, and pulmonary edema; and/or
(d) the disease or condition is an inflammatory disease or condition caused by excessive expression or concentration of alpha-synuclein.

12. The pharmaceutical composition for use of any one of claims 7-11, wherein the symptom is selected from the group consisting of:
(a) at least one non-motor aspect of experiences of daily living as defined by Part I of the Unified Parkinson's Disease Rating Scale selected from the group consisting of cognitive impairment, hallucinations and psychosis, depressed mood, anxious mood, apathy, features of dopamine dysregulation syndrome, sleep problems, daytime sleepiness, pain, urinary problems, constipation problems, lightheadedness on standing, and fatigue;
(b) at least one motor aspect of experiences of daily living as defined by Part II of the Unified Parkinson's Disease Rating Scale selected from the group consisting of speech, saliva and drooling, chewing and swallowing, eating tasks, dressing, hygiene, handwriting, turning in bed, tremors, getting out of a bed, a car, or a deep chair, walking and balance, and freezing;
(c) at least one motor symptom identified in Part III of the Unified Parkinson's Disease Rating Scale selected from the group consisting of speech, facial expression, rigidity, finger tapping, hand movements, pronation-supination movements of hands, toe tapping, leg agility, arising from chair, gait, freezing of gait, postural stability, posture, body bradykinesia, postural tremor of the hands, kinetic tremor of the hands, rest tremor amplitude, and constancy of rest tremor;
(d) at least one motor complication identified in Part IV of the Unified Parkinson's Disease Rating Scale selected from the group consisting of time spent with dyskinesias, functional impact of dyskinesias, time spent in the off state, functional impact of fluctuations, complexity of motor fluctuations, and painful off-state dystonia;
(e) constipation;
(f) depression;
(g) cognitive impairment;
(h) sleep problem, sleep disorder, or sleep disturbance;
(i) circadian rhythm dysfunction;
(j) hallucinations;
(k) fatigue;
(I) REM disturbed sleep;
(m) REM behavior disorder;
(n) erectile dysfunction;
(o) apnea;
(p) postural hypotension;
(q) correction of blood pressure or orthostatic hypotension;
(r) nocturnal hypertension;
(s) regulation of temperature;
(t) improvement in breathing or apnea;
(u) correction of cardiac conduction defect;
(v) amelioration of pain;
(w) restoration of bladder sensation and urination;
(x) urinary incontinence; and/or
(y) control of nocturia.
